(19) Europäisches Patentamt
European Patent Office
Office européen des brevets

(11) **EP 1 477 800 A1**

(12) **EUROPEAN PATENT APPLICATION**
published in accordance with Art. 158(3) EPC

(43) Date of publication:
**17.11.2004 Bulletin 2004/47**

(21) Application number: **03703116.8**

(22) Date of filing: **31.01.2003**

(51) Int Cl.[7]: **G01N 30/88**, G01N 30/48, G01N 30/26, G01N 30/74, G01N 30/60, G01N 27/62

(86) International application number:
**PCT/JP2003/000974**

(87) International publication number:
**WO 2003/065031 (07.08.2003 Gazette 2003/32)**

(84) Designated Contracting States:
**AT BE BG CH CY CZ DE DK EE ES FI FR GB GR HU IE IT LI LU MC NL PT SE SI SK TR**
Designated Extension States:
**AL LT LV MK RO**

(30) Priority: **31.01.2002 JP 2002024498**

(71) Applicant: **Gl Sciences Incorporated**
**Tokyo 163-1130 (JP)**

(72) Inventors:
- **KURODA, Ikuma, c/o GL Sciences Incorporated Iruma-shi, Saitama 358-0032 (JP)**
- **TAKAHASHI, Kouji, c/o GL Sciences Incorporated Iruma-shi, Saitama 358-0032 (JP)**
- **NAKAMURA, Hiroshi Chiba-shi, Chiba 262-0044 (JP)**

(74) Representative: **Staeger - Sperling**
**Müllerstrasse 3**
**80469 München (DE)**

(54) **METHOD AND APPARATUS FOR ANALYZING AMINO ACID, PEPTIDE, PROTEIN, SACCHARIDE OR LIPID**

(57) The present invention aims to easily and simply perform selective detection/identification, extraction, separation, and purification of a phosphorylated amino acid, phosphorylated peptide, or phosphorylated protein from a sample containing any one of them, comprising the steps of: retaining the phosphorylated amino acid, phosphorylated peptide, or phosphorylated protein in a pretreatment column by passing the sample containing the phosphorylated amino acid, phosphorylated peptide, or phosphorylated protein through a reversed phase HPLC column having a titanium dioxide column as the pretreatment column and retaining a non-phosphorylated amino acid, peptide, or protein in the reverse phase HPLC column; eluting the non-phosphorylated amino acid, peptide, or protein that is retained in the reversed phase HPLC column; eluting the phosphorylated amino acid, phosphorylated peptide, or phosphorylated protein retained in the pretreatment column with buffer; and detecting it.

Fig. 1

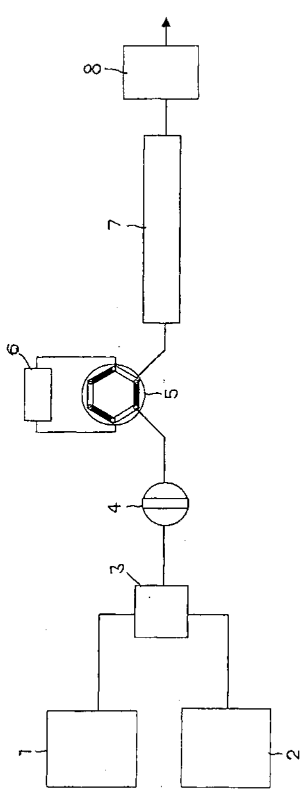

EP 1 477 800 A1

## Description

BACKGROUND OF THE INVENTION

Field of the Invention

The present invention relates to a method and an apparatus for simply extracting or separating a phosphorylated compound such as a phosphorylated amino acid, phosphorylated peptide, or phosphorylated protein.

Description of the Related Art

A phosphorylated protein plays an important role in vivo such as intracellular signaling. Therefore, a phosphorylated protein analysis technique is essential for obtaining knowledge on an intracellular signaling cascade. Signaling analysis in vivo is important in the fields of medicine, pharmaceuticals, and food, as well as in academic fields.

In order to know changes of intracellular states including signaling, proteomics that has appeared recently aims to determine changes of intracellular states including signaling by detecting and identifying all proteins expressed in a cell in a certain state (for example, a state in which some stimuli are applied) and analyzing an existing amount change of each protein, interactions between proteins, and their modifications or other active forms. In a currently common experimental technique, a fractionated protein is hydrolyzed with a digestive enzyme such as trypsin, the resultant peptide is subj ected to MS analysis, and the protein is identified and determined by utilizing a database. However, as the database has improved, the experimental technique itself is changing to hydrolyze a protein in the mixture form followed by MS analysis, which requires a technique to detect/determine a phosphorylated peptide from a peptide mixture. The present invention can be a technique that meets the requirement.

Among phosphorylated protein analysis methods currently developed, some methods will be specifically described and problems of respective methods will be given.

One phosphorylated protein detection method comprises combining two-dimensional electrophoresis and alkaline phosphatase treatment in which a spot that shifts by the phosphatase treatment is a phosphorylated protein and the phosphorylated protein is hydrolyzed followed by MS analysis for determining a phosphorylated site and for analyzing a phosphorylation degree of the protein. However, judging spot shifts by comparing two electrophoresis plates is an operation that requires time and experience.

Further, another method comprises chemically converting a phosphorylated amino acid residue to a partially deuterated biotin residue followed by biotin-avidin affinity, trypsin digestion, and MS/MS analysis so as to selectively condense or determine a phosphorylated protein or so as to determine a phosphorylation degree of the protein. However, this method is an indirect analysis method due to the chemical conversion of a phosphate group to a biotin residue.

Moreover, pretreatments for phosphorylated peptide analysis by MALDI-TOF (Matrix Assisted Laser Desorption Ionization-time of flight) MS or Nanoelectrospray MS include a method for enriching a phosphorylated peptide utilizing complex formation between a phosphate group and a metal ion. However, this method sometimes cannot recognize one phosphate group in a peptide. Further, when an aspartate residue, glutamate residue, or the like exists in a peptide, these amino acids also form a complex with a metal ion under a condition in which a phosphate group forms a complex with a metal ion. Therefore, selective enrichment is difficult.

As a technique for determining a phosphorylation degree of a protein after separating a desired phosphorylated protein by means of complex formation between a histidine fusion protein and a column of nickel, iron, steel, or the like, or affinity between GST (glutathione-s-transferase) fusion protein and a glutathione-conjugated column, a method for determining a phosphorylated amino acid by immunoblotting using an antibody can be mentioned. However, since an antibody is not useful to all of various phosphorylated amino acids, an appropriate antibody sometimes needs to be chosen from some antibodies, or an experiment sometimes needs to begin with producing an antibody, requiring trial and error. Alternatively, there is a method for determining beforehand a content by using an isotope $^{32}P$. However, this method requires time for labeling a protein and moreover has a risk that an experimenter is exposed to radioactivity on determining the content in the protein with $^{32}P$.

Techniques like the above are being used for analyzing a phosphorylated protein. However, among others, in a field such as proteomics, a lot of analytes have to be treated. Therefore, the analysis is desired to be simplified/accelerated and low-priced. Titanium dioxide ($TiO_2$) is receiving an attention as a chemoaffinity carrier due to the usefulness and the wide applicable range. Titanium dioxide has an excellent configuration recognition function as well as high acid and alkali resistance. In particular, it has become evident that titanium dioxide shows high adsorption selectivity to a phosphate group.

Consequently, the present invention aims to provide a method and an apparatus using the same for selective extraction or separation, purification, and detection of a phosphorylated compound such as a phosphorylated amino acid, phosphorylated peptide, phosphorylated protein, phosphorylated sugar, or phosphorylated lipid without destroying

their structures in a simple, safe, rapid, and low-priced manner.

SUMMARY OF THE INVENTION

In order to improve the speed, reduce the necessary cost, and reduce cumbersomeness and risks in experimental operations for analysis of a phosphorylated amino acid, a phosphorylated peptide, and a phosphorylated protein in academic and industrial fields by selectively extracting or separating a phosphorylated substance such as a phosphorylated amino acid, phosphorylated peptide, or phosphorylated protein in a simple and rapid manner, the present invention is firstly characterized by comprising the steps of: retaining a phosphorylated compound in which a sample containing a phosphorylated amino acid, phosphorylated peptide, phosphorylated protein, phosphorylated sugar, or phosphorylated lipid is retained in a titanium dioxide column as a pretreatment column under an acidic condition; detecting a non-phosphorylated compound in which an amino acid, peptide, protein, sugar, or lipid that has not been retained is separated and /or purified through a desired column and then detected with a desired detector, or the compound is detected with the desired detector without purification, or the compound is not either separated and purified or detected; eluting the phosphorylated compound in which the phosphorylated amino acid, phosphorylated peptide, phosphorylated protein, phosphorylated sugar, or phosphorylated lipid retained in the pretreatment column is eluted by a solution containing phosphate, phosphoric acid, or alkali; and detecting the phosphorylated compound in which the compound is separated and purified through the desired column and then detected with the desired detector, or the compound is detected with the desired detector without purification. This method makes it possible to achieve effects such as the speed improvement, simplification of experimental operations, and risk prevention for analysis of a phosphorylated amino acid, a phosphorylated peptide, a phosphorylated protein, a phosphorylated sugar, and a phosphorylated lipid in academic and industrial fields, by selectively extracting/separating a phosphorylated substance such as a phosphorylated amino acid, phosphorylated peptide, phosphorylated protein, phosphorylated sugar, or phosphorylated lipid in a simple and rapid manner, leading to a great benefit to industry, especially to the food and pharmaceutical industries. Further, this method makes it possible to discharge ingredients from a pretreatment column. At that time, an elution step from the column and also unsurprisingly a detection step can be omitted so that it remains only steps for necessary ingredients, leading to acceleration and simplification of the operations.

The present invention is secondly characterized by comprising the steps of: supplying a phosphorylated amino acid, phosphorylated peptide, phosphorylated protein, or sample containing them in an acidic solution at a pH of about 2 to a reversed phase HPLC column with a titanium dioxide column as a pretreatment column, selectively retaining the phosphorylated amino acid, phosphorylated peptide, or phosphorylated protein from the sample matrix, purifying an amino acid, peptide, or protein that has not been retained in the pretreatment column by the reversed phase HPLC column followed by detection thereof by a desired detector; eluting the phosphorylated amino acid, phosphorylated peptide, or phosphorylated protein retained in the pretreatment column with 50 mM or higher phosphate buffer followed by separation thereof through the reversed phase HPLC column and detection thereof with the desired detector. This method makes it possible to select a phosphorylated amino acid or the like from a sample by subjecting the sample containing a phosphorylated amino acid, phosphorylated peptide, or phosphorylated protein to this process. That is, this series of steps makes it possible to perform extraction, separation, purification, qualitative analysis, quantitative determination, or the like of a desired ingredient such as a phosphorylated amino acid, phosphorylated peptide, or phosphorylated protein by an extremely simple operation without requiring conventional complicated and difficult operations such as removal of contaminating substances and fractionation of eluted ingredients by selectively retaining the desired ingredient such as the phosphorylated amino acid in a precolumn of a titania column; eluting other ingredients and sending them to the reversed phase HPLC column; performing an operation such as detection; eluting the desired ingredient; and retaining the desired ingredient in the reversed phase HPLC column; performing an operation such as detection.

The present invention is thirdly characterized in that a sample containing the phosphorylated amino acid, phosphorylated peptide, phosphorylated protein, phosphorylated sugar, or phospholipid is retained in a titanium dioxide column as a pretreatment column under an acidic condition and then fractionated directly for use without transferring the phosphorylated compound eluted in an elution step to a detection step. This method makes it possible to use the selectively extracted phosphorylated amino acid, phosphorylated peptide, phosphorylated protein, phosphorylated sugar, or phospholipid in a biochemical assay more rapidly by eliminating the detection step.

The present invention is fourthly characterized by comprising the steps of: retaining a phosphorylated compound in which a sample containing the phosphorylated amino acid, phosphorylated peptide, phosphorylated protein, phosphorylated sugar, or phospholipid is retained in a titanium dioxide column as a pretreatment column under an acidic condition, the phosphorylated compound being retained in the titanium dioxide column as the pretreatment column that is treated with an acidic solution at a pH of 0.5 to 7.0 to detect a non-phosphorylated compound; and then eluting the phosphorylated compound with a solution containing 10 mM to 2, 000 mM phosphate, 0.06% or more phosphoric acid, or alkali at a pH of 7.0 or higher to detect a phosphorylated compound. This method makes it possible

to selectively extract/separate the phosphorylated amino acid, phosphorylated peptide, phosphorylated protein, phosphorylated sugar, or phospholipid by using a simple operation and an inexpensive reagent.

The present invention is fifthly characterized by comprising the step of detecting a phosphorylated compound in which a sample containing the phosphorylated amino acid, phosphorylated peptide, phosphorylated protein, phosphorylated sugar, or phospholipid is retained in a titanium dioxide column as a pretreatment column under an acidic condition or the like, using a reversed phase solid phase for separation and/or purification of the phosphorylated compound, and using an MS having an ionization part of a MALDI plate as a desired detector. This method can bring the condition of the phosphorylated amino acid, phosphorylated peptide, phosphorylated protein, phosphorylated sugar, or phospholipid, which is difficult to be ionized when a contaminating matrix or salt is mixed, into a condition to be easily ionized and measured in MS.

The present invention is sixthly characterized by comprising the steps of: retaining a phosphorylated compound by retaining a sample containing a phosphorylated sugar in a titanium dioxide column as a pretreatment column under an acidic condition in which the sample is retained by an acid solution at a pH of about 0.8; detecting a non-phosphorylated compound after separation and purification thereof by use of an anion exchange column; eluting the phosphorylated compound with a solution containing 50 mM or more phosphate, 1% or more phosphoric acid, or alkali at a pH of 11.25 or higher; and detecting the phosphorylated compound after separation and purification thereof by use of the anion exchange column. This method makes it possible to subject the phosphorylated sugar that is extracted/separated by using a simple operation and an inexpensive reagent to analysis using a column with a nature different from that of the titanium dioxide column for identification, further purification, qualitative analysis, or quantitative determination.

The present invention is seventhly characterized by comprising the steps of: retaining a phosphorylated compound by retaining a sample containing the phospholipid in a titanium dioxide column as a pretreatment column under an acidic condition in which the sample is retained by a solution containing an acid solution at a pH of about 2.0; detecting a non-phosphorylated compound after separation and purification thereof by use of a reversed phase column; eluting the phosphorylated compound by a solution containing 0.5% or more phosphoric acid; and detecting the phosphorylated compound after separation and purification thereof by use of a reversed phase column. This method makes it possible to subject the phospholipid that is extracted/separated by using a simple operation and an inexpensive reagent to analysis using a column with a nature different from that of the titanium dioxide column for identification, further purification, qualitative analysis, or quantitative determination.

The present invention is eighthly characterized by comprising the steps of: retaining a phosphorylated compound by retaining a phosphorylated amino acid, phosphorylated peptide, phosphorylated protein, or sample containing them in a titanium dioxide column as a pretreatment column under an acidic condition in which the sample is retained by a solution containing an acid solution at a pH of about 2.0; detecting a non-phosphorylated compound after separation and purification thereof by use of a reversed phase column; eluting the phosphorylated compound with a solution containing 50 mM or more phosphate, 0.5% or more phosphoric acid, or a solution containing alkali having a pH of 11.25 or higher; and detecting the phosphorylated compound after separation and purification thereof by use of a reversed phase column. This method makes it possible to subject the phosphorylated amino acid, phosphorylated peptide, or phosphorylated protein that is extracted/separated by using a simple operation and an inexpensive reagent to analysis using a column with a nature different from that of the titanium dioxide column for identification, further purification, qualitative analysis, or quantitative determination.

The present invention is ninthly characterized in that a desired number of solvents can be selectably delivered into a pump, and a switching valve is connected to the pump via an injector, while a titanium dioxide column and a reversed phase HPLC column are connected to the switching valve so that the titanium dioxide column can be arbitrarily set as a pretreatment column, and a detector is connected to the reversed phase HPLC column. This apparatus passes a sample ingredient through the titanium dioxide column to selectively retain a desired ingredient such as a phosphorylated amino acid or phosphorylated peptide, then allows the reversed phase HPLC column to retain ingredients other than the desired ingredient, shifts to a detection operation, allows buffer to elute the desired ingredient from the titanium dioxide column, that is to be retained by the reversed phase HPLC column followed by detection and identification operations. Therefore, this apparatus is important also for pursuing a protein phosphorylation degree change that responds to an intracellular condition change in proteomics such as an existing amount change of the desired ingredient.

The present invention is tenthly characterized in that a desired number of solvents can be selectably delivered into a pump, and a switching valve is connected to the pump via an injector, while a titanium dioxide column and a solid phase extraction column are connected to the switching valve so that the titanium dioxide column can be arbitrarily set as a pretreatment column, and a detector is connected to the solid phase extraction column. This apparatus passes a sample ingredient through the titanium dioxide column to selectively retain a desired ingredient such as a phosphorylated amino acid or phosphorylated peptide, then allows the solid phase column to retain ingredients other than the desired ingredient, shifts to a detection operation, allows buffer to elute the desired ingredient from the titanium dioxide

column, that is to be retained by the solid phase column followed by detection and identification operations. Therefore, this apparatus is important also for pursuing a protein phosphorylation degree change that responds to an intracellular condition change in proteomics such as an existing amount change of the desired ingredient.

BRIEF DESCRIPTION OF THE DRAWINGS

FIG. 1 is a diagram of an apparatus according to one embodiment of the present invention;
FIG. 2 is a diffractogram of X-ray peaks of anatase type and rutile type titanium dioxides;
FIG. 3 is a diffractogram of X-ray peaks of Titansphere (registered trademark) TiO packing materials;
FIG. 4 is a chromatogram comparing HPLC data of peptide (1) ;
FIG. 5 is a chromatogram comparing HPLC data of peptide (2) ;
FIG. 6 is a chromatogram comparing HPLC data of peptide (3) ;
FIG. 7 is a chromatogram comparing HPLC data of peptide (4) ;
FIG. 8 is a chromatogram comparing HPLC data of peptide (5) ;
FIG. 9 is a chromatogram comparing HPLC data of peptide (6) ;
FIG. 10 is a diagram of an apparatus according to another embodiment of the present invention;
FIG. 11 is a chromatogram of peptide (5) eluted from a titanium dioxide column;
FIG. 12 is a chromatogram of peptide (5) eluted from a titanium dioxide column;
FIG. 13 is a diagram of an apparatus for examining an elution condition from titanium dioxide;
FIG. 14 is a chromatogram of peptide (5) eluted from a titanium dioxide column;
FIG. 15 is a chromatogram of peptide (5) eluted from a titanium dioxide column;
FIG. 16 is a chromatogram of peptide (5) eluted from a titanium dioxide column;
FIG. 17 is a chromatogram showing behavior of peptides in phosphate buffer (pH 5.6);
FIG. 18 is a chromatogram showing behavior of peptides in phosphate buffer (pH 5.9);
FIG. 19 is a chromatogram showing behavior of peptides in phosphate buffer (pH 6.5);
FIG. 20 is a chromatogram showing behavior of peptides in phosphate buffer (pH 7.0);
FIG. 21 is a chromatogram showing behavior of peptides in phosphate buffer (pH 5.6);
FIG. 22 is a chromatogram showing behavior of peptides in phosphate buffer (pH 5.9);
FIG. 23 is a chromatogram showing behavior of peptides in phosphate buffer (pH 6.5);
FIG. 24 is a chromatogram showing behavior of peptides in phosphate buffer (pH 7.0);
FIG. 25 is a diagram of an apparatus for selective analysis of a phosphorylated peptide;
FIG. 26 is a chromatogram determining a baseline of an eluant used in selective analysis;
FIG. 27 is a chromatogram showing selective analysis of phosphorylated peptide (1);
FIG. 28 is a chromatogram showing selective analysis of phosphorylated peptide (2);
FIG. 29 is a chromatogram showing selective analysis of phosphorylated peptide (3);
FIG. 30 is a chromatogram showing selective analysis of phosphorylated peptide (4);
FIG. 31 is a chromatogram showing selective analysis of phosphorylated peptide (5);
FIG. 32 is a chromatogram showing selective analysis of phosphorylated peptide (6);
FIG. 33 is a diagram of a fractionation apparatus;
FIG. 34 is a chromatogram comparing HPLC data of a tryptic digest of casein;
FIG. 35 is a chromatogram showing peak shift of a peptide to the hydrophobicity;
FIG. 36 is a chromatogram showing peak shift of a trypsinized casein to the hydrophobicity;
FIG. 37 is a perspective view of a syringe-barrel type column;
FIG. 38 is a perspective view of a cartridge type column;
FIG. 39 is a perspective view of a pipette-tip type column;
FIG. 40 is a perspective view of a well plate;
FIG. 41 is a chromatogram showing positions of respective fractions of an analysis experiment using a phosphorylated sugar;
FIG. 42 is a chromatogram showing the above respective fractions;
FIG. 43 is a chromatogram showing positions of respective fractions of an analysis experiment using a phosphorylated lipid;
FIG. 44 is a chromatogram comparing the above fractions;
FIG. 45 is a chromatogram detailing the above fractions;
FIG. 46 is a chromatogram detailing the above fractions;
FIG. 47 is a chromatogram detailing the above fractions;
FIG. 48 is a chromatogram showing positions of respective fractions of an analysis experiment using another

phosphorylated lipid;
FIG. 49 is a chromatogram comparing the above fractions;
FIG. 50 is a chromatogram detailing the above fractions; and
FIG. 51 is a chromatogram detailing the above fractions.

DETAILED DESCRIPTION OF THE PREFERRED EMBODIMENTS

The present invention will be specifically described below.

As one of the present inventions, a pretreatment of reversed phase HPLC analysis by use of a titanium dioxide column (Titansphere (registered trademark) TiO) is performed so that a phosphorylated peptide, phosphorylated amino acid, or phosphorylated protein (hereinafter referred to as "phosphorylated peptide") is selectively retained and a non-phosphorylated peptide, amino acid, or protein (hereinafter referred to as "peptide") is passed through the titanium dioxide column, and then retained and concentrated at the inlet of a reversed phase HPLC column. Here, the non-phosphorylated peptide or the like retained in the reversed phase HPLC column is eluted by changing an eluant, and then detected and analyzed by a connected detector and an analyzer for HPLC or the like.

Then, in order to remove an organic solvent in the reversed phase HPLC column, water (that is recommended to contain 0.1% trifluoroacetic acid) is passed through. Subsequently, the phosphorylated peptides are eluted from the titanium dioxide column. In order to retain the peptides in the reversed phase HPLC column, buffer is passed through the pretreatment column and the reversed phase HPLC column.

The buffer used is 0.1 M phosphate buffer at a pH of 5.6 to 7.0 that is suitable as a solvent for eluting phosphorylated peptide from the titanium dioxide column. Further, when the concentration of phosphoric acid in the buffer is high, phosphorylated peptide or the like can be eluted from the titanium dioxide column even if the pH of the buffer is low.

An apparatus according to an embodiment of the present invention will be described below with reference to FIG. 1.

A solvent 1 and a solvent 2 can be selectably delivered into a pump 3.

The pump 3 is connected to a switching valve 5 via an injector 4. The switching valve 5 is linked with a titanium dioxide column 6, and on the other end, a reversed phase HPLC column 7 is connected. A UV detector 8 is connected to the reversed phase HPLC column 7. The main ingredient of the packing materials for the titanium dioxide column 6 is preferably of anatase type titanium dioxide.

As the detector, any detector connectable to a high performance liquid chromatograph can be used. The examples include a fluorescence detector, a mass spectrum detector, an electrochemical detector, and a differential refractive index detector.

X-ray crystal analytical data of anatase type and rutile type titanium dioxides are shown in FIG. 2. X-ray crystal analysis results of the packing materials are shown in FIG. 3.

In FIG. 2, the peaks with a circle represent the peaks of anatase type titanium dioxide and the peaks without a circle represent the peaks of rutile type titanium dioxide. In FIG. 3, peaks of anatase-type titanium dioxide appear, showing that the main ingredient of Titansphere (registered trademark) TiO, packing materials, is of anatase type.

A column filled with spheric titania gel is used. The particle size is 0.01 to 1,000 μm, preferably 1 to 10 μm in the case of HPLC, and 5 to 300 μm in the case of a solid phase. Moreover, a continuous form having a through hole of 0.1 to 20 μm is used.

<Example 1>

Followings are used as samples.

Four peptides of the same sequence having 19 amino acid residues, molecular weight around 2,000:

(1) A peptide in which the 11th threonine and the 12th tyrosine from the N-terminal are phosphorylated in admixture with a contaminating peptide
The one example is:

YSK IEK IGE GT(p) Y(p) GVV YKG R MW: 2307.46

(2) A peptide in which the 11th threonine from the N-terminal is phosphorylated in admixture with a contaminating non-phosphorylated peptide
The one example is:

YSK IEK IGE GT(p) Y GVV YKG R MW2227.46

(3) A peptide in which the 12th tyrosine from the N-terminal is phosphorylated
The one example is:

YSK IEK IGE GTY (p) GVV YKG R MW2227.46

(4) A non-phosphorylated peptide
The one example is:

YSK IEK IGE GTY GVV YKG R MW2147.46

and a human insulin receptor fragment (peptide of 12 amino acid residues)
(5) TRD IY (p) E TDY YRK (phosphate type)
(6) TRD IYE TDY YRK

Name of cited paper
Deshimaru S., Miyake Y., Ohmiya T., Tatsu Y., Endo Y., Yumoto N., and Toraya T., "cDNA cloning, expression, and catalytic properties of Cdc25 phosphatase domain of the starfish Asterina pectinifera," J. Biochem (2002), in press

Each of the samples was subjected to HPLC analysis with a reversed phase HPLC column (product name: Inertsil (registered trademark) WP300C8) and with a titanium dioxide column as a precolumn followed by a reversed phase HPLC column Inertsil (registered trademark) WP300C8 under the condition of gradient elution from 100% water containing 0.1% trifluoroacetic acid to 45% aqueous acetonitrile containing 0.1% trifluoroacetic acid. The outline of the HPLC analysis system used is shown in FIG. 1, and the analysis results are shown in FIGS. 4 to 9.

In FIG. 1, Titansphere (registered trademark) TiO (10 × 4.6 mm I.D., from GL Sciences Inc.) was used as the titanium dioxide column, and Inertsil (registered trademark) WP300C8 (10 × 4.6 mm I.D., from GL Sciences Inc.) as the reversed phase HPLC column. Water containing 0.1% trifluoroacetic acid was used as the solvent 1 and acetonitrile containing 0.1% trifluoroacetic acid as the solvent 2. As an HPLC system, followings were used: a pump of HITACHI Pump L-7120, a detector of HITACHI Diode Array Detector L-7455, a data processor of HITACHI SYSTEM MANAGER (all from HITACHI, Ltd.), an injector of RHEODYNE 8125 (from Rheodyne LLC), and a switching valve of SSC E1E010 (from Senshu Scientific co., ltd.). When using Inertsil (registered trademark) WP300C8 to which Titansphere (registered trademark) TiO was directly connected as a pretreatment column, the flowpath of the switching valve was white, and when using only Inertsil (registered trademark) WP300C8, the flowpath of the switching valve was black. All sample loops and pipings made from PEEK (registered trademark) were used.

FIG. 4 shows the analytical data of a peptide (1) of Example 1. In FIG. 4, among several peaks appeared on the data (a) from HPLC analysis using WP300C8, a main peak disappeared on the data (b) from HPLC analysis using Inertsil (registered trademark) WP300C8 to which Titansphere (registered trademark) TiO was directly connected as a pretreatment column.

Column: a. Inertsil (registered trademark) WP300C8 (150 × 4.6 mmI.D.)
b. Titansphere TiO (10 × 4.0 mmI.D.) → Inertsil (registered trademark) WP300C8 (150 × 4.6 mmI.D.)
Eluant: A. water (0.1% trifluoroacetic acid)
B. acetonitrile (0.1% trifluoroacetic acid)

A/B = 100/0 (0 min) → 55/45 (90 min)

Flow rate: 1.0 ml/min
Column temperature: room temperature
Detection wavelength: UV 210 nm
Sample: peptide (1)

FIG. 5 is analysis data of peptide (2) shown in Example 1. In FIG. 5, the strength of one peak appeared on data (a) provided by HPLC analysis using Inertsil (registered trademark) WP300C8 is decreased on data (b) provided by HPLC analysis using Inertsil (registered trademark) WP300C8 directly connected to Titansphere TiO as a pretreatment column.

Column: a. Inertsil (registered trademark) WP300C8 (150 × 4.6 mmI.D.)
b. Titansphere (registered trademark) TiO (10 × 4.0 mmI.D.) → Inertsil (registered trademark) WP300C8 (150 × 4.6 mmI.D.)
Eluant: A. water (0.1% trifluoroacetic acid)
B. acetonitrile (0.1% trifluoroacetic acid)

A/B = 100/0 (0 min) → 55/45 (90 min)

Flow rate: 1.0 ml/min
Column temperature: room temperature
Detection wavelength: UV 210 nm
Sample: peptide (2)

At a later date, peptide (2) was subjected to ion pair chromatography using a reversed phase HPLC column, and then two peaks were detected. This shows that peptide (2) appeared on data provided by the reversed phase HPLC column as one peak is a mixture with a peptide having similar polarity. Consequently, it is realized that reversed phase HPLC analysis by use of a titanium dioxide as a precolumn can analyze the phosphorylated degree of a phosphorylated peptide, which is difficult by reversed phase analysis.

FIG. 6 is analysis data of peptide (3) in Example 1. In FIG. 6, one peak appeared on data (a) provided by HPLC analysis using Inertsil (registered trademark) WP300C8 is disappeared nearly 100% on data (b) provided by HPLC analysis using Inertsil (registered trademark) WP300C8 directly connected to Titansphere (registered trademark) TiO as a pretreatment column.

Column: a. Inertsil (registered trademark) WP300C8 (150 × 4.6 mmI.D.)
b. Titansphere (registered trademark) TiO (10 × 4.0 mmI.D.) → Inertsil (registered trademark) WP300C8 (150 × 4.6 mmI.D.)
Eluant: A. water (0.1% trifluoroacetic acid)
B. acetonitrile (0.1% trifluoroacetic acid)

A/B = 100/0 (0 min) → 55/45 (90 min)

Flow rate: 1.0 ml/min
Column temperature: room temperature
Detection wavelength: UV 210 nm
Sample: peptide (3)

FIG. 7 is analysis data of peptide (4) in Example 1. In FIG. 7, the strength of one peak appeared on data (a) provided by HPLC analysis using Inertsil (registered trademark) WP300C8 is not decreased on data (b) provided by HPLC analysis using Inertsil (registered trademark) WP300C8 directly connected to Titansphere (registered trademark) TiO as a pretreatment column.

Column:a. Inertsil (registered trademark) WP300C8 (150 × 4.6 mmI.D.)
b. Titansphere (registered trademark) TiO (10 × 4.0 mmI.D.) → Inertsil (registered trademark) WP300C8 (150 × 4.6 mmI.D.)
Eluant: A. water (0.1% trifluoroacetic acid)
B. acetonitrile (0.1% trifluoroacetic acid)

A/B = 100/0 (0 min) → 55/45 (90 min)

Flow rate: 1.0 ml/min
Column temperature: room temperature
Detection wavelength: UV 210 nm
Sample: peptide (4)

FIG. 8 is analysis data of peptide (5) in Example 1. In FIG. 8, one peak appeared on data (a) provided by HPLC analysis using Inertsil (registered trademark) WP300C8 is disappeared nearly 100% on data (b) provided by HPLC analysis using Inertsil (registered trademark) WP300C8 directly connected to Titansphere (registered trademark) TiO as a pretreatment column.

Column: a. Inertsil (registered trademark) WP300C8 (150 × 4.6 mmI.D.)
b. Titansphere (registered trademark) TiO (10 × 4.0 mmI.D.) → Inertsil (registered trademark) WP300C8 (150 × 4.6 mmI.D.)
Eluant: A. water (0.1% trifluoroacetic acid)
B. acetonitrile (0.1% trifluoroacetic acid)

A/B = 100/0 (0 min) → 55/45 (45 min)

Flow rate: 1.0 ml/min
Column temperature: room temperature
Detection wavelength: UV 210 nm
Sample: peptide (5)

FIG. 9 is analysis data of peptide (6) in Example 1. In FIG. 9, the strength of one peak appeared on data (a) provided by HPLC analysis using Inertsil (registered trademark) WP300C8 is not decreased on data (b) provided by HPLC analysis using Inertsil (registered trademark) WP300C8 directly connected to Titansphere TiO as a pretreatment column.

Column: a. Inertsil (registered trademark) WP300C8 (150 × 4.6 mmI.D.)
b. Titansphere (registered trademark) TiO (10 × 4.0 mmI.D.) → Inertsil (registered trademark) WP300C8 (150 × 4.6 mmI.D.)
Eluant: A. water (0.1% trifluoroacetic acid)
B. acetonitrile (0.1% trifluoroacetic acid)

$$A/B = 100/0\ (0\ min) \rightarrow 55/45\ (45\ min)$$

Flow rate: 1.0 ml/min
Column temperature: room temperature
Detection wavelength: UV 210 nm
Sample: peptide (6)

These results show that, use of a titanium dioxide column (Titansphere (registered trademark) TiO) as a precolumn of a reversed phase HPLC column (Inertsil (registered trademark) WP300C8) results in that the peak of a phosphorylated peptide disappears on data provided by HPLC analysis using Inertsil (registered trademark) WP300C8 and that the peak strength of a non-phosphorylated peptide does not decrease. Consequently, it is realized that a titanium dioxide column (Titansphere (registered trademark) TiO) using a solvent with a ratio within the eluant condition for analyzing a peptide by use of Inertsil (registered trademark) WP300C8 (from an eluant of 100% water containing 0.1% trifluoroacetic acid to an eluant of 55% water containing 0.1% trifluoroacetic acid and 45% acetonitrile containing 0.1% trifluoroacetic acid) can selectively retain a phosphorylated peptide.

There is no limitation on the molecular weight range of a phosphorylated peptide of concern, and rather a phosphorylated peptide with any molecular weight can be analyzed. Peptide fragments with various molecular weights coexist in a trypsinized casein shown in Example 7 described below. However, even such a case, the phosphorylated peptide was selectively extracted. From this, it is evident that the molecular weight of a peptide of concern does not particularly influence selective extraction of a phosphorylated peptide by titanium dioxide.

In general, peptides of 20 or more of amino acid residues are known as a protein. A peptide of 19 amino acid residues could be selectively extracted as in Example 6 described below. This shows that a protein of about 20 amino acid residues can be duly extracted.

It is evident from an example of the enzyme-treated casein that the selective extraction by titanium dioxide does not depend on the number of amino acid residues (molecular weight). Further, it is widely known that titanium dioxide is effectual also in the extraction of a phosphorylated compound with a lower molecular weight such as AMP (Adenosine Mono Phosphate). Selective extraction of a phosphorylated amino acid by titanium dioxide was also possible in the same manner. Analysis of a phosphorylated amino acid is necessary for identifying an amino acid phosphorylated in a phosphorylated peptide or phosphorylated protein. One example of a method for identifying a phosphorylated site using titanium dioxide will be shown below.

1. A phosphorylated peptide or phosphorylated protein is hydrolyzed to an amino acid under a certain condition.
2. The mixture is let through a titanium dioxide column to selectively extract a phosphorylated amino acid.
3. The phosphorylated amino acid is eluted from the titanium dioxide column and then detected by a mass spectrometer (the kind and the molecular weight of an amino acid that constitutes a protein is already known, so the amino acid is readily identified by determining the molecular weight of the amino acid that has eluted by the mass spectrometer).

Identification of a phosphorylated site is important for elucidating a function expression mechanism in function analysis or the like as well as for elucidating a higher-order structure.

<Example 2>

Peptide (5) was injected into a titanium dioxide column (Titansphere (registered trademark) TiO) using 0.2 M

phosphate buffer at pH 8.0 (adjusted by 0.2 M disodium hydrogenphosphate and 0.2 M sodium dihydrogenphosphate) as a single eluant and then subjected to HPLC analysis. FIG. 10 shows the diagram of the analysis system used for the HPLC analysis, and FIG. 11 shows the analysis result.

In FIG. 10, Titansphere (registered trademark) TiO (4.6 x 10 mm, from GL Sciences Inc.) was used as the titanium dioxide column. A single eluant in accordance with each experiment was used as a solvent. The HPLC system except the column and the solvent was the same as in Example 1. The flow path of the switching valve was a white one.

In FIG. 11, peptide (5) was eluted in 0.40 minutes. This result suggests that 0.2 M phosphate buffer at pH 8.0 (adjusted by sodium dihydrogenphosphate and disodium hydrogenphosphate) is suitable as a solvent for eluting a phosphorylated peptide from a titanium dioxide column (Titansphere (registered trademark) TiO).

Column: Titansphere (registered trademark) TiO (10 × 4.0 mmI.D.)
Eluant: 0.2 M disodium hydrogenphosphate/0.2 M sodium dihydrogenphosphate = 20/1
Flow rate: 1.0 ml/min
Column temperature: room temperature
Detection wavelength: UV 210 nm
Sample: peptide (5)

<Example 3>

Peptide (5) was injected into a titanium dioxide column (Titansphere (registered trademark) TiO) using 0.05 M disodium hydrogenphosphate at pH 9.0 as a single eluant and then subjected to HPLC analysis. The same analysis system as in Example 2 was used. FIG. 12 shows the analysis result. Column: Titansphere (registered trademark) TiO (10 × 4.0 mmI.D.)
Eluant: 0.05 M disodium hydrogenphosphate
Flow rate: 1.0 ml/min
Column temperature: room temperature
Detection wavelength: UV 210 nm
Sample: peptide (5)

In FIG. 12, peptide (5) was eluted in 0.50 minutes. This result suggests that 0.05 M disodium hydrogenphosphate at pH 9.0 is suitable as a solvent for eluting a phosphorylated peptide from a titanium dioxide column (Titansphere (registered trademark) TiO).

<Example 4>

An eluant was let through a titanium dioxide column (Titansphere (registered trademark) TiO) under following conditions:

- a gradient of from 100% 0.05 M potassium dihydrogenphosphate to 100% 0.05 M dipotassium hydrogenphosphate
- a gradient of from 100% 0.1 M potassium dihydrogenphosphate to 100% 0.1 M dipotassium hydrogenphosphate
- a gradient of from 100% 0.2 M potassium dihydrogenphosphate to 100% 0.2 M dipotassium hydrogenphosphate.

Peptide (5) was injected into the column and subjected to HPLC analysis under the respective elution conditions. FIG. 13 shows the diagram of the analysis system used for the HPLC analysis, and FIGS. 14 to 16 show the analysis results.

In FIG. 13, Titansphere (registered trademark) TiO (4.6 × 10 mm, from GL Sciences Inc.) was used as the titanium dioxide column. Three kinds of, 0.05 M, 0.1 M, and 0.2 M, dipotassium hydrogenphosphate and three kinds of, 0.05 M, 0.1 M, and 0.2 M, potassium dihydrogenphosphate were used as a solvent. The HPLC system except the column and the solvent was the same as in Example 1. The flow path of the switching valve was a white one.

In FIG. 14, peptide (5) was eluted from Titansphere (registered trademark) TiO with a dipotassium hydrogenphosphate ratio of 45 to 50%.

Column: Titansphere (registered trademark) TiO (10 × 4.0 mmI.D.)
Eluant: A: 0.05 M dipotassium hydrogenphosphate
B: 0.05 M potassium dihydrogenphosphate

$$A/B = 0/100 \text{ (0 min)} \rightarrow 100/0 \text{ (10 min)}$$

Flow rate: 1.0 ml/min
Column temperature: room temperature
Detection wavelength: UV 210 nm

Sample: a: peptide (5)
b: none

In FIG. 15, peptide (5) was eluted from Titansphere (registered trademark) TiO with a dipotassium hydrogenphosphate ratio of 13 to 30%.

Column: Titansphere (registered trademark) TiO (10 × 4.0 mmI.D.)
Eluant: A: 0.1 M dipotassium hydrogenphosphate
B: 0.1 M potassium dihydrogenphosphate

$$A/B = 0/100\ (0\ min) \rightarrow 100/0\ (10\ min)$$

Flow rate: 1.0 ml/min
Column temperature: room temperature
Detection wavelength: UV 210 nm
Sample: a: peptide (5)
b: none

In FIG. 16, peptide (5) was eluted from Titansphere (registered trademark) TiO with a dipotassium hydrogenphosphate ratio of 13 to 30%.

Column: Titansphere (registered trademark) TiO (10 × 4.0 mmI.D.)
Eluant: A: 0.2 M dipotassium hydrogenphosphate
B: 0.2 M potassium dihydrogenphosphate

$$A/B = 0/100\ (0\ min) \rightarrow 100/0\ (10\ min)$$

Flow rate = 1.0 ml/min
Column temperature: room temperature
Detection wavelength: UV 210 nm
Sample: a: peptide (5)
b: none

These results suggest that a phosphorylated peptide can be eluted from a titanium dioxide column (Titansphere (registered trademark) TiO) with buffer of a low pH if the phosphate concentration in the buffer is high, and 0.05 to 0.2 M phosphate buffer is suitable as a solvent for eluting a phosphorylated peptide from a titanium dioxide column (Titansphere (registered trademark) TiO). It is also suggested that 0.05 to 0.2 M potassium dihydrogenphosphate can selectively retain a phosphorylated peptide with a titanium dioxide column (Titansphere (registered trademark) TiO).

<Example 5>

Peptides (1) to (6) were injected into a titanium dioxide column (Titansphere (registered trademark) TiO) using 0.1 M phosphate buffer at four pH values of 5.6, 5.9, 6.5, and 7.0 (adjusted by 0.1 M dipotassium hydrogenphosphate and 0.1 M potassium dihydrogenphosphate) as a single eluant and then subjected to HPLC analysis, respectively. The same analysis system as in Example 2 was used. FIGS. 17 to 24 show the analysis results.

In FIG. 17, peptide (1) was eluted in 1.30 minutes, peptide (2) was eluted in 1.75 minutes, peptide (3) was eluted in 2.20 minutes, and peptide (4) was eluted in 4.25 minutes. Column: Titansphere (registered trademark) TiO (10 × 4.0 mmI.D.)

Eluant: 0.1 M dipotassium hydrogenphosphate/0.1 M potassium dihydrogenphosphate = 5/95 (pH 5.6)
Flow rate: 1.0 ml/min.
Column temperature: room temperature
Detection wavelength: UV 210 nm
Sample: peptides (1), (2), (3), (4)

In FIG. 18, peptide (1) was eluted in 0.75 minutes, peptide (2) was eluted in 1.20 minutes, peptide (3) was eluted in 1.30 minutes, and peptide (4) was eluted in 3.15 minutes. Column: Titansphere (registered trademark) TiO (10 × 4.0 mmI.D.)

Eluant: 0.1 M dipotassium hydrogenphosphate/0.1 M potassium dihydrogenphosphate = 10/90 (pH 5.9)
Flow rate: 1.0 ml/min
Column temperature: room temperature
Detection wavelength: UV 210 nm
Sample: peptides (1), (2), (3), (4)

In FIG. 19, peptide (1) was eluted in 0.40 minutes, peptide (2) was eluted in 0.70 minutes, peptide (3) was eluted in 0.70 minutes, and peptide (4) was eluted in 1.70 minutes. Column: Titansphere (registered trademark) TiO (10 × 4.0 mmI.D.)
Eluant: 0.1 M dipotassium hydrogenphosphate/0.1 M potassium dihydrogenphosphate = 30/70 (pH 6.5)
Flow rate: 1.0 ml/min
Column temperature: room temperature
Detection wavelength: UV 210 nm
Sample: peptides (1), (2), (3), (4)

In FIG. 20, peptide (1) was eluted in 0.40 minutes, peptide (2) was eluted in 0.55 minutes, peptide (3) was eluted in 0.55 minutes, and peptide (4) was eluted in 1.05 minutes. Column: Titansphere (registered trademark) TiO (10 × 4.0 mmI.D.)
Eluant: 0.1 M dipotassium hydrogenphosphate/0.1 M potassium dihydrogenphosphate = 58/42 (pH 7.0)
Flow rate: 1.0 ml/min
Column temperature: room temperature
Detection wavelength: UV 210 nm
Sample: peptides (1), (2), (3), (4)

In FIG. 21, peptide (5) was eluted in 0.45 minutes, and peptide (6) was eluted in 0.60 minutes.
Column: Titansphere (registered trademark) TiO (10 × 4.0 mmI.D.)
Eluant: 0.1 M dipotassium hydrogenphosphate/0.1 M potassium dihydrogenphosphate = 5/95 (pH 5.6)
Flow rate: 1.0 ml/min
Column temperature: room temperature
Detection wavelength: UV 210 nm
Sample: peptides (5), (6)

In FIG. 22, peptide (5) was eluted in 0.40 minutes, and peptide (6) was eluted in 0.50 minutes.
Column: Titansphere (registered trademark) TiO (10 × 4.0 mmI.D.)
Eluant: 0.1 M dipotassium hydrogenphosphate/0.1 M potassium dihydrogenphosphate = 10/90 (pH 5.9)
Flow rate: 1.0 ml/min
Column temperature: room temperature
Detection wavelength: UV 210 nm
Sample: peptides (5), (6)

In FIG. 23, peptide (5) was eluted in 0.35 minutes, and peptide (6) was eluted in 0.45 minutes.
Column: Titansphere (registered trademark) TiO (10 × 4.0 mmI.D.)
Eluant: 0.1 M dipotassium hydrogenphosphate/0.1 M potassium dihydrogenphosphate = 30/70 (pH 6.5)
Flow rate: 1.0 ml/min
Column temperature: room temperature
Detection wavelength: UV 210 nm
Sample: peptides (5), (6)

In FIG. 24, peptide (5) was eluted in 0.35 minutes, and peptide (6) was eluted in 0.40 minutes.
Column: Titansphere (registered trademark) TiO (10 × 4.0 mmI.D.)
Eluant: 0.1 M dipotassium hydrogenphosphate/0.1 M potassium dihydrogenphosphate = 58/42 (pH 7.0)
Flow rate: 1.0 ml/min
Column temperature: room temperature
Detection wavelength: UV 210 nm
Sample: peptides (5), (6)

These results suggest that 0.1 M phosphate buffer at pH 5.6 to 7.0 is suitable as a solvent for eluting a phosphorylated peptide from a titanium dioxide column (Titansphere (registered trademark) TiO). It is also found that, when using 0.1 M phosphate buffer as an eluant in a titanium dioxide column (Titansphere (registered trademark) TiO), the buffer at a lower pH causes slower elution and more phosphorylated peptides are eluted faster with respect to peptides having a same amino acid sequence.

<Example 6>

A reversed phase HPLC system for selectively extracting and analyzing a phosphorylated peptide was constructed by utilizing a column switching system using a titanium dioxide column as a precolumn, and peptides (1) to (6) were analyzed. FIG. 25 shows the diagram of the LC system. Table 1 shows eluant conditions used for the analysis. FIG. 26 shows a baseline using eluants shown in Table 1. FIGS. 27 to 32 show the analysis results of peptides (1) to (6).

[Table 1]

| Time (min) | Water (0.1% trifluoroacetic acid) | Acetonitrile (0.1% trifluoroacetic acid) | 0.1 M phosphate buffer (pH 7.0) | Column |
|---|---|---|---|---|
| 0 | 100 | 0 | 0 | Titanium dioxide column → reversed phase HPLC column |
| 4.9 | 100 | 0 | 0 | |
| 5 | 100 | 0 | 0 | Reversed phase HPLC column |
| 20 | 55 | 45 | 0 | |
| 25 | 0 | 100 | 0 | |
| 30 | 0 | 100 | 0 | |
| 30.1 | 100 | 0 | 0 | |
| 39.9 | 100 | 0 | 0 | |
| 40 | 0 | 0 | 100 | Titanium dioxide column → reversed phase HPLC column |
| 59.9 | 0 | 0 | 100 | |
| 60 | 100 | 0 | 0 | Reversed phase HPLC column |
| 70 | 100 | 0 | 0 | |
| 85 | 55 | 45 | 0 | |
| 90 | 0 | 100 | 0 | |
| 95 | 0 | 100 | 0 | |
| 95.1 | 100 | 0 | 0 | |
| 105 | 100 | 0 | 0 | |

In FIG. 25, Titansphere (registered trademark) TiO (10 × 4.6 mmI.D., from GL Sciences Inc.) was used as the titanium dioxide column, and Inertsil (registered trademark) WP300C8 (150 × 4.6 mmI.D., from GL Sciences Inc.) was used as the reversed phase HPLC column. Water containing 0.1% trifluoroacetic acid, acetonitrile containing 0.1% trifluoroacetic acid, and 0.1 M phosphate buffer at pH 7.0 were used as a solvent. The HPLC system except the column and the solvent was the same as in Example 1. The flow path of the switching valve was a white one when using Inertsil (registered trademark) WP300C8 directly connected to Titansphere (registered trademark) TiO as a pretreatment column, and the flow path of the switching valve was a black one when using Inertsil (registered trademark) WP300C8 only.

In FIG. 26, at first, the flow path of the switching valve is the white one. In order to retain a phosphorylated peptide from a sample injected at 0 minute in Titansphere (registered trademark) TiO and to retain a peptide that passes straight through Titansphere (registered trademark) TiO in Inertsil (registered trademark) WP300C8, water containing 0.1% trifluoroacetic acid is let through Titansphere (registered trademark) TiO and Inertsil (registered trademark) WP300C8 for 0 to 4.9 minutes. Then, in order to elute the peptide retained in Inertsil (registered trademark) WP300C8, the switching valve is changed to the black one, and a gradient is applied to Inertsil (registered trademark) WP300C8 to have 45% acetonitrile containing 0.1% trifluoroacetic acid for 5 to 20 minutes. In order to wash Inertsil (registered trademark) WP300C8, a gradient is applied to Inertsil (registered trademark) WP300C8 to have 100% acetonitrile containing 0.1% trifluoroacetic acid for 20 to 25 minutes, and further acetonitrile containing 0.1% trifluoroacetic acid is let through Inertsil (registered trademark) WP300C8 for 25 to 30 minutes. In order to remove an organic solvent from Inertsil (registered trademark) WP300C8, water containing 0.1% trifluoroacetic acid is let through Inertsil (registered trademark) WP300C8 for 30.1 to 39. 9 minutes. In order to elute a phosphorylated peptide from Titansphere (registered trademark) TiO and to retain a phosphorylated peptide in Inertsil (registered trademark) WP300C8, the flow path of the switching valve is changed to the white one, and 0.1 M phosphate buffer at pH 7.0 is let through Titansphere (registered trademark) TiO and Inertsil (registered trademark) WP300C8 for 40 to 59.9 minutes. In order to remove phosphate buffer from Inertsil (registered trademark) WP300C8, the flow path of the switching valve is changed to the black one, and water containing 0.1% trifluoroacetic acid is let through Inertsil (registered trademark) WP300C8 for 60 to 70 minutes. In order to elute the phosphorylated peptide retained in Inertsil (registered trademark) WP300C8, a gradient is applied to Inertsil (registered trademark) WP300C8 to have 45% acetonitrile containing 0.1% trifluoroacetic acid for 70 to 85 minutes. In order to wash Inertsil (registered trademark) WP300C8, a gradient is applied to Inertsil (registered trademark) WP300C8 to have 100% acetonitrile containing 0.1% trifluoroacetic acid for 85 to 90 minutes, and further acetonitrile containing 0.1% trifluoroacetic acid is let through Inertsil (registered trademark) WP300C8 for 90 to 95 minutes. In order to remove an organic solvent from Inertsil (registered trademark) WP300C8, water containing 0.1% trifluoroacetic acid is let through Inertsil (registered trademark) WP300C8 for 95.1 to 105 minutes.

Column: a: Inertsil (registered trademark) WP300C8 (150 × 4.6 mmI.D.)

b: Titansphere (registered trademark) TiO (10 × 4.0 mmI.D.) → Inertsil (registered trademark) WP300C8 (150 × 4.6 mmI.D.)

Eluant: see Table 1
Flow rate: 1.0 ml/min
Column temperature: room temperature
Detection wavelength: UV 210 nm
Sample: none

In FIG. 27, phosphorylated peptide (1) is retained in and eluted through a reversed phase HPLC column after being retained in and eluted through a titanium dioxide column.

Column: a: Inertsil (registered trademark) WP300C8 (150 × 4.6 mmI.D.)

b: Titansphere (registered trademark) TiO (10 × 4.0 mmI.D.) → Inertsil (registered trademark) WP300C8 (150 × 4.6 mmI.D.)

Eluant: see Table 1
Flow rate: 1.0 ml/min
Column temperature: room temperature
Detection wavelength: UV 210 nm
Sample: peptide (1)

In FIG. 28, phosphorylated peptide (2) is retained in and eluted through a reversed phase HPLC column after being retained in and eluted through a titanium dioxide column.

Column: a: Inertsil (registered trademark) WP300C8 (150 × 4.6 mmI.D.)

b: Titansphere (registered trademark) TiO (10 × 4.0 mmI.D.) → Inertsil (registered trademark) WP300C8 (150 × 4.6 mmI.D.)

Eluant: see Table 1
Flow rate: 1.0 ml/min
Column temperature: room temperature
Detection wavelength: UV 210 nm

Sample: peptide (2)

In FIG. 29, phosphorylated peptide (3) is retained in and eluted through a reversed phase HPLC column after being retained in and eluted through a titanium dioxide column.

Column: a: Inertsil (registered trademark) WP300C8 (150 × 4.6 mmI.D.)
b: Titansphere (registered trademark) TiO (10 × 4.0 mmI.D.) → Inertsil (registered trademark) WP300C8 (150 × 4.6 mmI.D.)
Eluant: see Table 1
Flow rate: 1.0 ml/min
Column temperature: room temperature
Detection wavelength: UV 210 nm
Sample: peptide (3)

In FIG. 30, peptide (4) is not retained in a titanium dioxide column, but is retained in and eluted through a reversed phase HPLC column.

Column: a: Inertsil (registered trademark) WP300C8 (150 × 4.6 mmI.D.)
b: Titansphere (registered trademark) TiO (10 × 4.0 mmI.D.) → Inertsil (registered trademark) WP300C8 (150 × 4.6 mmI.D.)
Eluant: see Table 1
Flow rate: 1.0 ml/min
Column temperature: room temperature
Detection wavelength: UV 210 nm
Sample: peptide (4)

In FIG. 31, phosphorylated peptide (5) is retained in and eluted through a reversed phase HPLC column after being retained in and eluted through a titanium dioxide column.

Column: a: Inertsil (registered trademark) WP300C8 (150 × 4.6 mmI.D.)
b: Titansphere (registered trademark) TiO (10 × 4.0 mmI.D.) → Inertsil (registered trademark) WP300C8 (150 × 4.6 mmI.D.)
Eluant: see Table 1
Flow rate: 1.0 ml/min
Column temperature: room temperature
Detection wavelength: UV 210 nm
Sample: peptide (5)

In FIG. 32, peptide (6) is not retained in a titanium dioxide column, but is retained in and eluted through a reversed phase HPLC column.

Column: a: Inertsil (registered trademark) WP300C8 (150 × 4.6 mmI.D.)
b: Titansphere (registered trademark) TiO (10 × 4.0 mmI.D.) → Inertsil (registered trademark) WP300C8 (150 × 4.6 mmI.D.)
Eluant: see Table 1
Flow rate: 1.0 ml/min
Column temperature: room temperature
Detection wavelength: UV 210 nm
Sample: peptide (6)

These results show that it is possible to selectively extract and analyze a phosphorylated peptide online by using the constructed HPLC system.

<Example 7>

Using a trypsinized casein that is a phosphorylated protein contained in milk or the like as a sample, HPLC analysis was performed with a reversed phase HPLC column (Inertsil (registered trademark) WP300C8) and Inertsil (registered trademark) WP300C8 with a titanium dioxide column (Titansphere (registered trademark) TiO) as a precolumn under a gradient condition of from 100% water containing 0.1% trifluoroacetic acid to an aqueous 45% acetonitrile solution containing 0.1% trifluoroacetic acid. The same HPLC analysis system as in Example 1 was used.

Next, after injecting a trypsinized casein to a titanium dioxide column (Titansphere (registered trademark) TiO) under a condition in which water containing 0.1% trifluoroacetic acid was used as a single solvent, 0.05 M borate buffer containing 0.05 M lithium chloride (pH 8.0) was used to elute a peptide from the titanium dioxide column (Titansphere (registered trademark) TiO) and then the peptide was fractionated. FIG. 33 shows the diagram of the HPLC system used for the fractionation. The fractionated peptide was subjected to HPLC analysis by a reversed phase HPLC column (Inertsil (registered trademark) WP300C8) under a gradient condition of from 100% water containing 0.1% trifluoroacetic acid to an aqueous 45% acetonitrile solution containing 0.1% trifluoroacetic acid. The same HPLC analysis system as in Example 1 was used. FIG. 34 shows the analysis results.
Column: a and c: Inertsil (registered trademark) WP300C8 (150 × 4.6 mmI.D.)
b: Titansphere (registered trademark) TiO (10 × 4.0 mmI.D.) → WP300C8 (150 × 4.6 mmI.D.)
Eluant: A: water (0.1% trifluoroacetic acid)
B: acetonitrile (0.1% trifluoroacetic acid)

$$A/B = 100/0\ (0\ min) \rightarrow 55/45\ (90\ min)$$

Flow rate: 1.0 ml/min
Column temperature: room temperature
Detection wavelength: UV 210 nm
Sample: a and b: trypsinized casein
c: peptide in trypsinized casein retained in and eluted through titanium dioxide column

In order to show that the fractionated peptide was a phosphorylated peptide, the following experiment was performed. The fractionated peptide was separated into two vessels: one was a control and the other was subjected to a dephosphorylation treatment using alkaline phosphatase. Each was subjected to HPLC analysis by a reversed phase HPLC column (Inertsil (registered trademark) WP300C8) under a gradient condition of from 100% water containing 0.1% trifluoroacetic acid to an aqueous 45% acetonitrile solution containing 0.1% trifluoroacetic acid. Comparison of the two data confirmed that the peaks were shifted by the alkaline phosphatase treatment. The same HPLC analysis system as in Example 1 was used. FIG. 35 shows the analysis results. Column: a and b: Inertsil (registered trademark) WP300C8 (150 × 4.6 mmI.D.)
Eluant: A: water (0.1% trifluoroacetic acid)
B: acetonitrile (0.1% trifluoroacetic acid)

$$A/B = 100/0\ (0\ min) \rightarrow 55/45\ (90\ min)$$

Flow rate: 1.0 ml/min
Column temperature: room temperature
Detection wavelength: UV 210 nm
Sample: a: peptide in trypsinized casein retained in and eluted through titanium dioxide column (control of alkaline phosphatase treatment)
b: sample (a) subjected to alkaline phosphatase treatment

In order to show that the fractionated peptide was a phosphorylated peptide, the following experiment was performed. The trypsinized casein was separated into two vessels: one was a control and the other was subjected to a dephosphorylation treatment using alkaline phosphatase. Each was subjected to HPLC analysis by a reversed phase HPLC column (Inertsil (registered trademark) WP300C8) under a gradient condition of from 100% water containing 0.1% trifluoroacetic acid to an aqueous 45% acetonitrile solution containing 0.1% trifluoroacetic acid. Comparison of the two data confirmed that three peaks were shifted by the alkaline phosphatase treatment. The same HPLC analysis system as in Example 1 was used. FIG. 36 shows the analysis results.
Column: a, b, and c: Inertsil (registered trademark) WP300C8 (150 × 4.6 mmI.D.)
Eluant: A: water (0.1% trifluoroacetic acid)
B: acetonitrile (0.1% trifluoroacetic acid)

$$A/B = 100/0\ (0\ min) \rightarrow 55/45\ (90\ min)$$

Flow rate: 1.0 ml/min
Column temperature: room temperature
Detection wavelength: UV 210 nm
Sample: a: trypsinized casein (control of alkaline phosphatase treatment)
b: trypsinized casein subjected to alkaline phosphatase treatment
c: peptide in trypsinized casein retained in and eluted through titanium dioxide column

In FIG. 33, Titansphere (registered trademark) TiO (10 x 4.6 mmI.D., from GL Sciences Inc.) was used as the titanium dioxide column. Water containing 0.1% trifluoroacetic acid and 0.05 M borate-sodium hydroxide buffer containing 0.05 M lithium chloride (pH 8.0) was used as a solvent. In the HPLC system, HITACHI Pump L-7120 was used as a pump 31, HITACHI Pump L-7100 as a pump 32, HITACHI Diode Array Detector L-7455 as a detector, HITACHI

SYSTEM MANAGER as a data processor (all above, from HITACHI), RHEODYNE 8125 (from Rheodyne) as an injector, and SSC E1E010 (Senshu Scientific co., ltd.) as a switching valve. PEEK (registered trademark) was used for all the sample loops and pipings. When using water containing 0.1% trifluoroacetic acid to Titansphere (registered trademark) TiO as a single solvent, the flow path of the switching valve was a white one. When a peptide was eluted from Titansphere (registered trademark) TiO using 0.05 M borate buffer containing 0. 05 M lithium chloride (pH 8.0) and then fractionated, the flow path of the switching valve was changed to a black one.

In FIG. 34, for a trypsinized casein, the peak strengths indicated by arrows were decreased when using Titansphere (registered trademark) TiO as a precolumn. Further, the peaks of the peptide eluted from Titansphere (registered trademark) TiO with 0.05 M borate buffer containing 0.05 M lithium chloride (pH 8.0) were coincident with the strength-decreased peaks.

In FIG. 35, three peptides out of a trypsinized casein that was retained in and eluted through Titansphere (registered trademark) TiO is increased in the hydrophobicity, and the peaks have shifted.

In FIG. 36, the peaks that were shifted by subjecting the trypsinized casein to an alkaline phosphatase treatment are roughly coincident with the peaks of a peptide in a trypsinized casein that was retained in and eluted through Titansphere (registered trademark) TiO.

These results show that, among peptides in the trypsinized casein, a peptide that was assumed to be a phosphorylated peptide was selectively retained in a titanium dioxide column (Titansphere (registered trademark) TiO) and eluted with 0.05 M borate buffer containing 0.05 M lithium chloride (pH 8.0).

<Example 8>

Examples using titanium dioxide as a solid phase include a column with a following shape.

A column described here is a chromatograph tube that has been in a condition where column chromatography can be performed by filling packing material, coating the inner surface with a solid phase, introducing a chemical bonding group to inner surface, or a treatment such as corrosion. The configuration is not to be particularly limited, but any configuration is acceptable as long as it is a vessel having two openings in which a solid phase can be filled or held, and that has a configuration so as to allow a solution to enter from one of the openings, pass through the solid phase, and discharge from the other opening. Therefore, it may have a configuration of a column for solid phase extraction as shown in FIGS. 37 to 39 as well as of a general LC column.

(Description on form of solid phase)

A solid phase described here is a term against a mobile phase in chromatography, which has affinity to a substance and does not move. The form is not to be particularly limited, but any form is acceptable as long as a solution containing a desired ingredient can contact with titanium dioxide in the solid phase. Therefore, the form may be a porous spherical particle, a porous fractured particle, a non-porous spherical particle, a non-porous fractured particle and a steric continuous form having a through hole, each of which is made from titanium dioxide; a porous spherical particle, a porous fractured particle, a non-porous spherical particle, a non-porous fractured particle and a steric continuous form having a through hole, each of which is made from complex oxide of titanium dioxide and other metal oxide; and a porous spherical particle, a porous fractured particle, a non-porous spherical particle, a non-porous fractured particle and a steric continuous form having a through hole, each of which is made from a substance other than titanium dioxide and provided with titanium dioxide coating on the surface that contacts with a solution. The steric continuous form having a through hole described here is a solid body made from an ingredient or a plurality of ingredients and having a cavity that runs through from one side to the other side. The one example is a monolith skeleton. The monolith skeleton is titanium dioxide of double pore structure in which a titanium dioxide skeleton having pores of 0. 5 to 500 μm in size (through pore) and pores of 2 to 500 nm in size (meso pore) and a polymer skeleton are entangled to each other (for example, Naohiro Soga, Kazuki Nakanishi, Hiroyoshi Mizuguchi; Japanese Patent Laid-Open No. 11-287791).

(Description on how to use)

For example, protein hydrolysate by a digestive enzyme such as trypsin is dissolved in water containing 0.1% trifluoroacetic acid and allowed to contact with a solid phase by a method such as injection or suction so that the solid phase selectively retains a phosphorylated peptide. After washing the solid phase by a method such as injection or suction using water containing 0.1% trifluoroacetic acid, 0.1 M phosphate buffer at pH 7.0 is made contact with the solid phase by a method such as injection or suction to elute a phosphorylated peptide (a solvent for selectively retainding a phosphorylated peptide in the solid phase, a solvent for washing the solid phase, and the solvent for eluting a phosphorylated peptide from the solid phase are not limited to the above examples, but solvents used in Examples

1 to 7 are also included).

The eluted phosphorylated peptide is retained in the reversed phase solid phase, demineralized, and eluted with an organic solvent. The eluted peptide is subjected to qualitative analysis and quantitative determination with an analytical instrument such as MS. When the peptide is eluted with a solvent (for example, phosphoric acid) that does not require a demineralization operation, the peptide can also be directly detected by MS or the like using a MALDI plate or the like.

Further, the present method can be duly applicable also to the following proteins:

JAK (Janus Kinase) family protein, STAT (signal transducer and activator of transcription) family protein, TGF-β (transforming growth factor β) family protein, Smad family protein, and other signaling factors that are activated or inactivated by phosphorylation.

Next, experiment results confirmed that, for both of a phosphorylated sugar and a phospholipid, only phosphorylated compounds were retained in a titania column, not passing through the column, by applying a phosphorylated compound and a non-phosphorylated compound to the titania column under an acidic condition, collecting a fraction passed through, analyzing the fraction by respective analysis columns, and comparing with previously collected data of the mixture of the phosphorylated compound and the non-phosphorylated compound.

Next, it was confirmed that a phosphorylated compound was eluted, by letting an elution solvent containing a phosphorylated compound through a titania column in which a phosphorylated compound had been retained, collecting the fraction, analyzing the fraction by respective analysis columns, and comparing with the data of the mixture as described in the above.

First, in the experiment on sugar, a saccharide, glucose, to which one phosphate group was attached (two types) was compared with glucose. At the beginning, in Example 1, a sugar mixture was applied under the condition with which the phosphorylated peptide had been retained ($TFA/H_2O$ = 1/1000, about pH 2), but both of a phosphorylated sugar and a non-phosphorylated sugar were passed through. The condition was changed to HCl solution (pH 0.88), and then only the phosphorylated sugar could be retained. Further, the elution was possible with 0. 05 M $NaH_2PO_4$ (about pH 4.5). Since $pK_1$ of the phosphorylated sugar that was used this time is 1.11, it is considered that a solution having pH above that cannot selectively retain the phosphorylated sugar. At this time, dissociation of the phosphate group attached to the sugar is prevented and the phosphate group should have two OH groups. It is shown that the elution condition may be acceptable in which the pH is not particularly around 7 and other buffer than potassium phosphate buffer used in the peptide experiment can be used.

The number of the phosphorylated peptides that were retained was smaller when using 20 mM Tris-HCl buffer at about pH 7.0 comparing with when using a 0.1% TFA aqueous solution at about pH 2.0. Further, the phosphorylated sugar ($pK_1$ = 1.11) was not retained in the titanium dioxide column when using a 0.1% TFA aqueous solution at about pH 2.0, but retained when using an HCl aqueous solution at about pH 0.8.

Consequently, in order to retain a phosphorylated compound, a condition is required in which dissociation of a phosphate part of the phosphorylated compound is prevented.

For the elution from a titanium dioxide column, each experiment shows that the elution effect is enhanced as the phosphate concentration or the pH during elution increases.

<Example 9>

Followings are used as a sample.

(1) D(+)-glucose KISHIDA CHEMICAL CO., LTD. 000-34105 D(+)-Glucose FW:180.16 13.6 mg/500 µl $H_2O$ 40 µl
The chemical formula of one sample is shown:

(chemical formula 1)

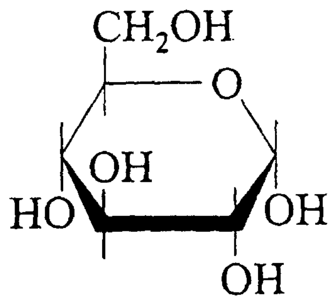

(2) α-D-glucose-1-phosphate Sigma G7000 α-D-Glucose 1-phosphate Disodium Salt Hydrate FW:304.1 20.2 mg/ 300 μl $H_2O$ 40 μl
The chemical formula of one sample is shown:

(chemical formula 2)

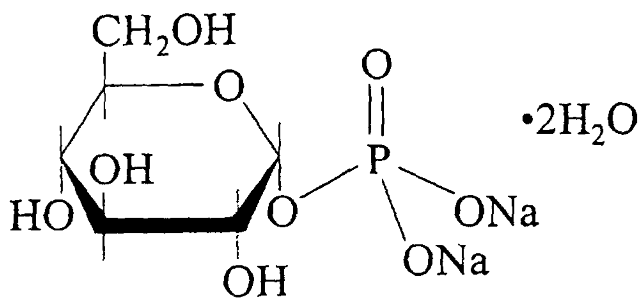

(3) D-glucose-6-phosphate Sigma G7250
D-Glucose 6-phosphate Disodium Salt Hydrate FW:304.1 21.1 mg/300 μl $H_2O$ 40 μl The chemical formula of one sample is shown:

(chemical formula 3)

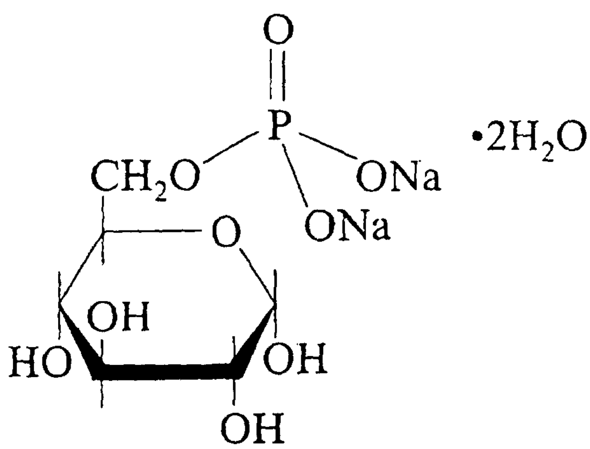

The above (1) to (3) were mixed.

Experiment procedure:

To Titansphere (registered trademark) TiO (10 x 4.0 mmI.D.) to which an aqueous HCl solution (pH 0.88) is let through, 40 μl of the sample mixture is applied, and the fractions that pass through are collected. This fraction is 1.
The solvent is changed to 50 mM $NaH_2PO_4$, and the fractions eluted from Titansphere (registered trademark) TiO are fractionated. These fractions are 2 to 5. (FIG. 41 is a chromatogram showing respective positions of the fractions.)
The fractions are subjected to chromatography using an ion exchange mode column, Asahipak (registered trademark) NH2P-50 4E. Comparison with the chromatogram of the sample mixture shows that the phosphorylated sugar that is used as a sample here can be selectively extracted by use of Titansphere (registered trademark) TiO.
Column: Titansphere (registered trademark) TiO (10 × 4.0 mmI.D.)
Eluant: A. dilute hydrochloric acid (pH 0.88)
B. 50 mM $NaH_2PO_4$ (about pH 4.0)
0-3 min A/B = 100/0
3.1-12 min A/B = 0/100
Flow rate: 1.0 ml/min
Column temperature: room temperature
Detection wavelength = UV 260 nm
Sample: adjusted sugar mixtures (1), (2), (3) 40 μl
In FIG. 42, two phosphorylated sugars confirmed in the sample mixture are confirmed in fractions 2 to 5. Therefore, it is understood that this separation method is effective for extracting and separating the phosphorylated sugar.
Column: Asahipak (registered trademark) NH2P-50 4E (250 × 4.0 mmI.D.)
Eluant: 50 mM $NaH_2PO_4$ (about pH 4.0)
Flow rate: 1.0 ml/min
Column temperature: room temperature
Detection: RI
Sample: aforementioned adjusted sugar mixture 25 μl fractions of 1 to 5 each 50 μl
Moreover, this method can be completely applicable also to the following sugar-related substances.
Fructose 6-phosphate, fructose 1,6-bisphosphate, D-glucono-1,5-lactone phosphate, 6-phospho-D-gluconate, D-ribulose-5-phosphate, D-xylulose-5-phosphate, D-ribose-5-phosphate, D-sedoheptulose-7-phosphate, D-erythrose-4-phosphate, phosphoglycolate, D-ribulose-5-phosphate, D-ribulose-1,5-bisphosphate, sedoheptulose-1,7-bisphosphate, ADP glucose, UDP glucose, UDP glucuronate, 1-phospho-α-D-glucuronate, D-xylulose-5-phosphate, and sucrose-6-phosphate.
The same applies to a water-soluble low-molecular-weight phosphorylated compound.
D-glyceraldehyde 3-phosphate, dihydroxyacetone phosphate, glucosamine 6-phosphate, sn-glycerol 3-phosphate, 1,3-bisphosphoglycerate, 2,3-bisphosphoglycerate, 3-phosphoglycerate, 2-phosphoglycerate, phosphoenolpyruvate, GMP, GDP, GTP, AMP, ADP, ATP, CAMP, UMP, UDP, UTP, and pyrophosphate.
Next, the experiment for lipid clarified that an organic solvent added with TFA can selectively retain a non-water-soluble sample, and an organic solvent added with phosphate can elute the non-water-soluble sample. Therefore, it was also shown that not only water-soluble samples, but also low-polarity phosphorylated compound could be selectively retained and eluted. Further, it was also shown that not only compounds having a phosphate group at the end, but also esters could be selectively retained and eluted.
For example, sphingomyelin (chemical formula 6) has phosphate in an ester form, but the experiment result confirmed that sphingomyelin was selectively retained. Moreover, separation and analysis are possible also for L-α-phosphatidyl inositol (chemical formula 7), L-α-phosphatidyl ethanolamine (chemical formula 8), L-α-phosphatidylcholine (chemical formula 9), L-α-phosphatidyl-L-serine (chemical formula 10), L-α-phosphatidyl-L-threonine (chemical formula 11), and L-α-phosphatidate (chemical formula 12).

(chemical formula 6)

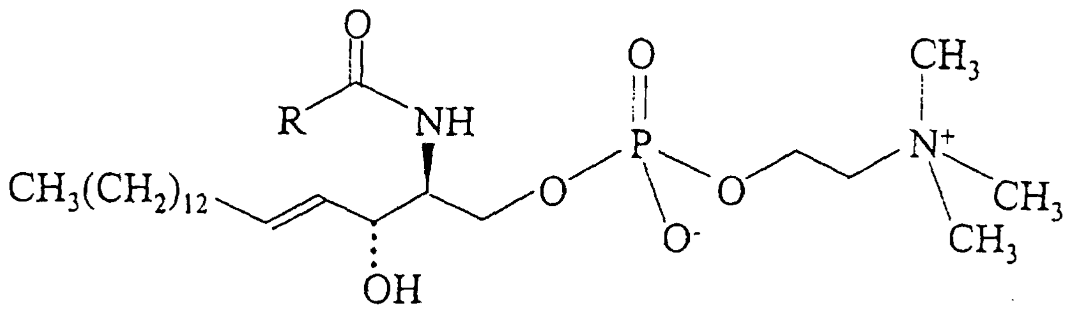

(chemical formula 7)

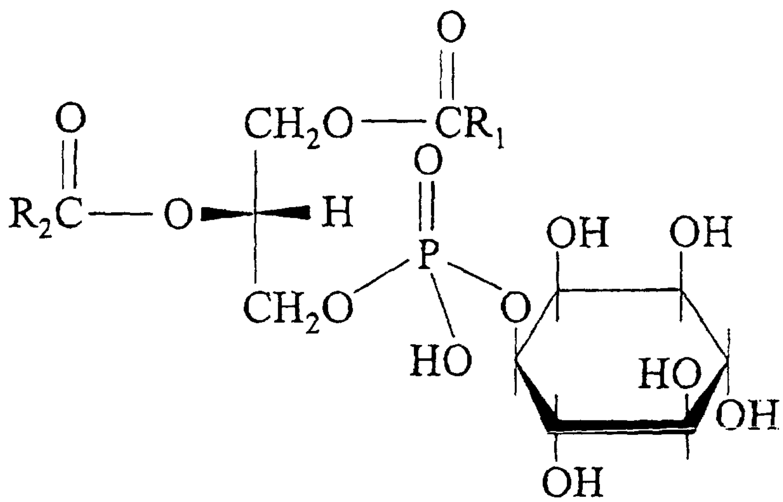

(chemical formula 8)

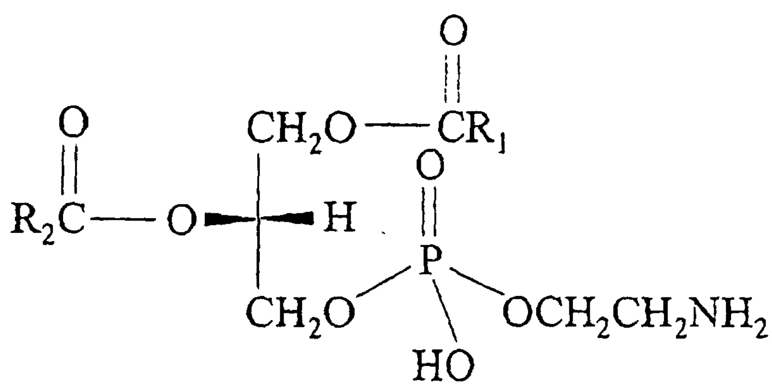

(chemical formula 9)

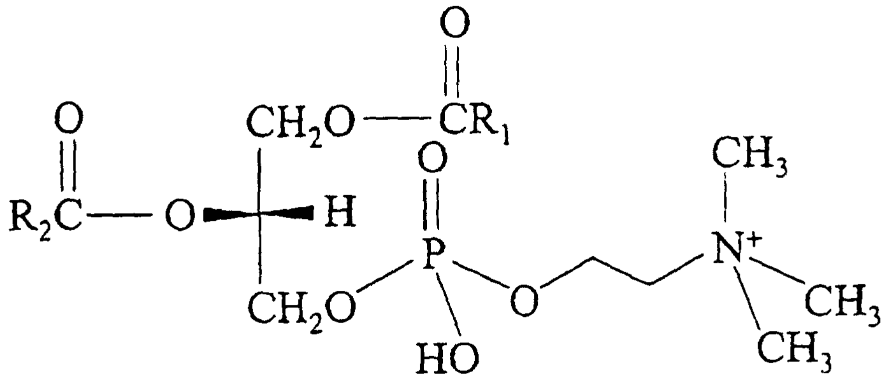

(chemical formula 10)

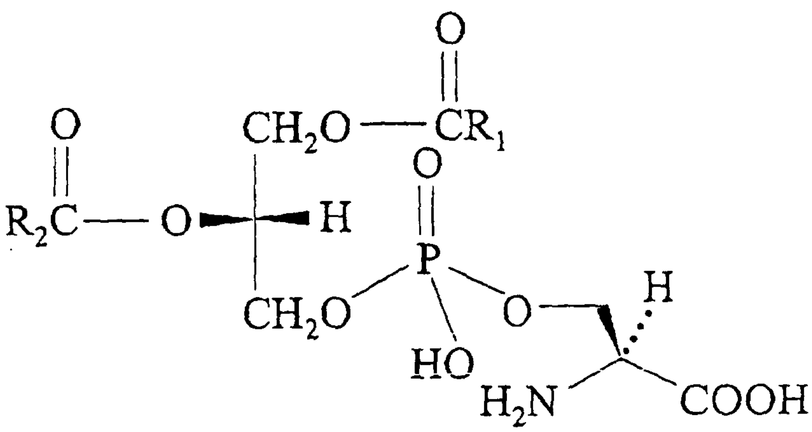

(chemical formula 11)

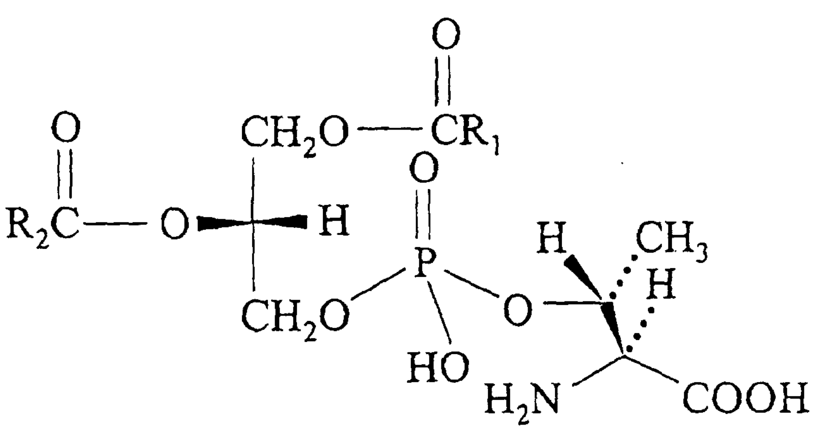

(chemical formula 12)

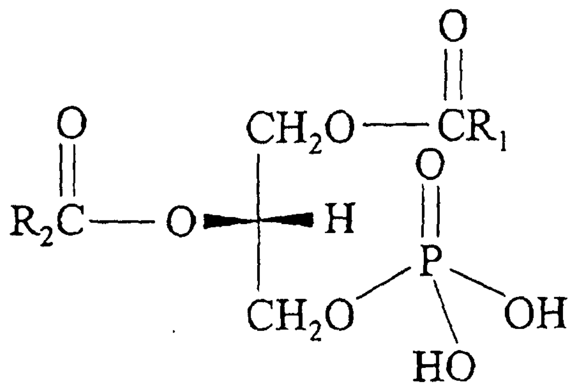

<Example 10>

Following samples are used.

(1) D-erythro-sphingosine Wako 192-11161 D-erythro-Sphingosine FW:299.5 1.6 mg/1,000 μl MeOH 100 μl

(chemical formula 4)

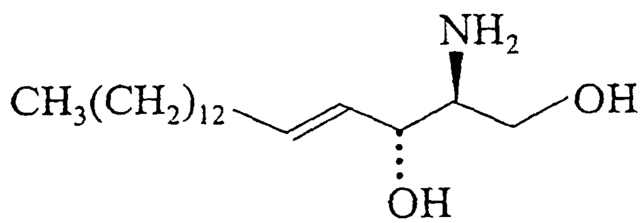

(2) Sphingosine-1-phosphate Sigma S9666 Sphingosine 1-phosphate FW:379.5 1.0 mg/1,000 μl MeOH 100 μl

(chemical formula 5)

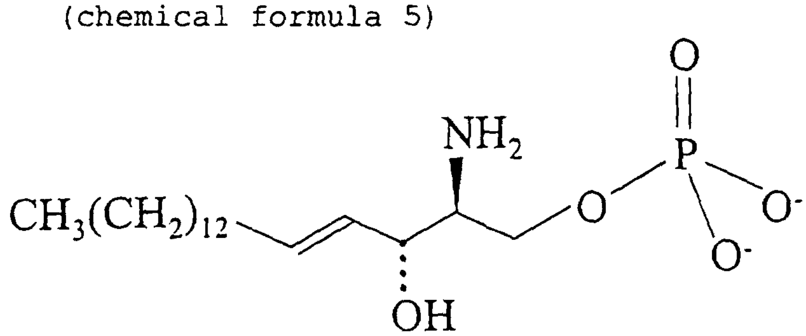

The above (1) and (2) were mixed.

Experiment procedure:

To Titansphere (registered trademark) TiO (10 × 4.0 mmI.D.) to which a $H_2O$ (0.05% TFA) :MeOH = 30:70

mixed solution is let through, 160 µl of the adjusted sample mixture is applied, and the fractions that pass through are collected. These fractions are 1 and 2.

The solvent is changed to a $H_2O$:MeOH:$H_3PO_4$ = 30:70:5 solution, and the fractions eluted from Titansphere (registered trademark) TiO are fractionated. These fractions are 3 to 9. (FIG. 43 is a chromatogram showing respective positions of the fractions.)

Column: Titansphere (registered trademark) TiO (10 × 4.0 mmI.D.)
Eluant: A: $H_2O$(0.05% TFA):MeOH = 30:70
B: $H_2O$:MeOH:$H_3PO_4$ = 30:70:5
0-3.9 min A/B = 100/0
4-18 min A/B = 0/100
Flow rate = 1.0 ml/min
Column temperature: 25°C
Detection wavelength: UV 205 nm
Sample : adjusted lipid mixture 160 µl

The fractions are subjected to chromatography using Inertsil (registered trademark) ODS-3 (4.6 mmI.D. × 150). Comparison with the chromatogram of the adjusted sample mixture shows that the phospholipid (sphingosine 1-phosphate) that is used as a sample here can be selectively extracted by use of Titansphere (registered trademark) TiO.

FIG. 44 is a chromatogram comparing the fractions to each other. The experiment data will be shown below.

Column: Inertsil (registered trademark) ODS-3 (4.6 mmI.D. × 150)
Eluant:A: $H_2O$ (0.05% TFA)
B: MeOH
0-2 min A/B = 70/30
2-12 min A/B = 70/30 → 0/100
12-17 min A/B = 0/100
17.1-22 min A/B = 70/30
Flow rate: 1.0 ml/min
Column temperature: 25°C
Detection wavelength: UV 205 nm
Sample: aforementioned adjusted lipid mixture 10 µl fractions of 1 to 10 each 200 µl

FIGS. 45, 46, and 47 are chromatograms detailing the above.

These chromatograms clearly show that sphingosine 1-phosphate is extracted.

<Example 11>

Following samples are used.

(1) D-erythro-sphingosine Wako 192-11161
D-erythro-Sphingosine FW:299.5
1.8 mg/500 µl MeOH 40 µl
(2) Sphingomyelin Sigma S9666
Sphingomyelin (FW deficient due to fatty-acid residue side chain)
1.8 mg/500 µl MeOH 40 µl

The above (1) and (2) were mixed.

Experiment procedure:

To Titansphere (registered trademark) TiO (10 × 4.0 mmI.D.) to which a $H_2O$ (0.1% TFA) : $CH_3CN$:MeOH = 8:82:10 mixed solution is let through, 50 µl of the adjusted sample mixture is applied, and the fractions that pass through are collected. These fractions are 1 and 2.

The solvent is changed to a $H_2O$:$CH_3CN$:MeOH:$H_3PO_4$ = 8:82:10:0.6, and the fractions eluted from Titansphere (registered trademark) TiO are fractionated. These fractions are 3 to 10. (FIG. 48 is a chromatogram showing respective positions of the fractions.)

Column: Titansphere (registered trademark) TiO (10 × 4.0 mmI.D.)
Eluant: A: $H_2O$(0.1% TFA):$CH_3CN$:MeOH = 8:82:10
B: $H_2O$:$CH_3CN$:MeOH:$H_3PO_4$ = 8:82:10:0.6
0-2.9 min A/B = 100/0

3-20 min A/B = 0/100
Flow rate: 1.0 ml/min
Column temperature: room temperature
Detection wavelength: UV 210 nm
Sample: adjusted lipid mixture 50 µl

The fractions are subjected to chromatography using Inertsil (registered trademark) $NH_2$ (4.6 mmI.D. × 150). Comparison with the chromatogram of the adjusted sample mixture shows that the phospholipid (sphingomyelin) that is used as a sample here can be selectively extracted by use of Titansphere (registered trademark) TiO.

FIG. 49 is a chromatogram comparing the factions to each other. The experiment data will be shown below.
Column: Inertsil (registered trademark) $NH_2$ (4.6 mmI.D. × 150)
Eluant: A: $H_2O$: $CH_3CN$: MeOH = 8:87:5
Flow rate: 1.0 ml/min
Column temperature: room temperature
Detection wavelength: UV 205 nm
Sample: aforementioned adjusted lipid mixture diluted by the factor of 66 with the eluant 50 µl
fractions of 1 to 10 each 50 µl

FIGS. 50 and 51 are chromatograms detailing the above.

These chromatograms clearly show that sphingomyelin is extracted.

Further, this method can be completely applicable also to the following phospholipid-related substances.

Glycerophospholipids (for example, phosphatidyl inositol), sphingophospholipids, and triose phosphate.

Moreover, this method can be completely applicable also to the following in-vivo-metabolism-related substances due to the properties.

CoA (coenzyme A), acetyl CoA, succinyl CoA, acyl CoA, $NAD(P)^+$, NAD(P)H, $NAD^+$, NADH, FAD, $FADH_2$, and FADH.

## Industrial Applicability

The method of claim 1 of the present invention comprises the steps of: retaining a phosphorylated compound in which a sample containing a phosphorylated amino acid, phosphorylated peptide, phosphorylated protein, phosphorylated sugar, or phosphorylated lipid is retained in a titanium dioxide column as a pretreatment column under an acidic condition; detecting a non-phosphorylated compound in which an amino acid, peptide, protein, sugar, or lipid that has not been retained is separated and purified through a desired column and then detected with a desired detector, or the compound is detected with the desired detector without purification, or the compound is not either separated and purified or detected; eluting the phosphorylated compound in which the phosphorylated amino acid, phosphorylated peptide, phosphorylated protein, phosphorylated sugar, or phosphorylated lipid retained in the pretreatment column is eluted by a solution containing phosphate, phosphoric acid, or alkali; and detecting the phosphorylated compound in which the compound is separated and purified through the desired column and then detected with the desired detector, or the compound is detected with the desired detector without purification. Therefore, this method makes it possible to achieve effects such as the speed improvement, simplification of experimental operations, and risk prevention for analysis of a phosphorylated amino acid, a phosphorylated peptide, a phosphorylated protein, a phosphorylated sugar, and a phosphorylated lipid in academic and industrial fields, by selectively extracting/separating a phosphorylated substance such as a phosphorylated amino acid, phosphorylated peptide, phosphorylated protein, phosphorylated sugar, or phosphorylated lipid in a simple and rapid manner, leading to a great benefit to industry, especially to the food and pharmaceutical industries. Further, this method makes it possible to discharge ingredients from a pretreatment column. At that time, an elution step from the column and also unsurprisingly a detection step can be omitted so that it remains only steps for necessary ingredients, leading to acceleration and simplification of the operations.

The method of claim 2 of the present invention comprises the steps of: passing a sample containing a phosphorylated amino acid, phosphorylated peptide, or phosphorylated protein in an acidic solution at a pH of about 2 through a reversed phase HPLC column with a titanium dioxide column as a pretreatment column, selectively retaining the phosphorylated amino acid, phosphorylated peptide, or phosphorylated protein in a precolumn, purifying a non-phosphorylated amino acid, peptide, or protein that has not been retained in the above column by the reversed phase HPLC column followed by detection thereof by a desired detector; and eluting the phosphorylated amino acid, phosphorylated peptide, or phosphorylated protein retained in the precolumn with buffer followed by detection thereof. Therefore, this method makes it possible to select a phosphorylated amino acid or the like from a sample by subjecting the sample containing a phosphorylated amino acid, phosphorylated peptide, or phosphorylated protein to this process. That is, this series of steps makes it possible to perform extraction, separation, purification, qualitative analysis, quantitative determination, or the like of a desired ingredient such as a phosphorylated amino acid, phosphorylated peptide, or phosphorylated protein by an extremely simple operation without requiring conventional complicated and difficult

operations such as removal of contaminating substances and fractionation of eluted ingredients by selectively retaining the desired ingredient such as the phosphorylated amino acid in a titania column as a precolumn; eluting other ingredients and sending them to the reversed phase HPLC column; performing an operation such as detection; eluting the desired ingredient; and retaining the desired ingredient in the reversed phase HPLC column; performing an operation such as detection.

In claim 3 of the present invention, a sample containing a phosphorylated amino acid, phosphorylated peptide, phosphorylated protein, phosphorylated sugar, or phospholipid is retained in a titanium dioxide column as a pretreatment column under an acidic condition and then fractionated directly for use without transferring the phosphorylated compound eluted in an elution step to a detection step. Therefore, this method makes it possible to use the selectively extracted phosphorylated amino acid, phosphorylated peptide, phosphorylated protein, phosphorylated sugar, or phospholipid in a biochemical assay more rapidly by eliminating the detection step.

The method of claim 4 of the present invention comprises the steps of: retaining a phosphorylated compound in which a sample containing the phosphorylated amino acid, phosphorylated peptide, phosphorylated protein, phosphorylated sugar, or phospholipid is retained in a titanium dioxide column as a pretreatment column under an acidic condition, the phosphorylated compound being retained in the titanium dioxide column as the pretreatment column that is treated with an acidic solution at a pH of 0.5 to 7.0 to detect a non-phosphorylated compound; and then eluting the phosphorylated compound with a solution containing 10 mM to 2, 000 mM phosphate, 0.06% or more phosphoric acid, or alkali at a pH of 7.0 or higher. Therefore, this method makes it possible to selectively extract/separate the phosphorylated amino acid, phosphorylated peptide, phosphorylated protein, phosphorylated sugar, or phospholipid by using a simple operation and an inexpensive reagent.

The method of claim 5 of the present invention comprises the step of detecting a phosphorylated compound in which a sample containing the phosphorylated amino acid, phosphorylated peptide, phosphorylated protein, phosphorylated sugar, or phospholipid is retained in a titanium dioxide column as a pretreatment column under an acidic condition or the like, using a reversed phase solid phase for separation and/or purification of the phosphorylated compound, and using an MS having an ionization part of a MALDI plate as a desired detector. Therefore, this method can bring the condition of the phosphorylated amino acid, phosphorylated peptide, phosphorylated protein, phosphorylated sugar, or phospholipid, which is difficult to be ionized when a contaminating matrix or salt is mixed, into a condition to be easily ionized and measured in MS.

The method of claim 6 of the present invention comprises the steps of: retaining a phosphorylated compound by retaining a sample containing a phosphorylated sugar in a titanium dioxide column as a pretreatment column under an acidic condition in which the sample is retained by an acid solution at a pH of about 0.8; detecting a non-phosphorylated compound after separation and purification thereof by use of an anion exchange column; eluting the phosphorylated compound with a solution containing 50 mM or more phosphate, 1% or more phosphoric acid, or alkali at a pH of 11.25 or higher; and detecting the phosphorylated compound after separation and purification thereof by use of the anion exchange column. Therefore, this method makes it possible to subject the phosphorylated sugar that is extracted/separated by using a simple operation and an inexpensive reagent to analysis using a column with a nature different from that of the titanium dioxide column for identification, further purification, qualitative analysis, or quantitative determination.

The method of claim 7 of the present invention comprises the steps of: retaining a phosphorylated compound by retaining a sample containing the phospholipid in a titanium dioxide column as a pretreatment column under an acidic condition in which the sample is retained by a solution containing an acid solution at a pH of about 2.0; detecting a non-phosphorylated compound after separation and purification thereof by use of a reversed phase column; eluting the phosphorylated compound by a solution containing 0.6% or more phosphoric acid; and detecting the phosphorylated compound after separation and purification thereof by use of a reversed phase column. Therefore, this method makes it possible to subject the phospholipid that is extracted/separated by using a simple operation and an inexpensive reagent to analysis using a column with a nature different from that of the titanium dioxide column for identification, further purification, qualitative analysis, or quantitative determination.

The method of claim 8 of the present invention comprises the steps of: retaining a phosphorylated compound by retaining a phosphorylated amino acid, phosphorylated peptide, phosphorylated protein, or sample containing them in a titanium dioxide column as a pretreatment column under an acidic condition in which the sample is retained by a solution containing an acid solution at a pH of about 2.0; detecting a non-phosphorylated compound after separation and purification thereof by use of a reversed phase column; eluting the phosphorylated compound with a solution containing 50 mM or more phosphate, 0.5% or more phosphoric acid, or a solution containing alkali having a pH of 11.25 or higher; and detecting the phosphorylated compound after separation and purification thereof by use of a reversed phase column. Therefore, this method makes it possible to subject the phosphorylated amino acid, phosphorylated peptide, or phosphorylated protein that is extracted/separated by using a simple operation and an inexpensive reagent to analysis using a column with a nature different from that of the titanium dioxide column for identification, further purification, qualitative analysis, or quantitative determination.

In claim 9 of the present invention, a desired number of solvents can be selectably delivered into a pump, and a switching valve is connected to the pump via an injector, while a titanium dioxide column and a reversed phase HPLC column are connected to the switching valve so that the titanium dioxide column can be arbitrarily set as a pretreatment column, and a detector is connected to the reversed phase HPLC column. This apparatus passes a sample ingredient through the titanium dioxide column to selectively retain a desired ingredient such as a phosphorylated amino acid or phosphorylated peptide, then allows the reversed phase HPLC column to retain ingredients other than the desired ingredient, shifts to a detection operation, allows buffer to elute the desired ingredient that is to be retained by the reversed phase HPLC column followed by detection and identification operations. Therefore, this apparatus is important also for pursuing a protein phosphorylation degree change that responds to an intracellular condition change in proteomics such as an existing amount change of the desired ingredient.

In claim 10 of the present invention, a desired number of solvents can be selectably delivered into a pump, and a switching valve is connected to the pump via an injector, while a titanium dioxide column and a solid phase extraction column are connected to the switching valve so that the titanium dioxide column can be arbitrarily set as a pretreatment column, and a detector is connected to the solid phase extraction column. Consequently, this apparatus passes a sample ingredient through the titanium dioxide column to selectively retain a desired ingredient such as a phosphorylated amino acid or phosphorylated peptide, then allows the solid phase column to retain ingredients other than the desired ingredient, shifts to a detection operation, allows buffer to elute the desired ingredient that is to be retained by the solid phase column followed by detection and identification operations. Therefore, this apparatus is important also for pursuing a protein phosphorylation degree change that responds to an intracellular condition change in proteomics such as an existing amount change of the desired ingredient.

**Claims**

1. A method for analyzing an amino acid, peptide, protein, sugar, or lipid, comprising the steps of:

    retaining a phosphorylated compound in which a sample containing a phosphorylated amino acid, phosphorylated peptide, phosphorylated protein, phosphorylated sugar, or phosphorylated lipid is retained in a titanium dioxide column as a pretreatment column under an acidic condition;
    detecting a non-phosphorylated compound in which an amino acid, peptide, protein, sugar, or lipid that has not been retained is separated and/or purified through a desired column and then detected with a desired detector, or the compound is detected with the desired detector without purification, or the compound is not either separated and purified or detected;
    eluting the phosphorylated compound in which the phosphorylated amino acid, phosphorylated peptide, phosphorylated protein, phosphorylated sugar, or phosphorylated lipid retained in the pretreatment column is eluted by a solution containing phosphate, phosphoric acid, or alkali; and
    detecting the phosphorylated compound in which the compound is separated and purified through the desired column and then detected with the desired detector, or the compound is detected with the desired detector without purification.

2. Amethod for analyzing an amino acid, peptide, or protein comprising extraction/separation of a phosphorylated amino acid, phosphorylated peptide, or phosphorylated protein, comprising the steps of:

    supplying a phosphorylated amino acid, phosphorylated peptide, phosphorylated protein, or sample containing them in an acidic solution at a pH of about 2 to a reversed phase HPLC column with a titanium dioxide column as a pretreatment column, selectively retaining the phosphorylated amino acid, phosphorylated peptide, or phosphorylated protein from the sample matrix, purifying an amino acid, peptide, or protein that has not been retained in the pretreatment column by the reversed phase HPLC column followed by detection thereof by a desired detector;
    eluting the phosphorylated amino acid, phosphorylated peptide, or phosphorylated protein retained in the pretreatment column with 50 mM or higher phosphate buffer followed by separation thereof through the reversed phase HPLC column and detection thereof with the desired detector.

3. A method for extracting, separating, and purifying a phosphorylated amino acid, phosphorylated peptide, phosphorylated protein, phosphorylated sugar, or phosphorylated lipid,
    wherein a sample containing the phosphorylated amino acid, phosphorylated peptide, phosphorylated protein, phosphorylated sugar, or phosphorylated lipid is retained in a titanium dioxide column as a pretreatment column under an acidic condition and then fractionated directly for use without transferring the phosphorylated

compound eluted in an elution step to a detection step.

**4.** A method for analyzing an amino acid, peptide, protein, sugar, or lipid comprising extraction/separation of a phosphorylated amino acid, phosphorylated peptide, phosphorylated protein, phosphorylated sugar, or phosphorylated lipid, comprising the steps of:

retaining a phosphorylated compound in which a sample containing the phosphorylated amino acid, phosphorylated peptide, phosphorylated protein, phosphorylated sugar, or phosphorylated lipid is retained in a titanium dioxide column as a pretreatment column under an acidic condition, the phosphorylated compound being retained in the titanium dioxide column as the pretreatment column that is treated with an acidic solution at a pH of 0.5 to 7.0 to detect a non-phosphorylated compound; and then
eluting the phosphorylated compound with a solution containing 10 mM to 2,000 mM phosphate, 0.06% or more phosphoric acid, or alkali at a pH of 7.0 or higher.

**5.** A method for analyzing an amino acid, peptide, protein, sugar, or lipid comprising extraction/detection of a phosphorylated amino acid, phosphorylated peptide, phosphorylated protein, phosphorylated sugar, or phosphorylated lipid comprising the step of:

detecting a phosphorylated compound in which a sample containing the phosphorylated amino acid, phosphorylated peptide, phosphorylated protein, phosphorylated sugar, or phosphorylated lipid is retained in a titanium dioxide column as a pretreatment column under an acidic condition or the like, using a reversed phase solid phase for separation and/or purification of the phosphorylated compound, and using an MS having an ionization part of a MALDI plate as a desired detector.

**6.** A method for analyzing a sugar including extraction/separation of a phosphorylated sugar, comprising the steps of:

retaining a phosphorylated compound by retaining a sample containing a phosphorylated sugar in a titanium dioxide column as a pretreatment column in an acidic condition in which the sample is retained by an acid solution at a pH of about 0.8;
detecting a non-phosphorylated compound after separation and purification thereof by use of an anion exchange column;
eluting the phosphorylated compound with a solution containing 50 mM or more phosphate, 1% or more phosphoric acid, or alkali at a pH of 11.25 or higher; and
detecting the phosphorylated compound after separation and purification thereof by use of the anion exchange column.

**7.** A method for analyzing a lipid including extraction/separation of a phosphorylated lipid, comprising the steps of:

retaining a phosphorylated compound by retaining a sample containing the phosphorylated lipid in a titanium dioxide column as a pretreatment column in an acidic condition in which the sample is retained by a solution containing an acid solution at a pH of about 2.0;
detecting a non-phosphorylated compound after separation and purification thereof by use of a reversed phase column;
eluting the phosphorylated compound by a solution containing 0.6% or more phosphoric acid; and
detecting the phosphorylated compound after separation and purification thereof by use of a reversed phase column.

**8.** A method for analyzing an amino acid, peptide, or protein including extraction/separation of a phosphorylated amino acid, phosphorylated peptide, or phosphorylated protein, comprising the steps of:

retaining a phosphorylated compound by retaining a phosphorylated amino acid, phosphorylated peptide, phosphorylated protein, or sample containing them in a titanium dioxide column as a pretreatment column in an acidic condition in which the sample is retained by a solution containing an acid solution at a pH of about 2.0;
detecting a non-phosphorylated compound after separation and purification thereof by use of a reversed phase column;
eluting the phosphorylated compound with a solution containing 50 mM or more phosphate, 0.6% or more phosphoric acid, or a solution containing alkali having a pH of 11.25 or higher; and
detecting the phosphorylated compound after separation and purification thereof by use of a reversed phase

column.

**9.** An apparatus for selectively extracting and separating a phosphorylated amino acid and the like,
wherein a desired number of solvents can be selectably delivered into a pump, and a switching valve is connected to the pump via an injector, while a titanium dioxide column and a reversed phase HPLC column are connected to the switching valve so that the titanium dioxide column can be arbitrarily set as a pretreatment column, and a detector is connected to the reversed phase HPLC column.

**10.** An apparatus for selectively extracting and separating a phosphorylated amino acid and the like,
wherein a desired number of solvents can be selectably delivered into a pump, and a switching valve is connected to the pump via an injector, while a titanium dioxide column and a solid phase extraction column are connected to the switching valve so that the titanium dioxide column can be arbitrarily set as a pretreatment column, and a detector is connected to the solid phase extraction column.

Fig. 1

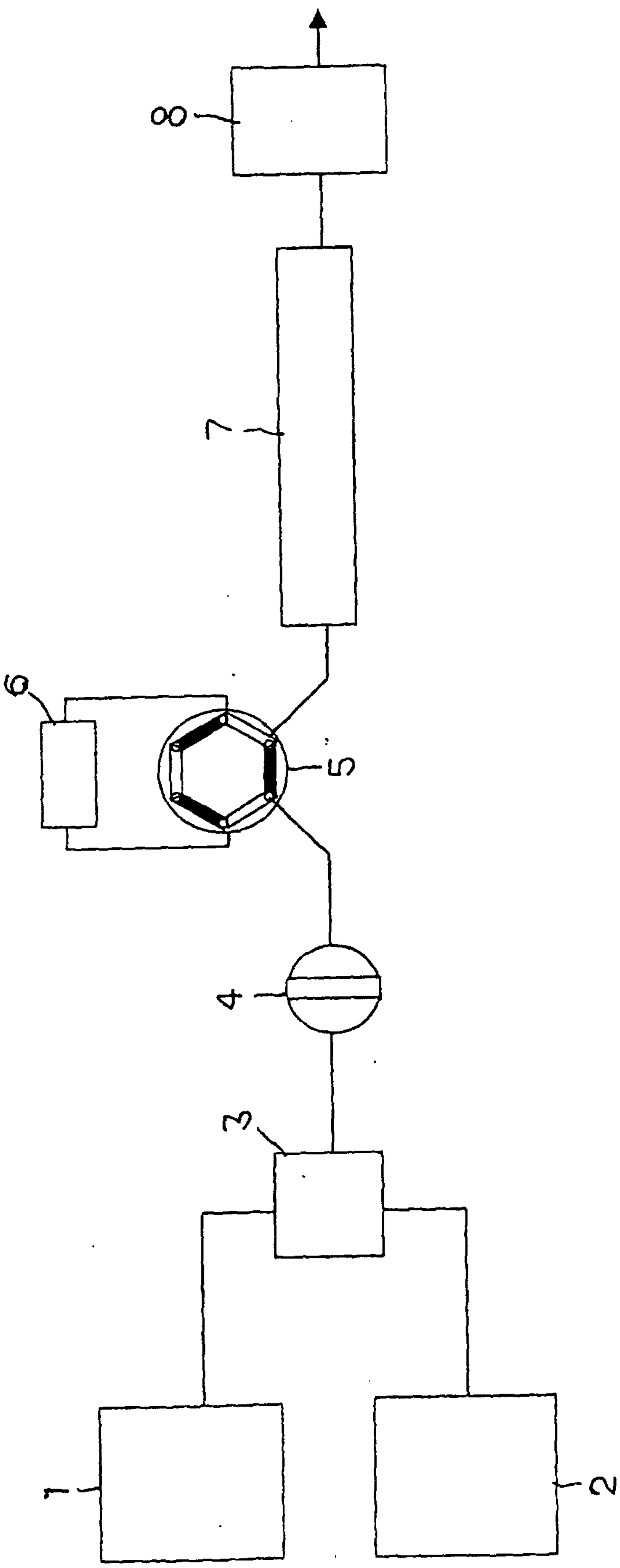

Fig. 2

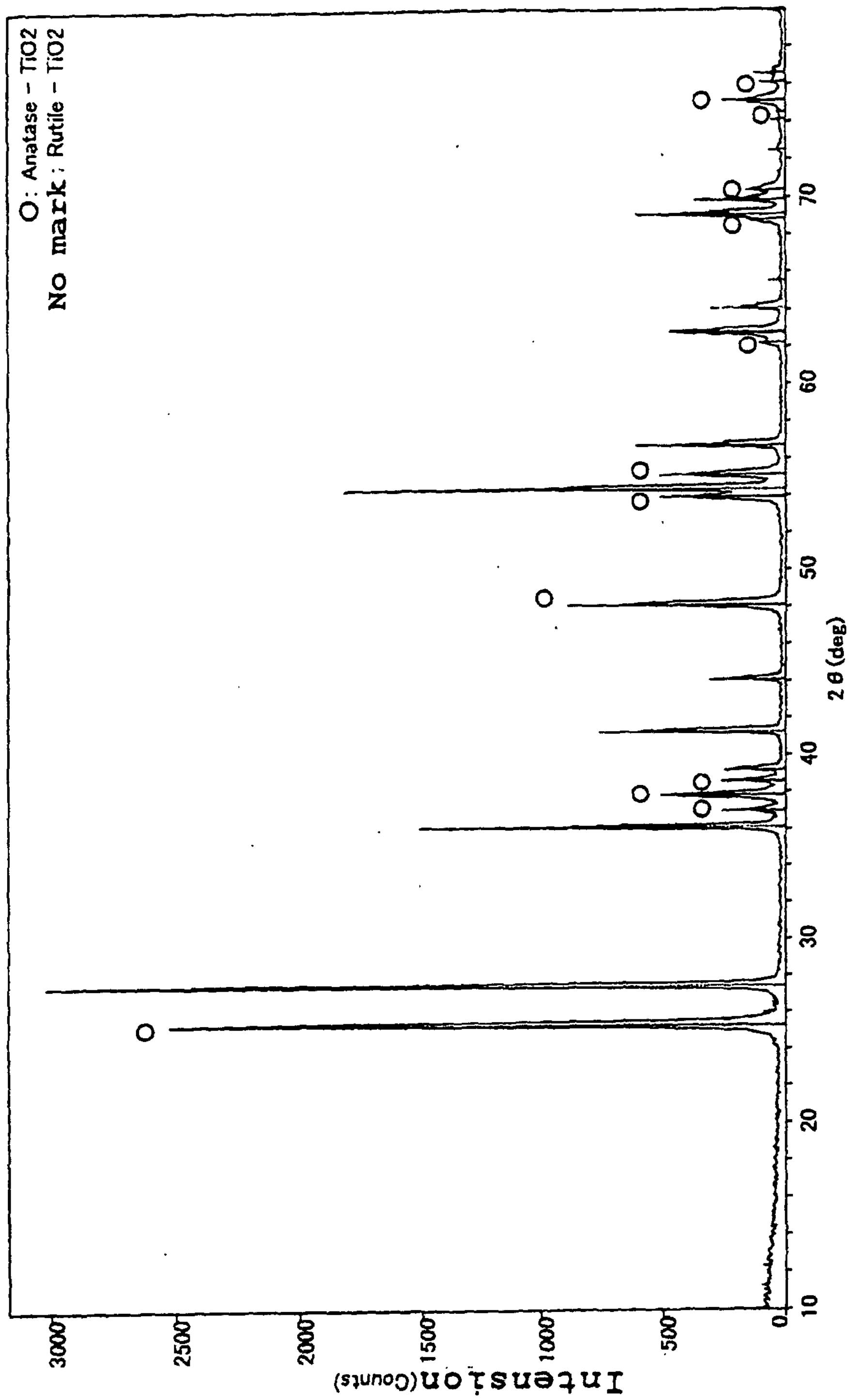
O: Anatase - TiO2
No mark: Rutile - TiO2
Intension(Counts)
2θ(deg)
0
500
1000
1500
2000
2500
3000
10
20
30
40
50
60
70

Fig. 3

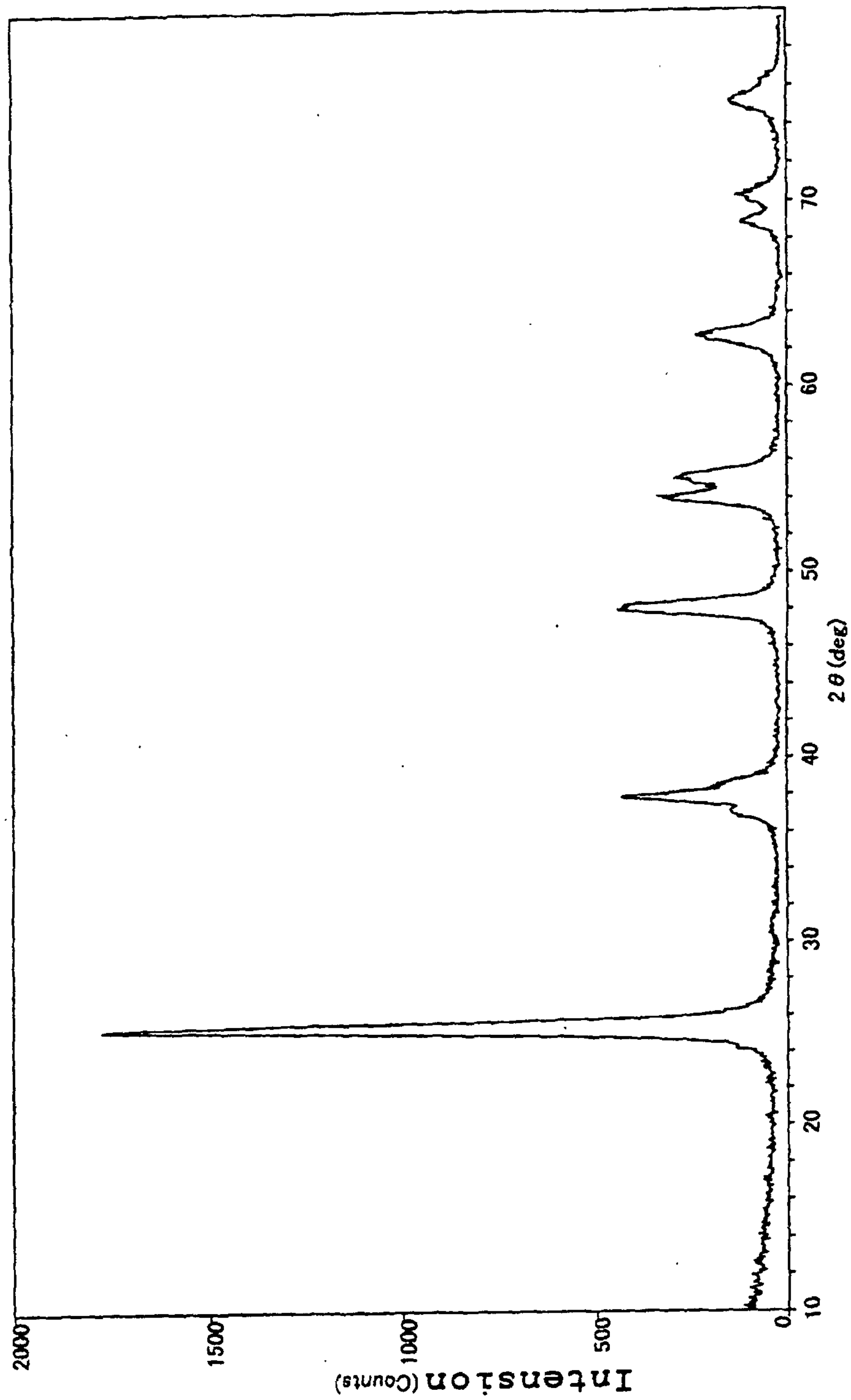
Intension (Counts)
2000
1500
1000
500
0
10
20
30
40
50
60
70
2θ (deg)

Fig. 4

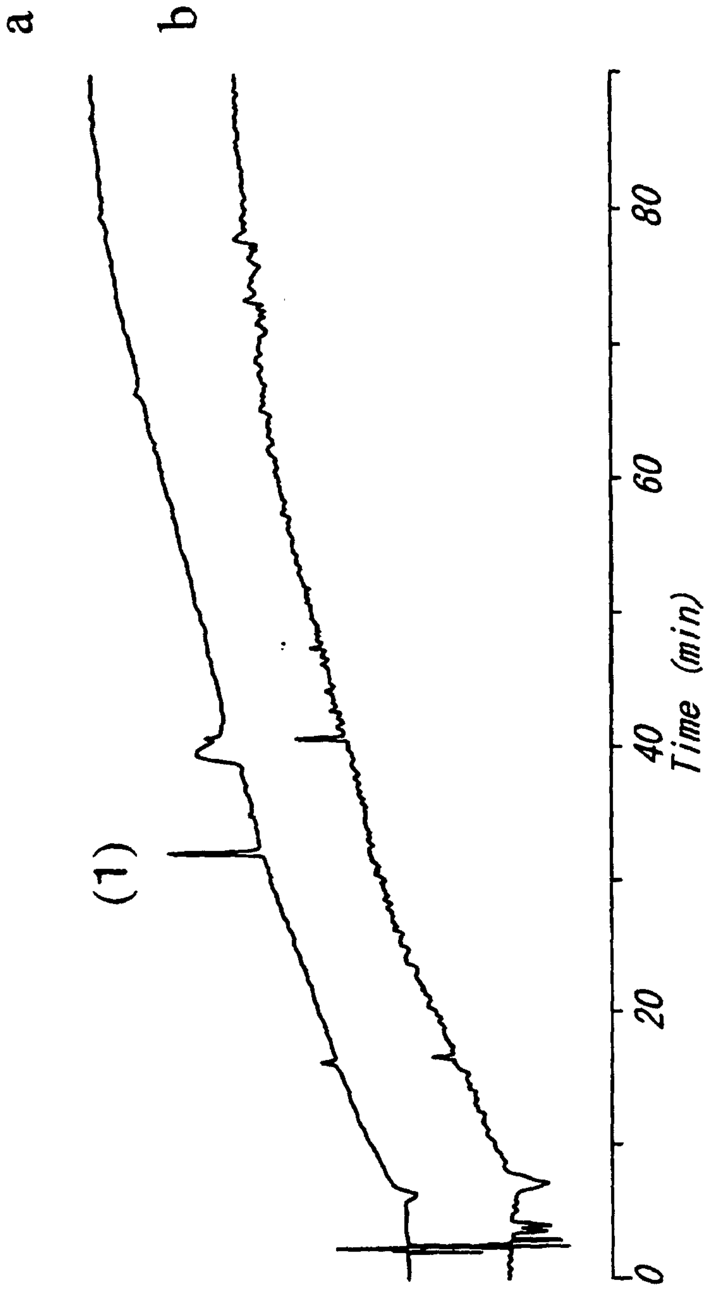

Fig. 5

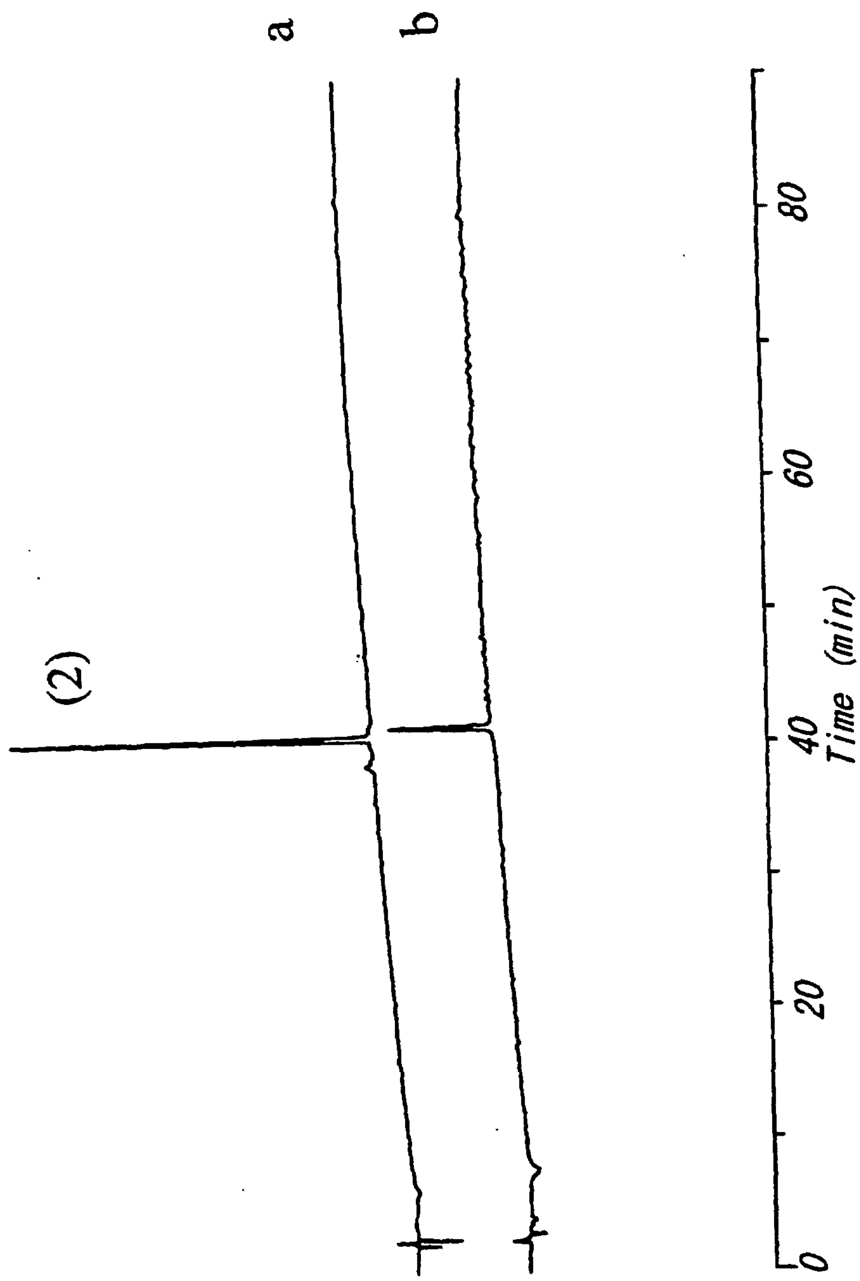

Fig. 6

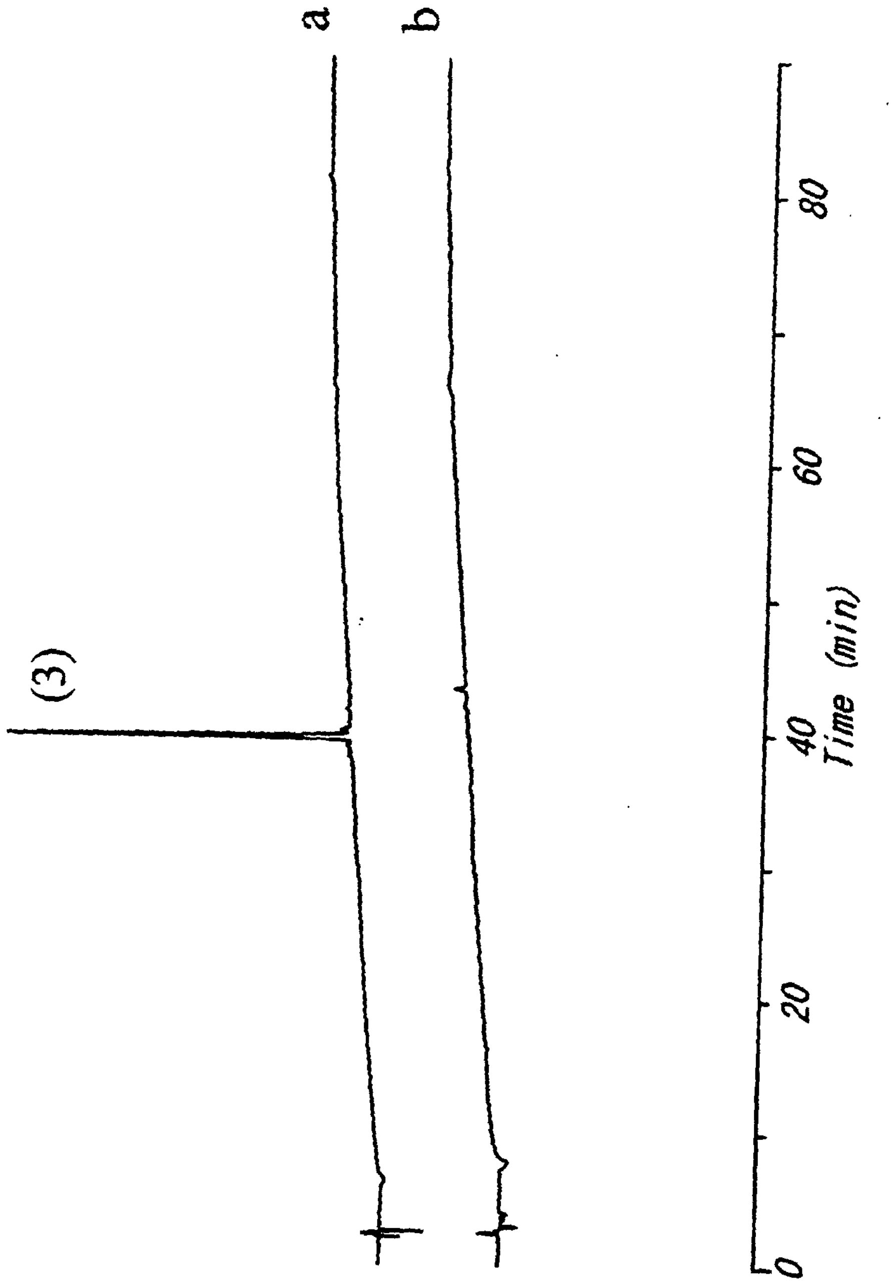

Fig. 7

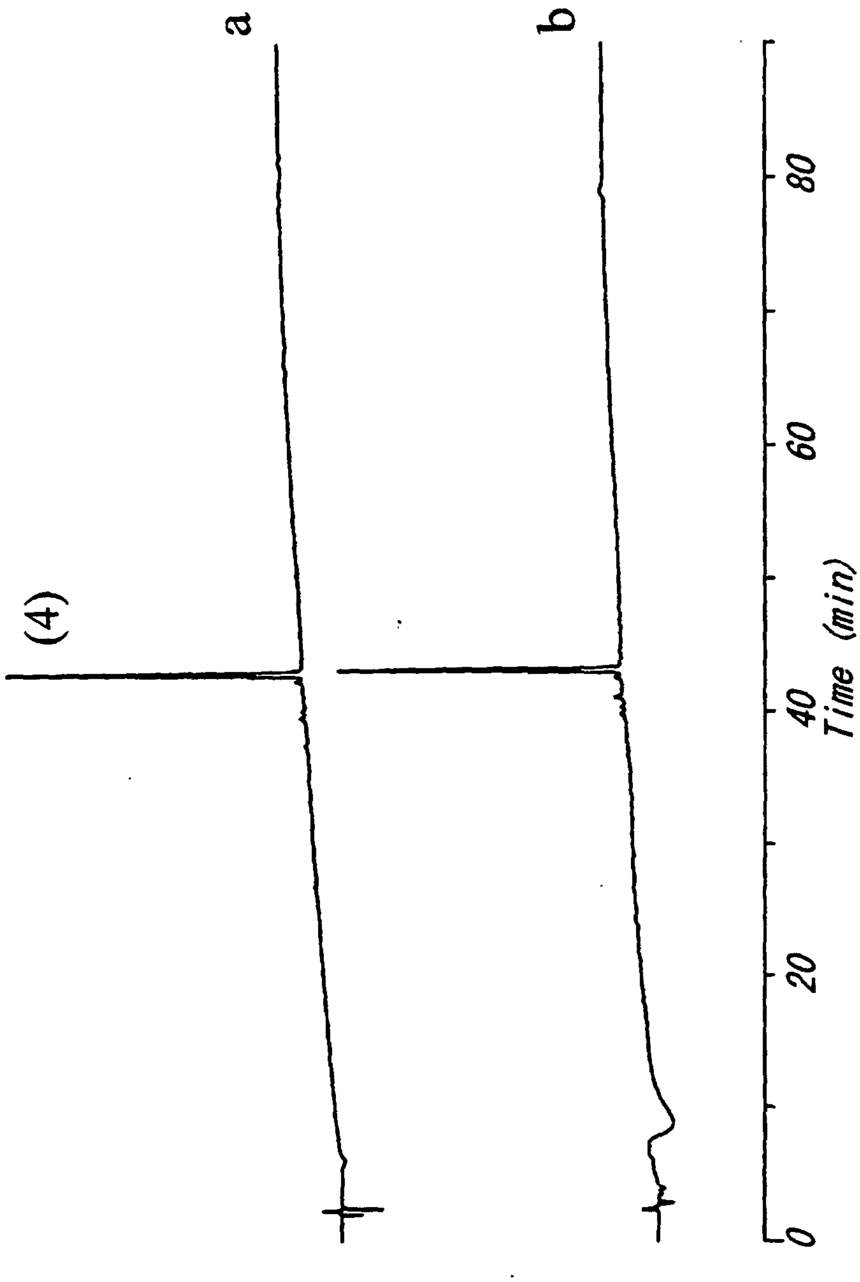

Fig. 8

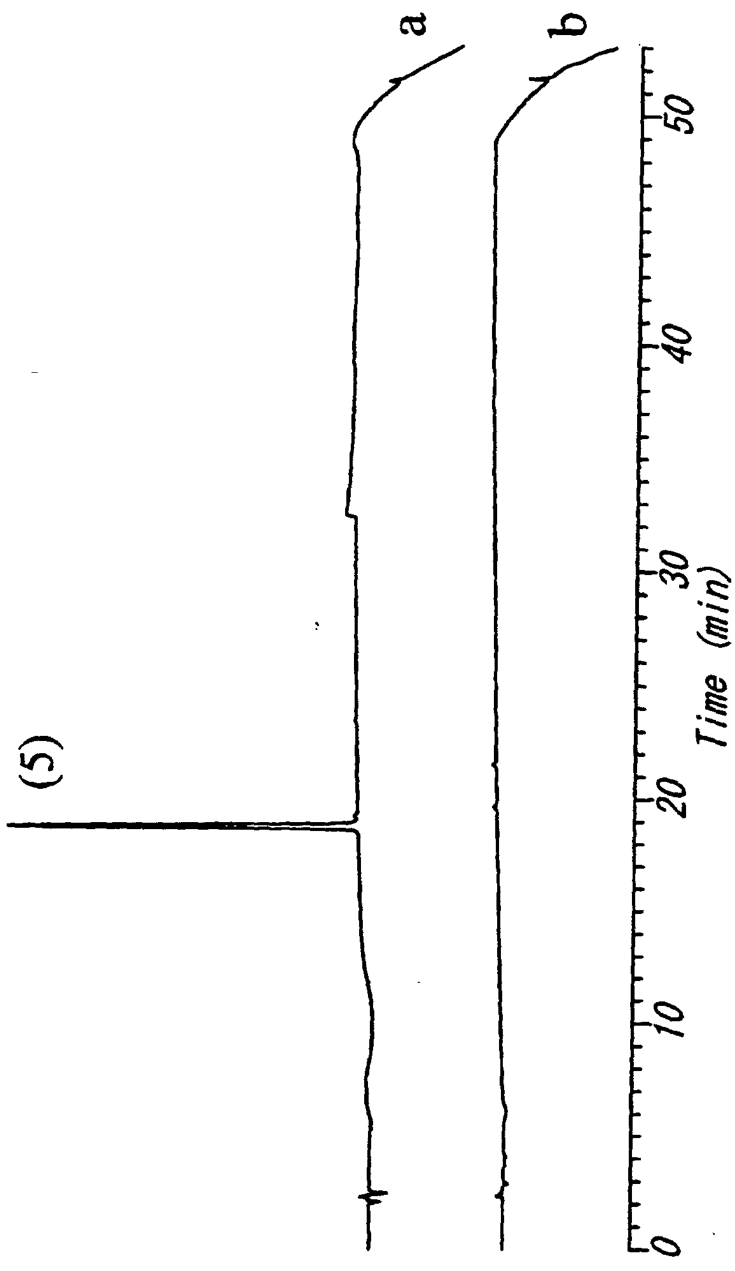

Fig. 9

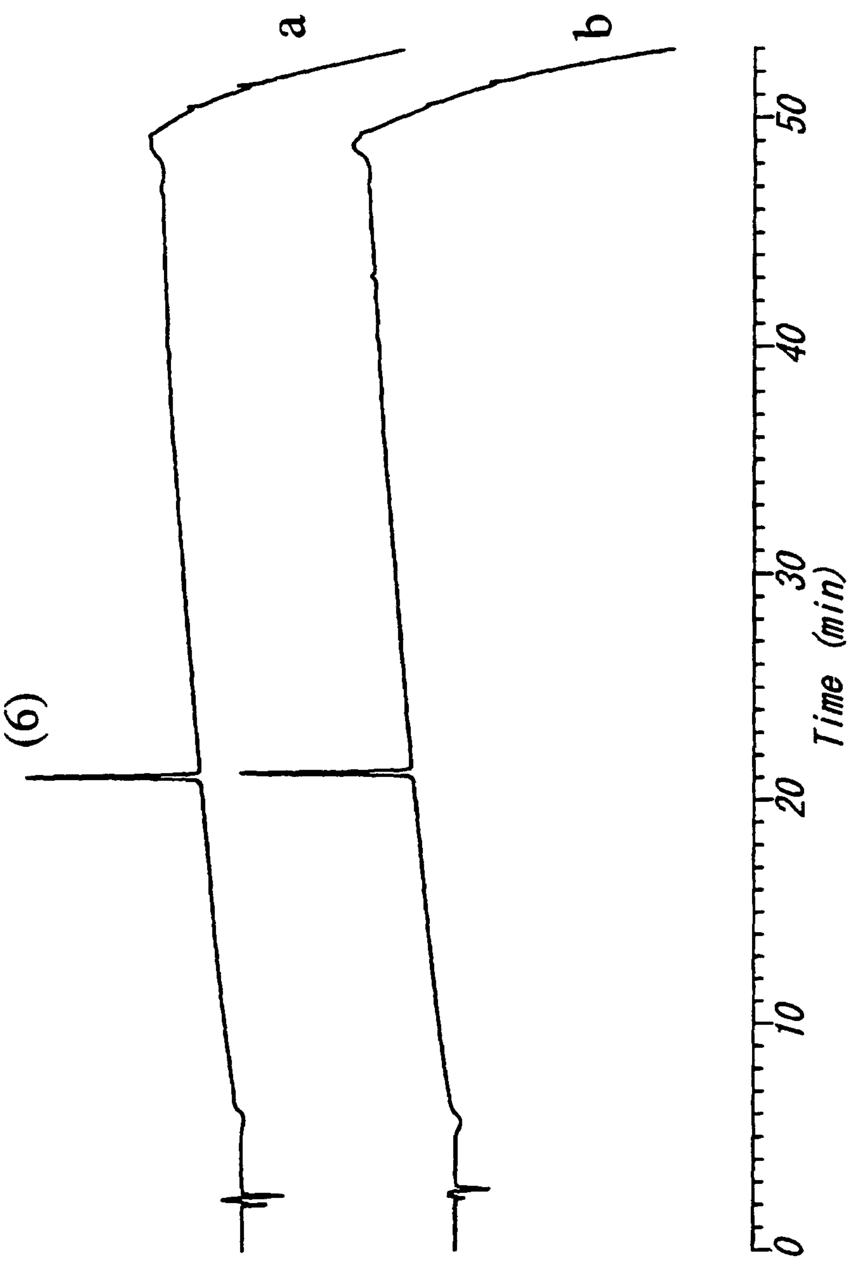

Fig. 10

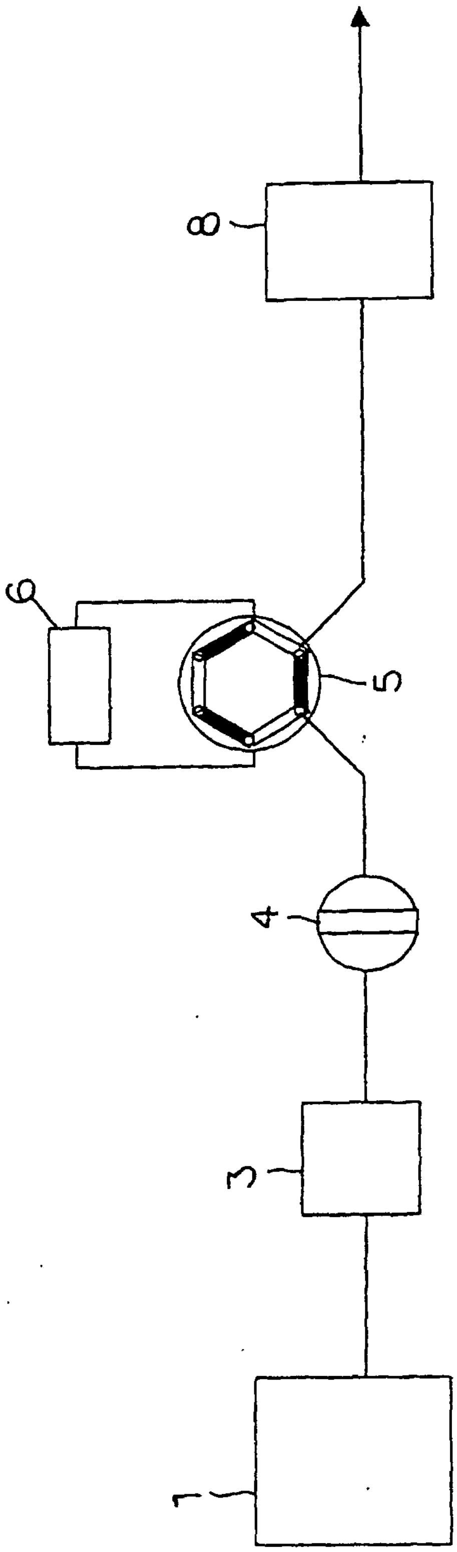

Fig. 11

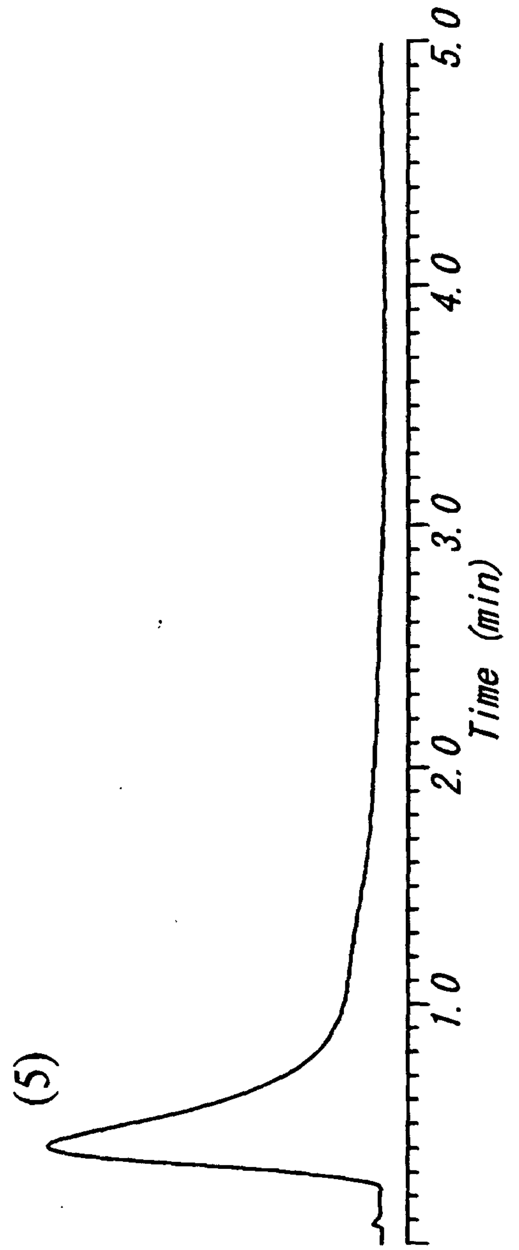

Fig. 12

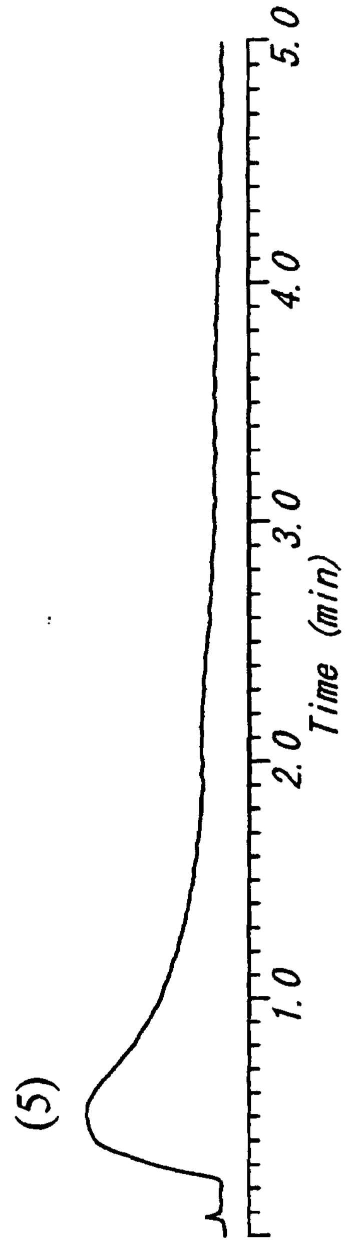

Fig. 13

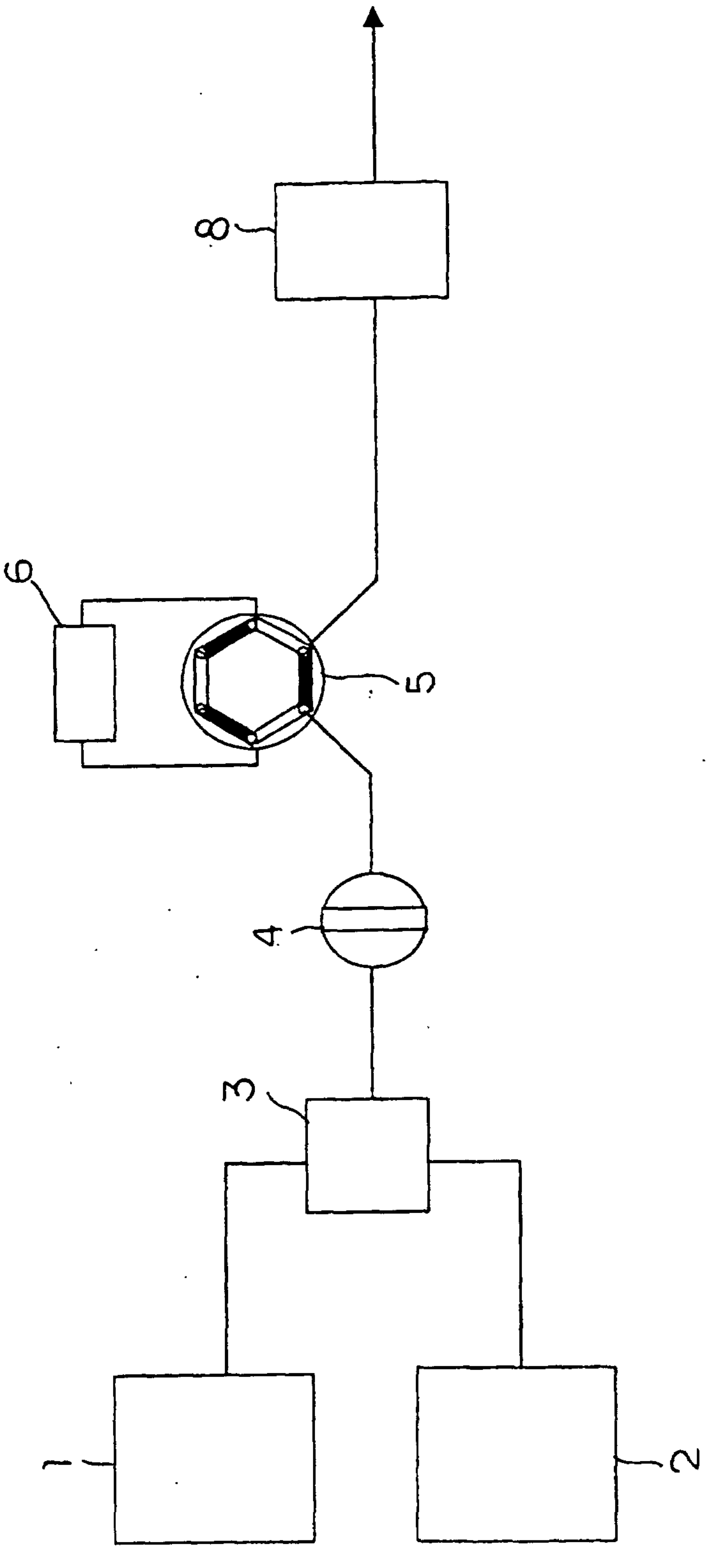

Fig. 14

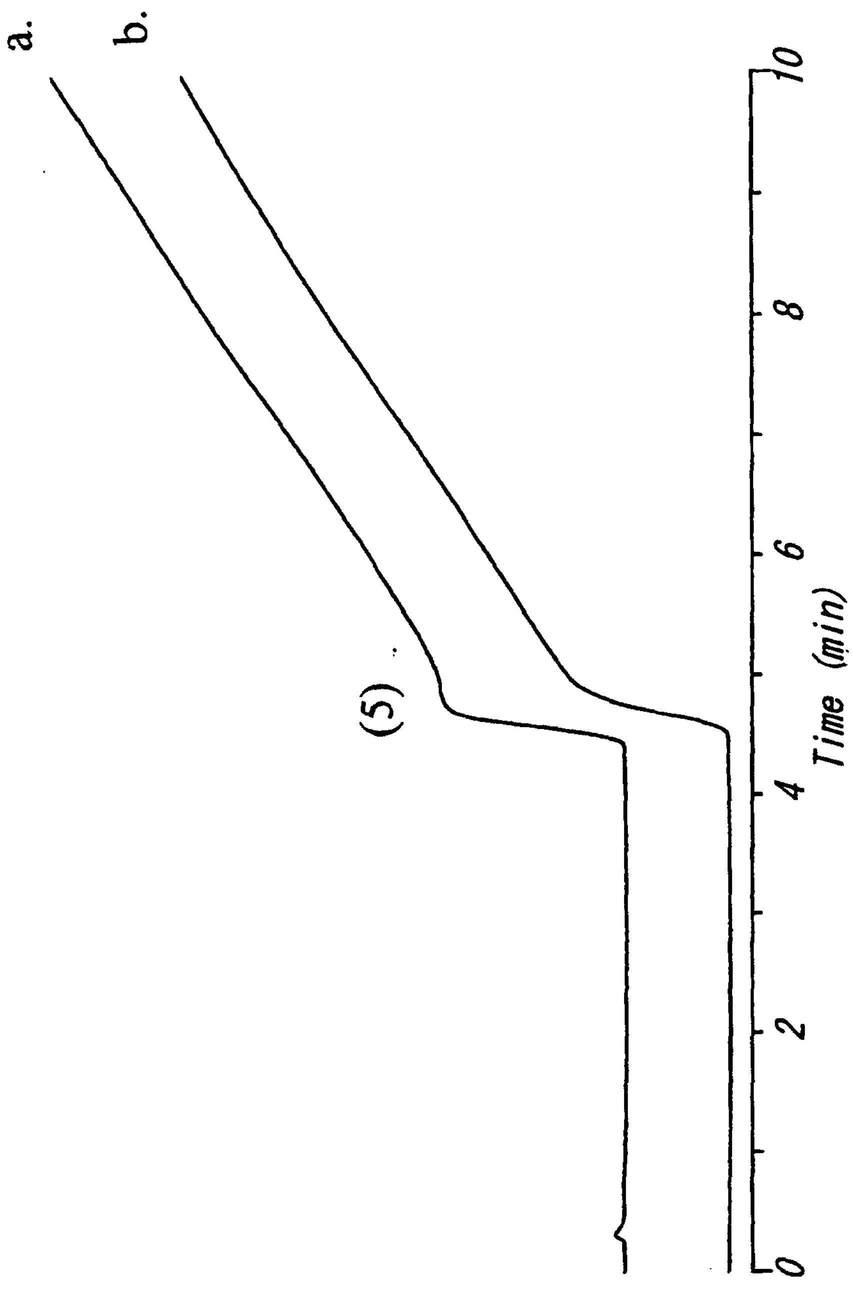

Fig. 15

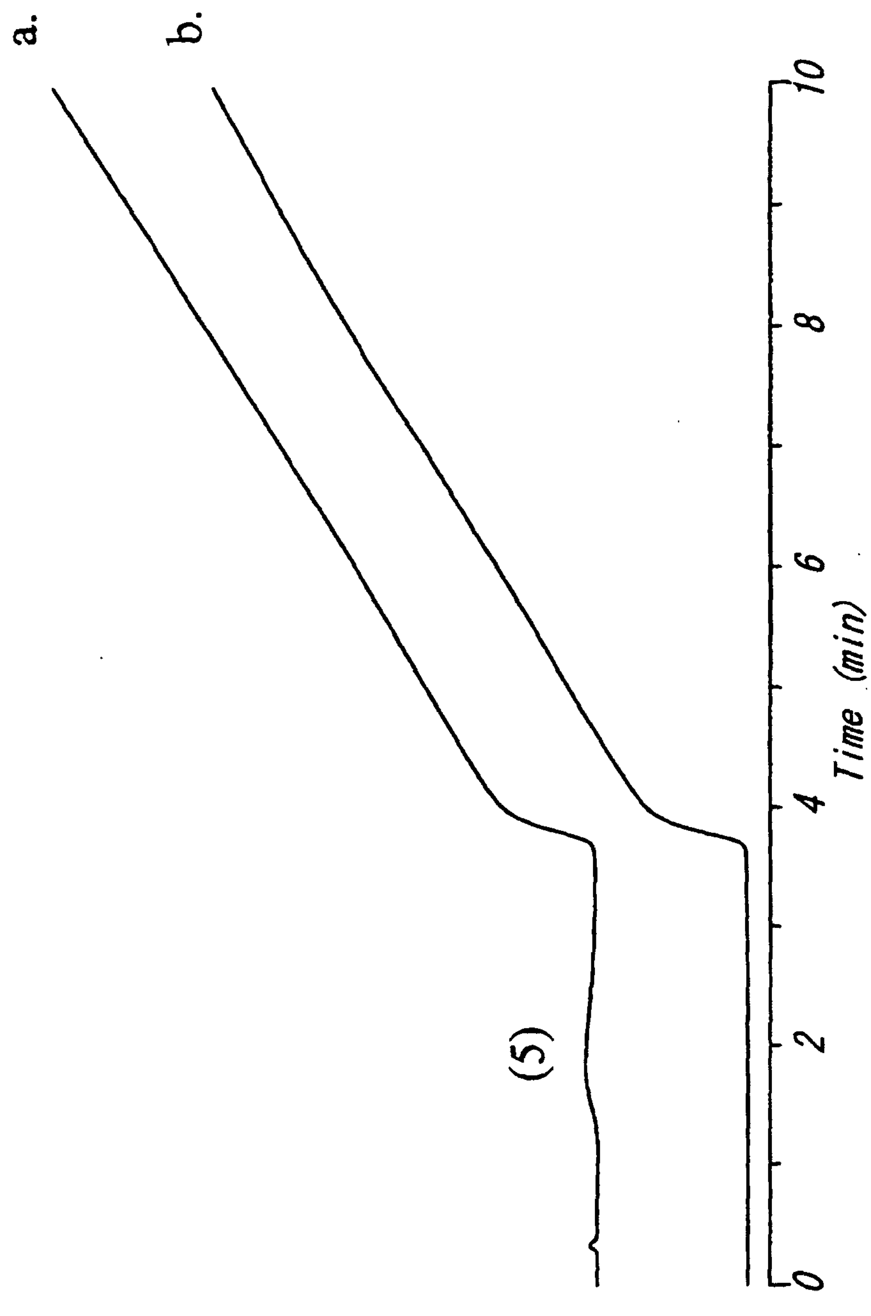

Fig. 16

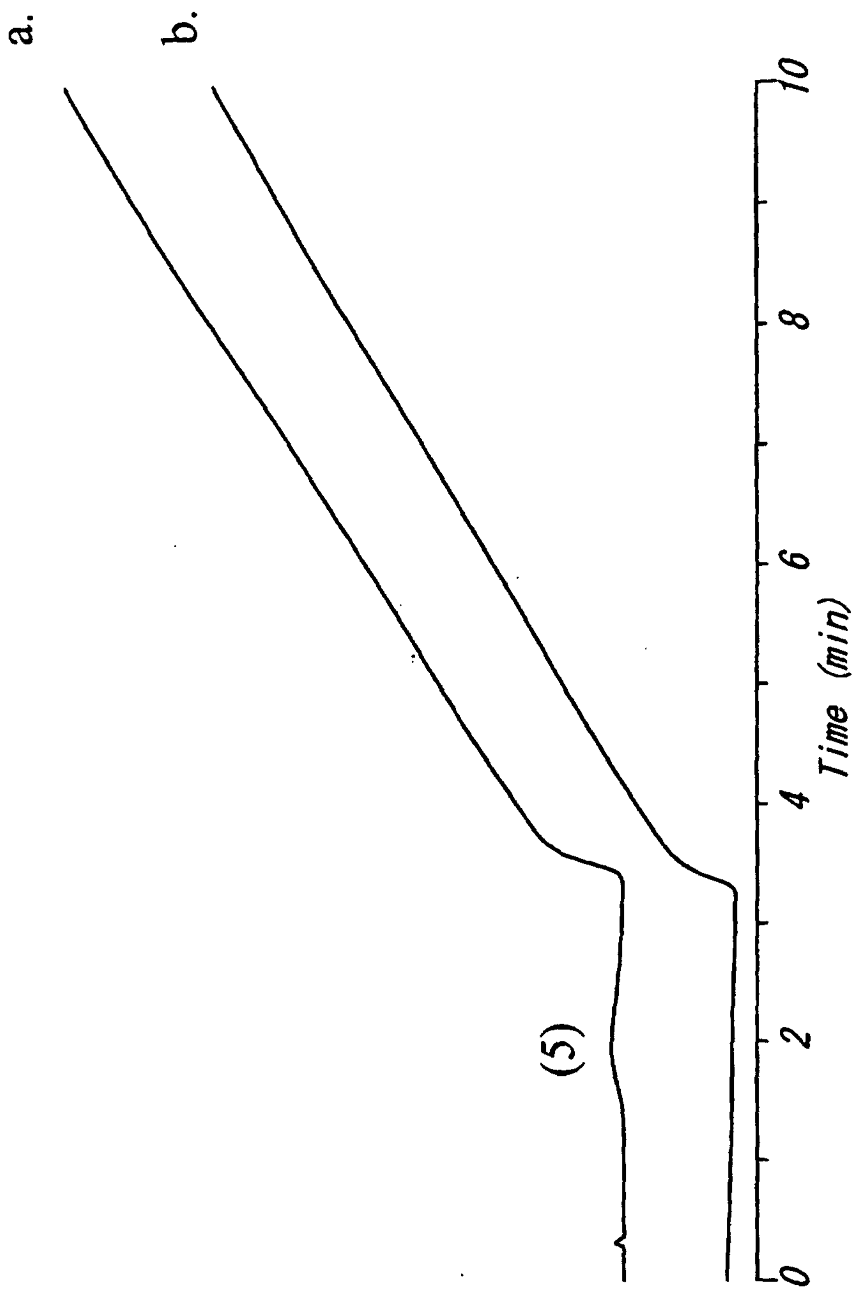

Fig. 17

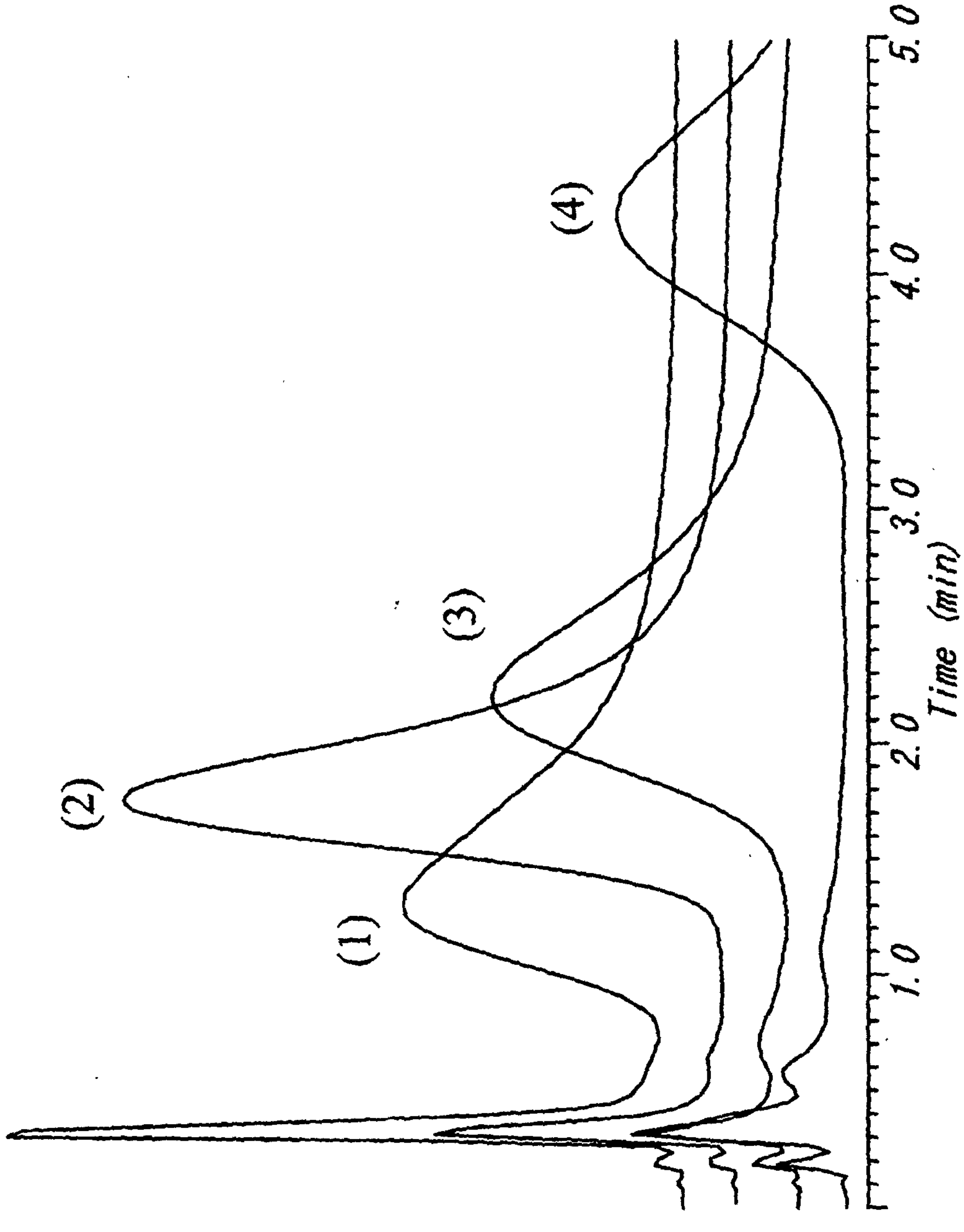

Fig. 18

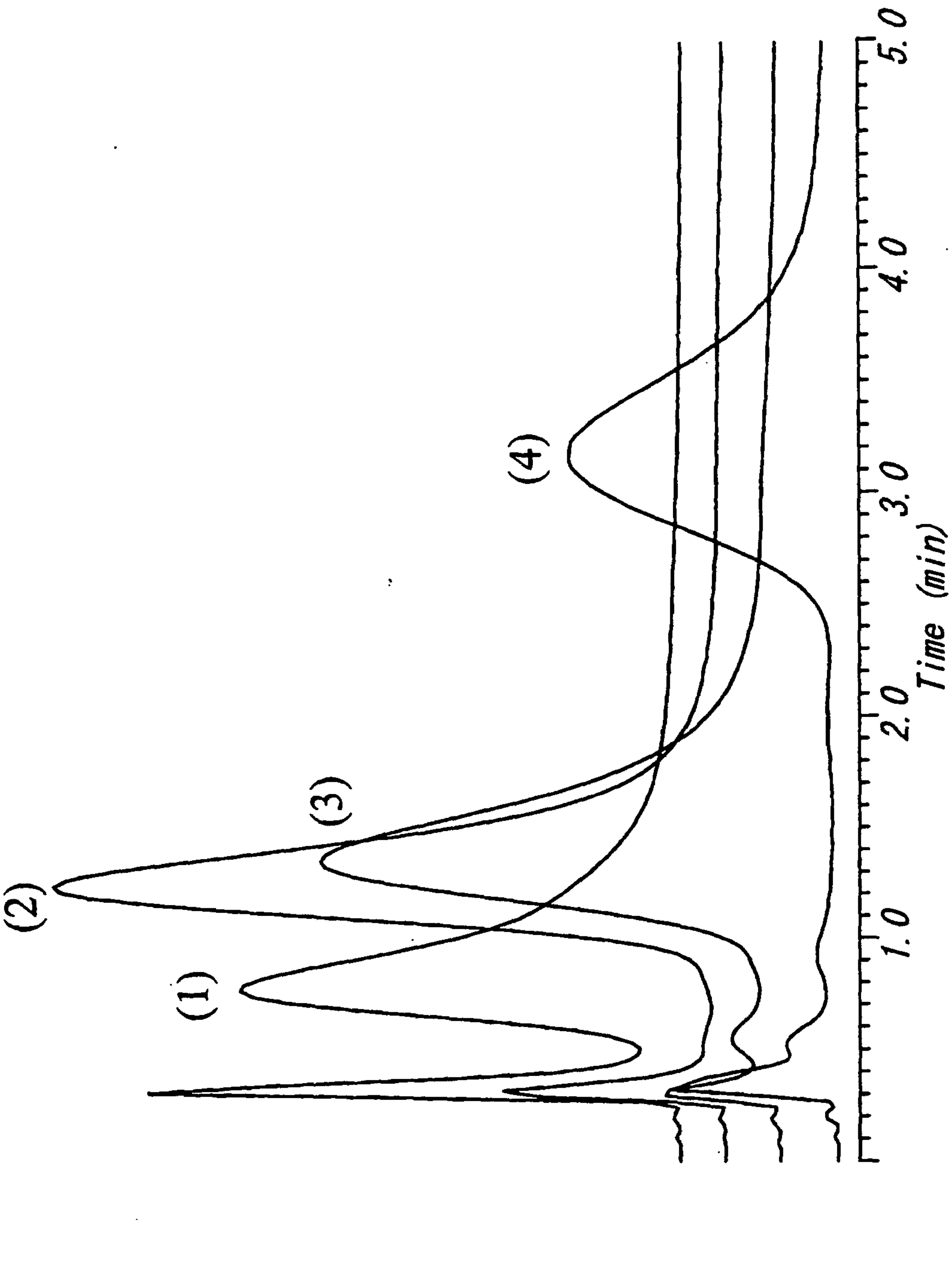

Fig. 19

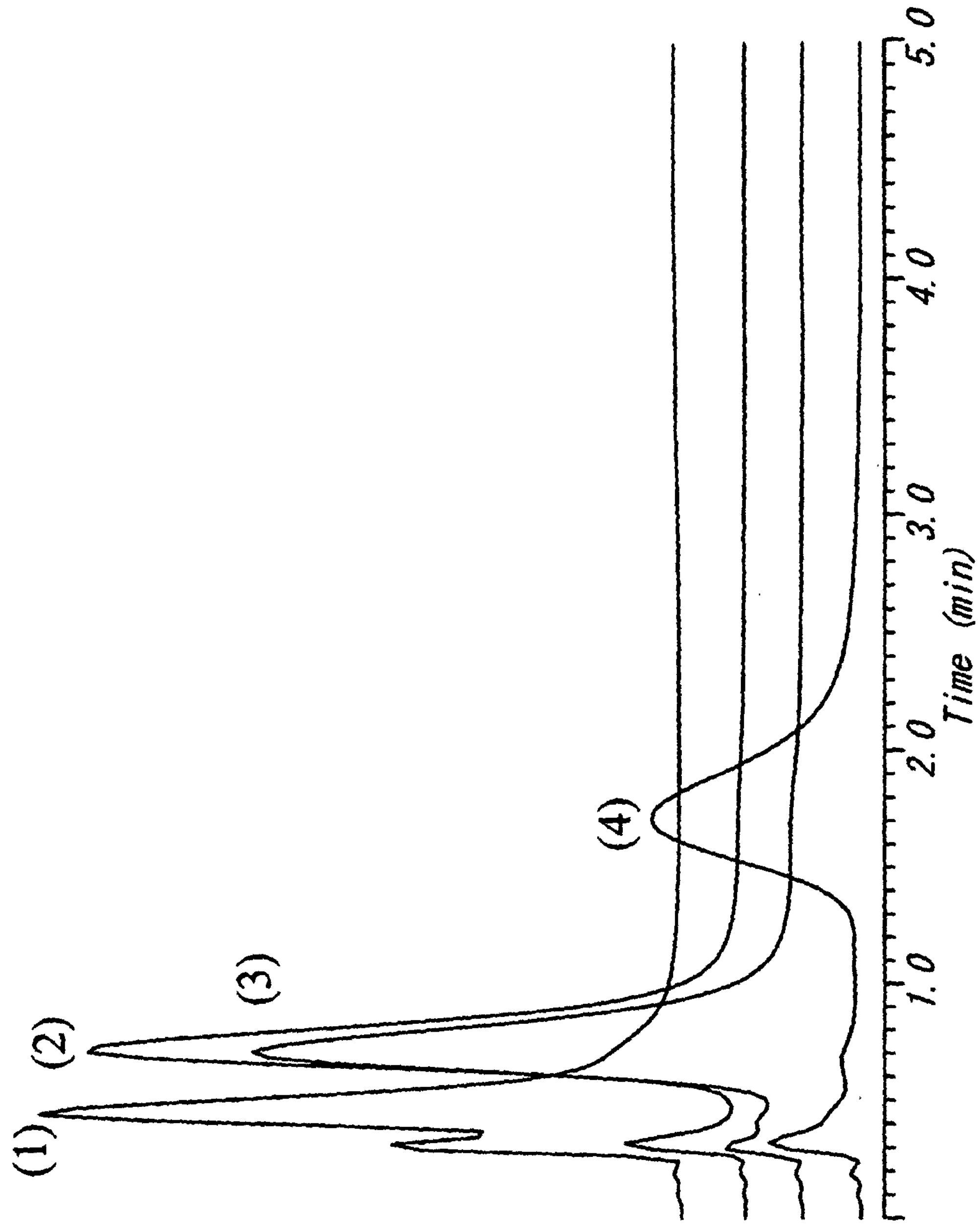

Fig. 20

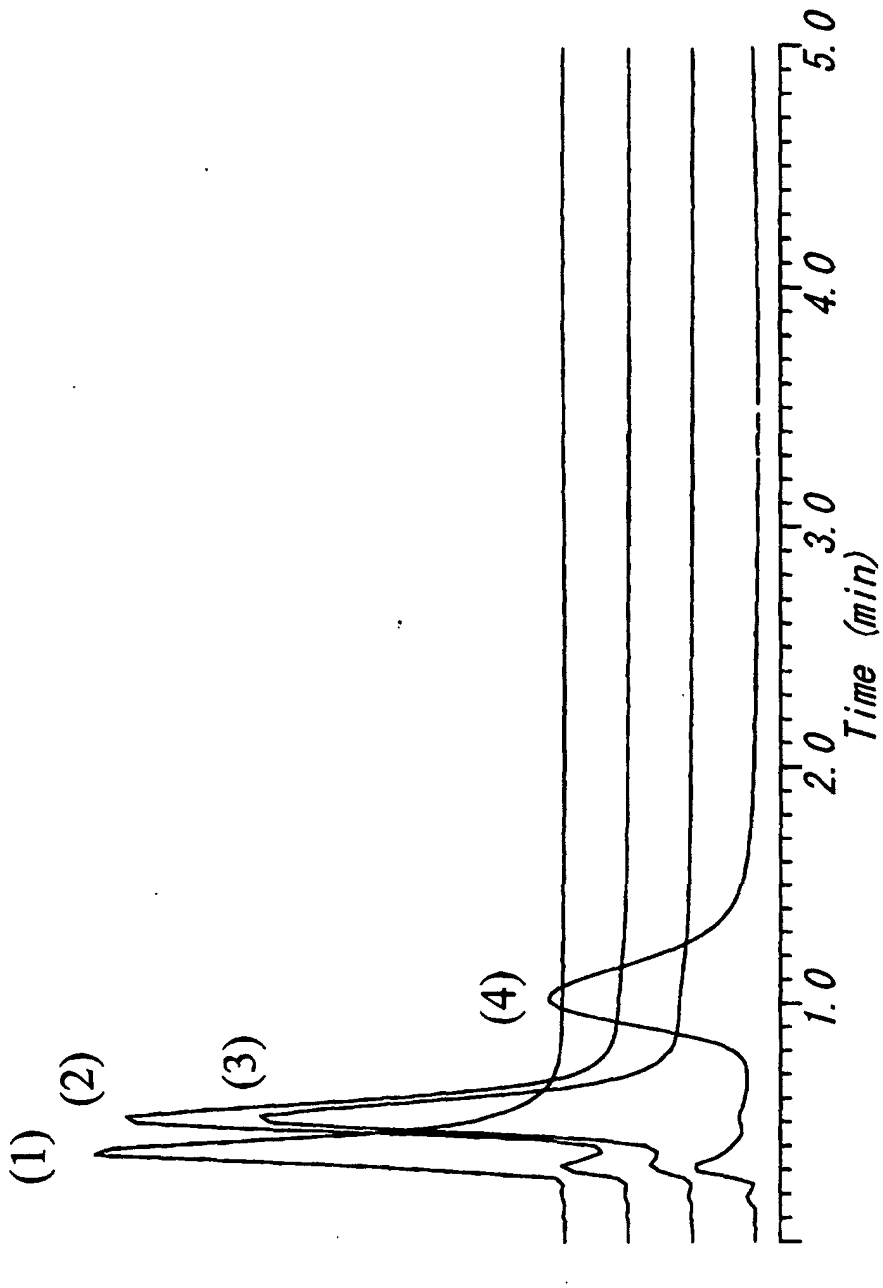

Fig. 21

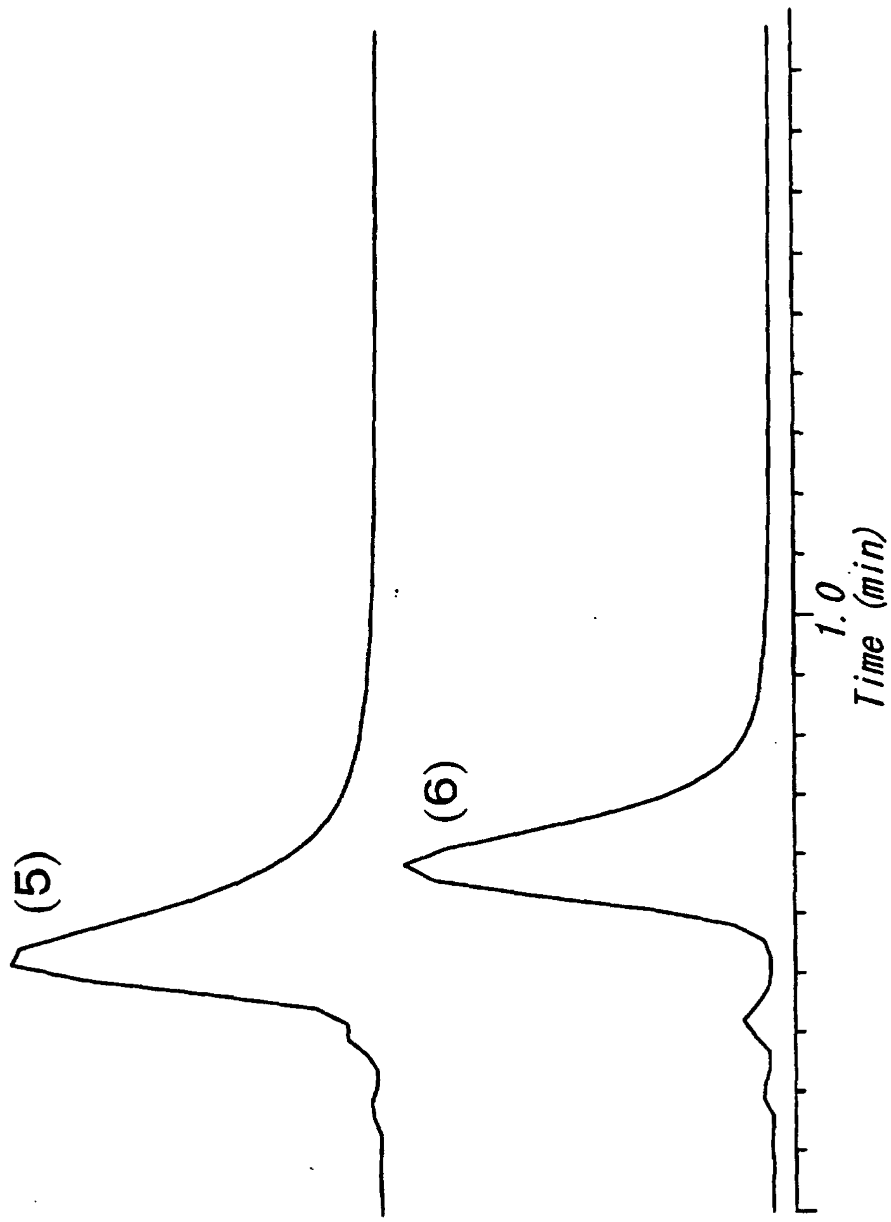

Fig. 22

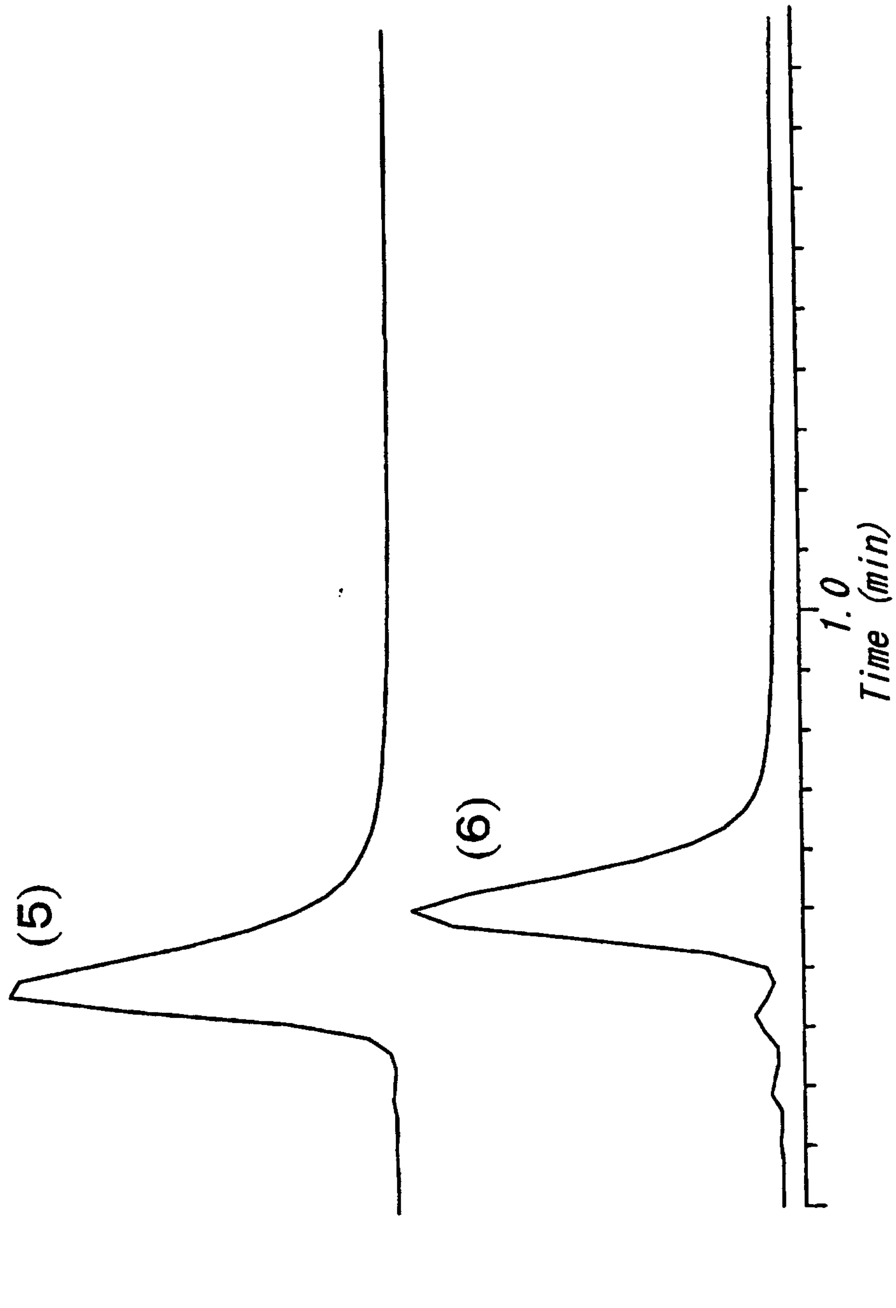

Fig. 23

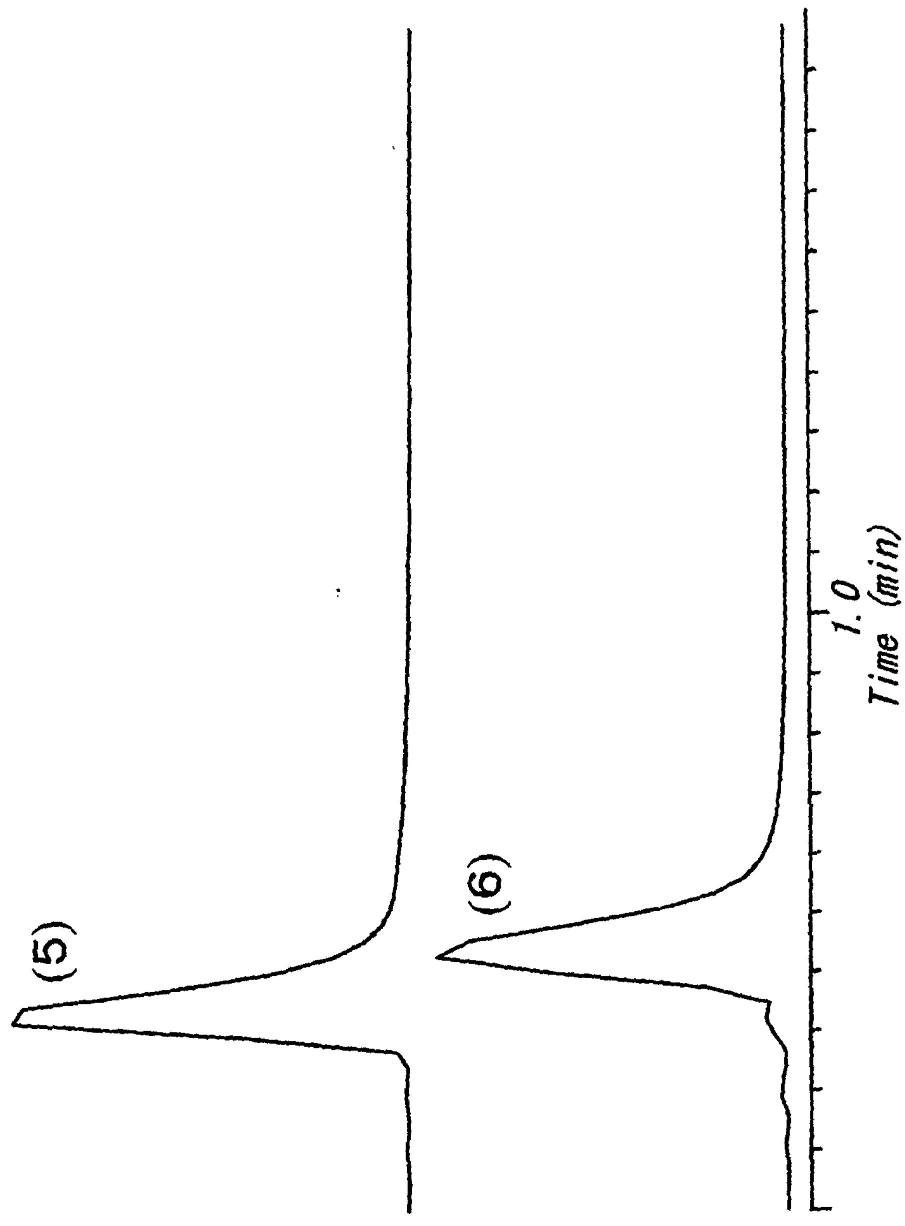

Fig. 24

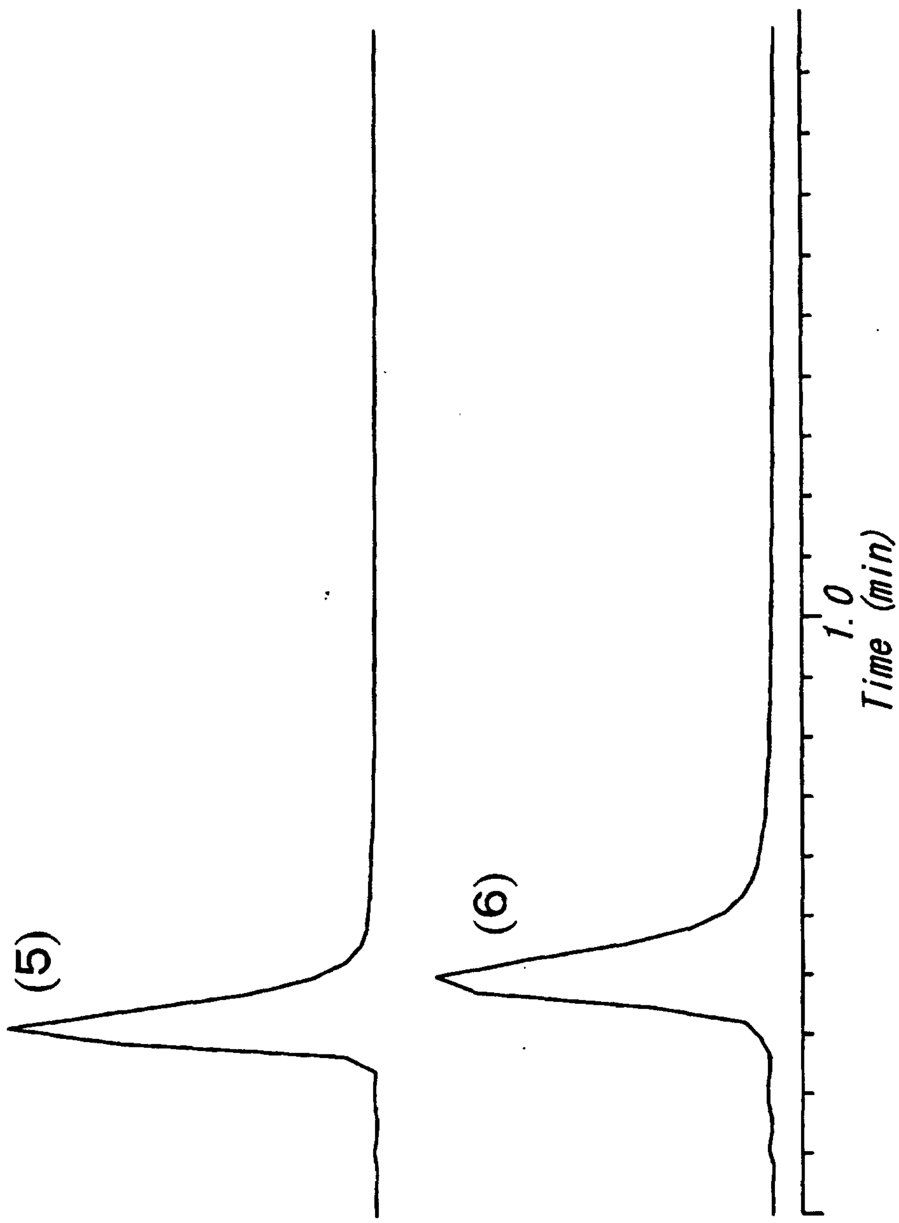

Fig. 25

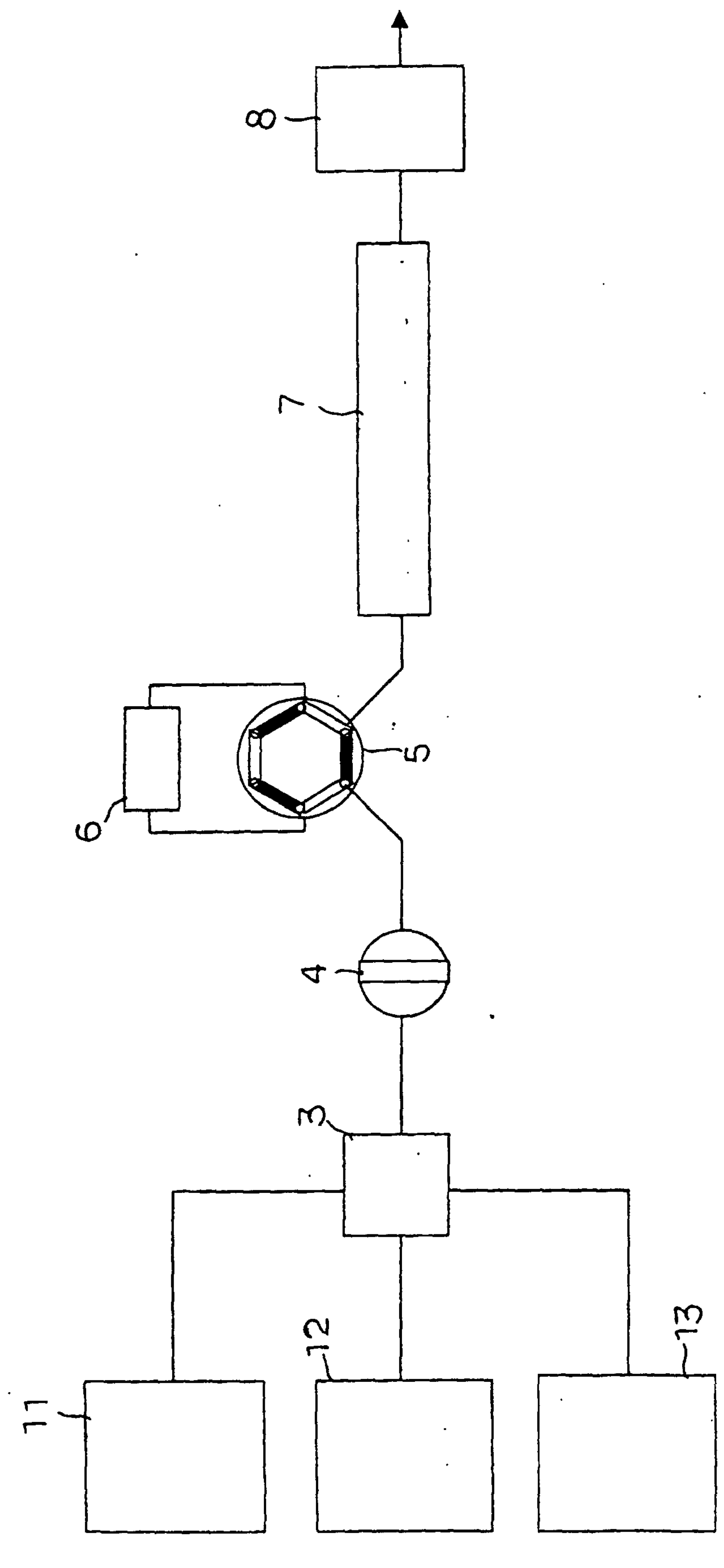

Fig. 26

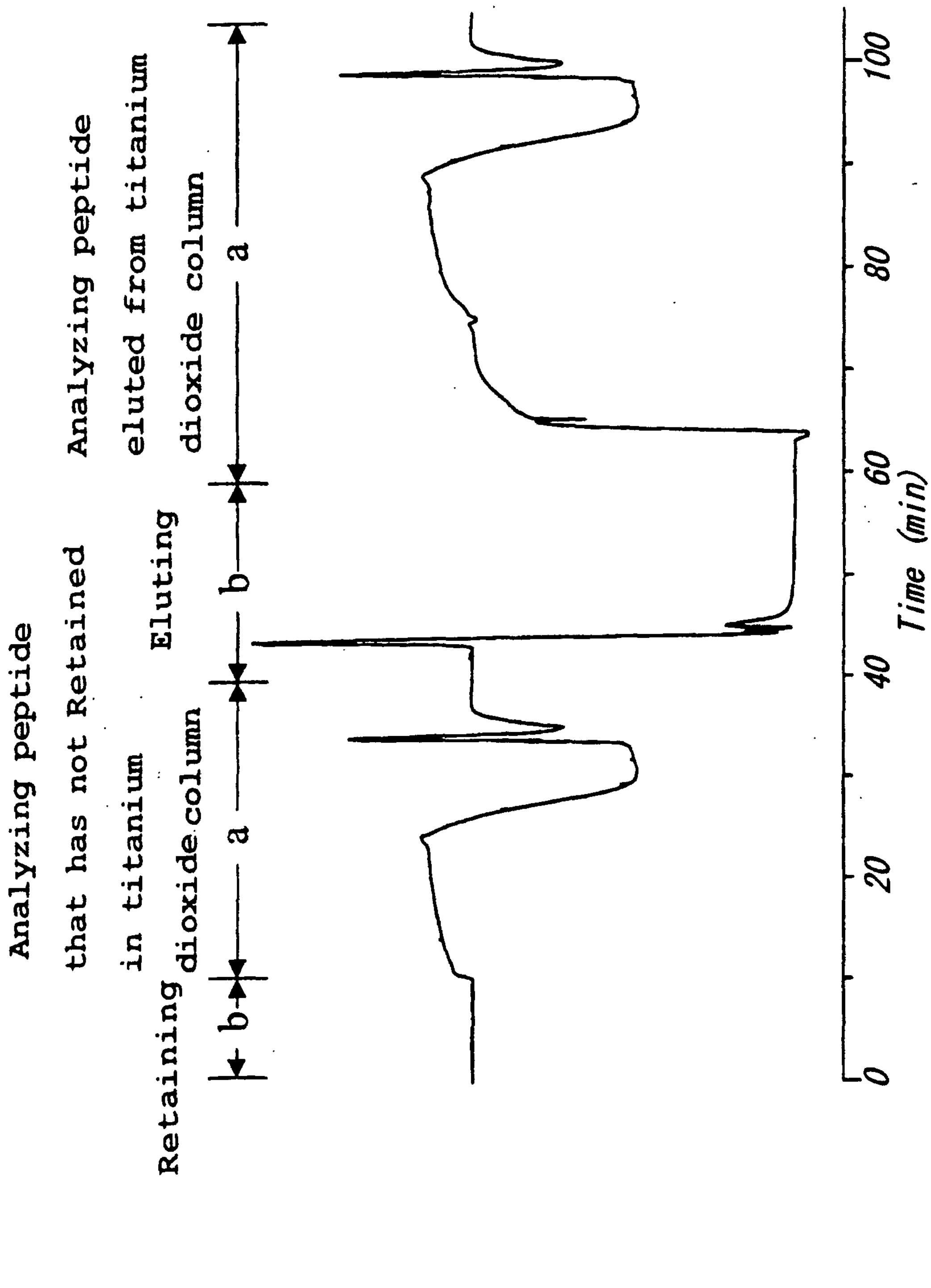
Retaining
Analyzing peptide
that has not Retained
in titanium
dioxide column
Eluting
Analyzing peptide
eluted from titanium
dioxide column
b
a
b
a
0
20
40
60
80
100
Time (min)

Fig. 27

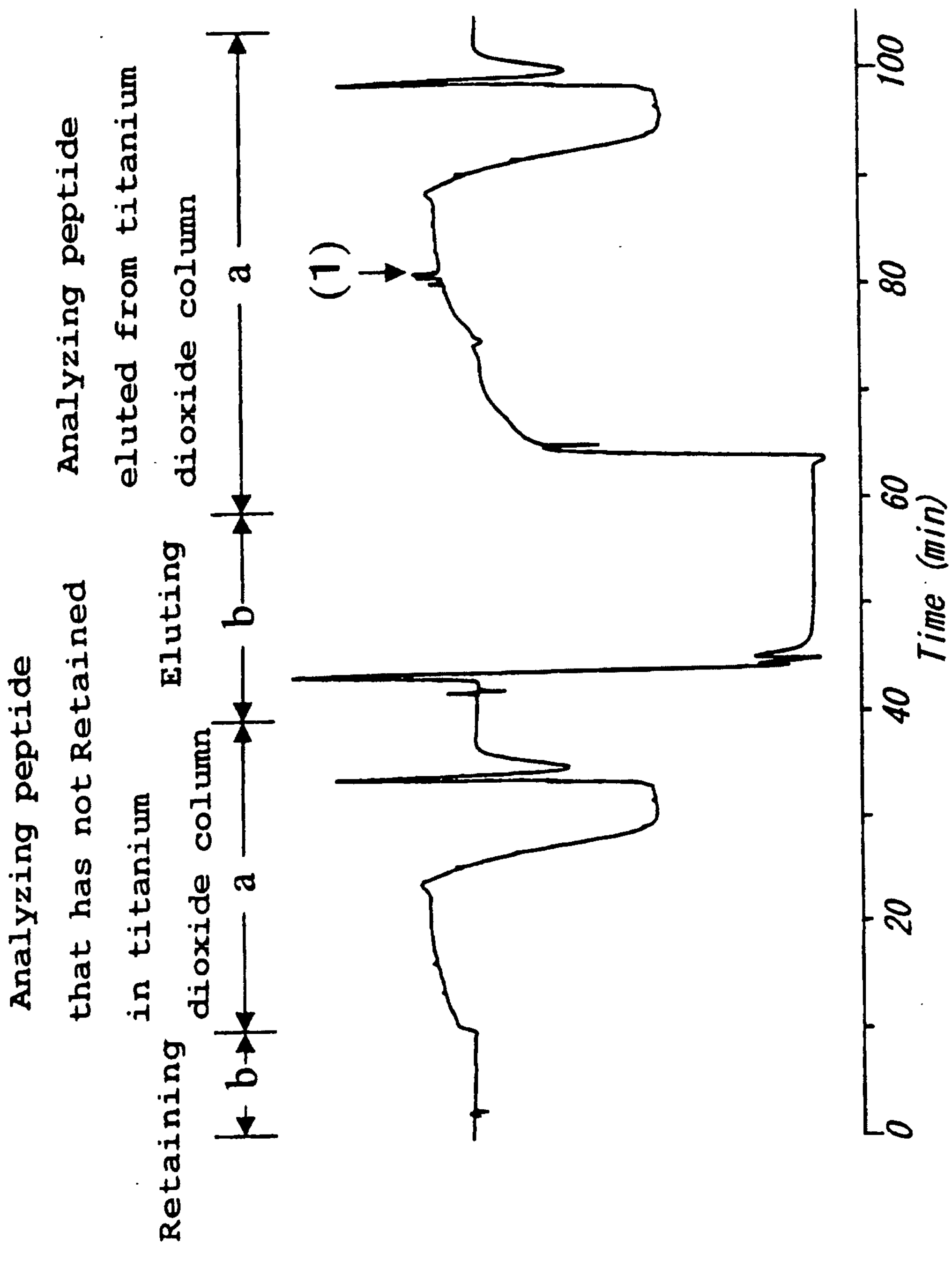
Analyzing peptide that has not Retained in titanium dioxide column
Analyzing peptide eluted from titanium dioxide column
Retaining
Eluting
b
a
b
a
(1)
0
20
40
60
80
100
Time (min)

Fig. 28

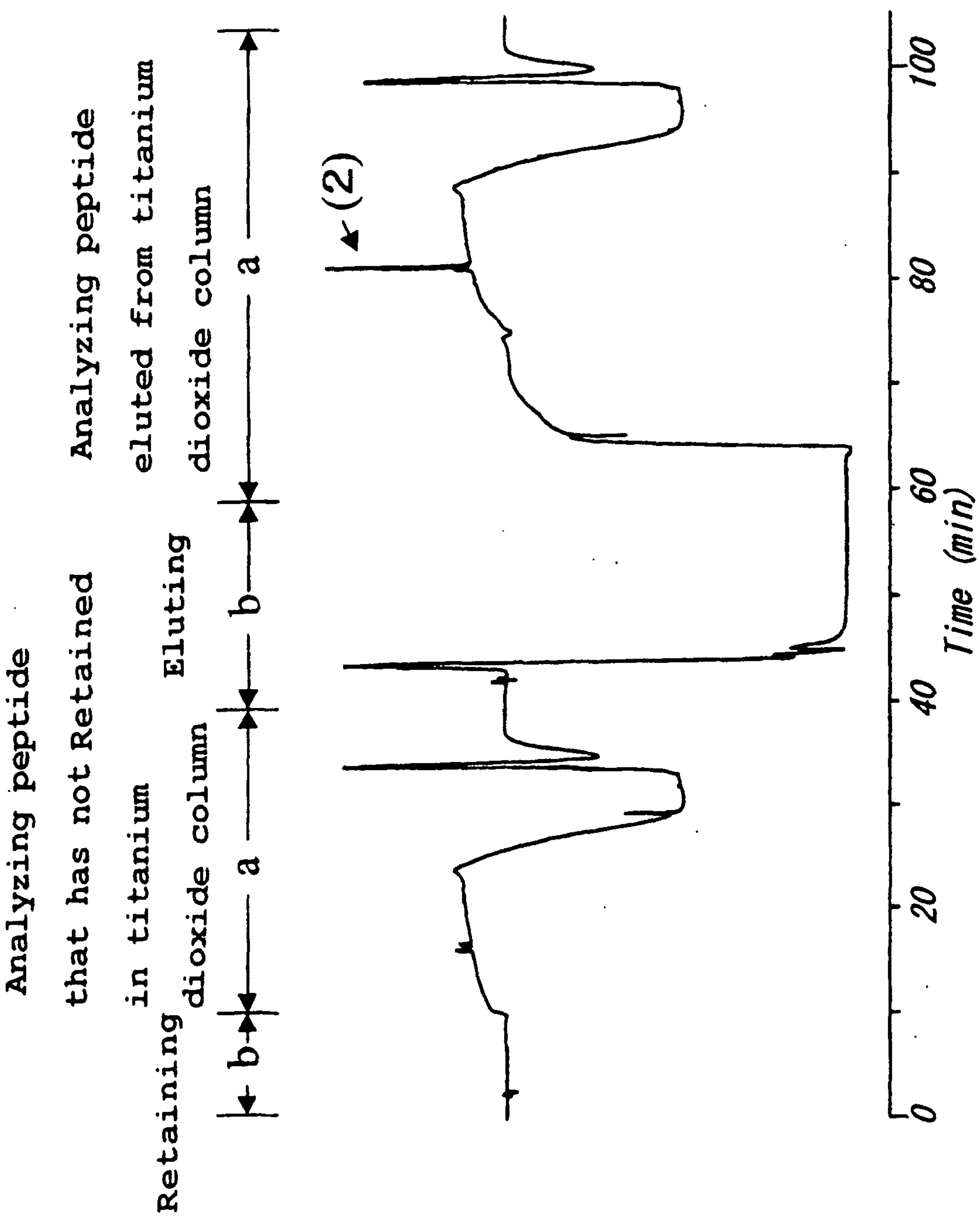
Analyzing peptide that has not Retained in titanium dioxide column
Analyzing peptide eluted from titanium dioxide column
Retaining
Eluting
b
a
b
a
(2)
0
20
40
60
80
100
Time (min)

Fig. 29

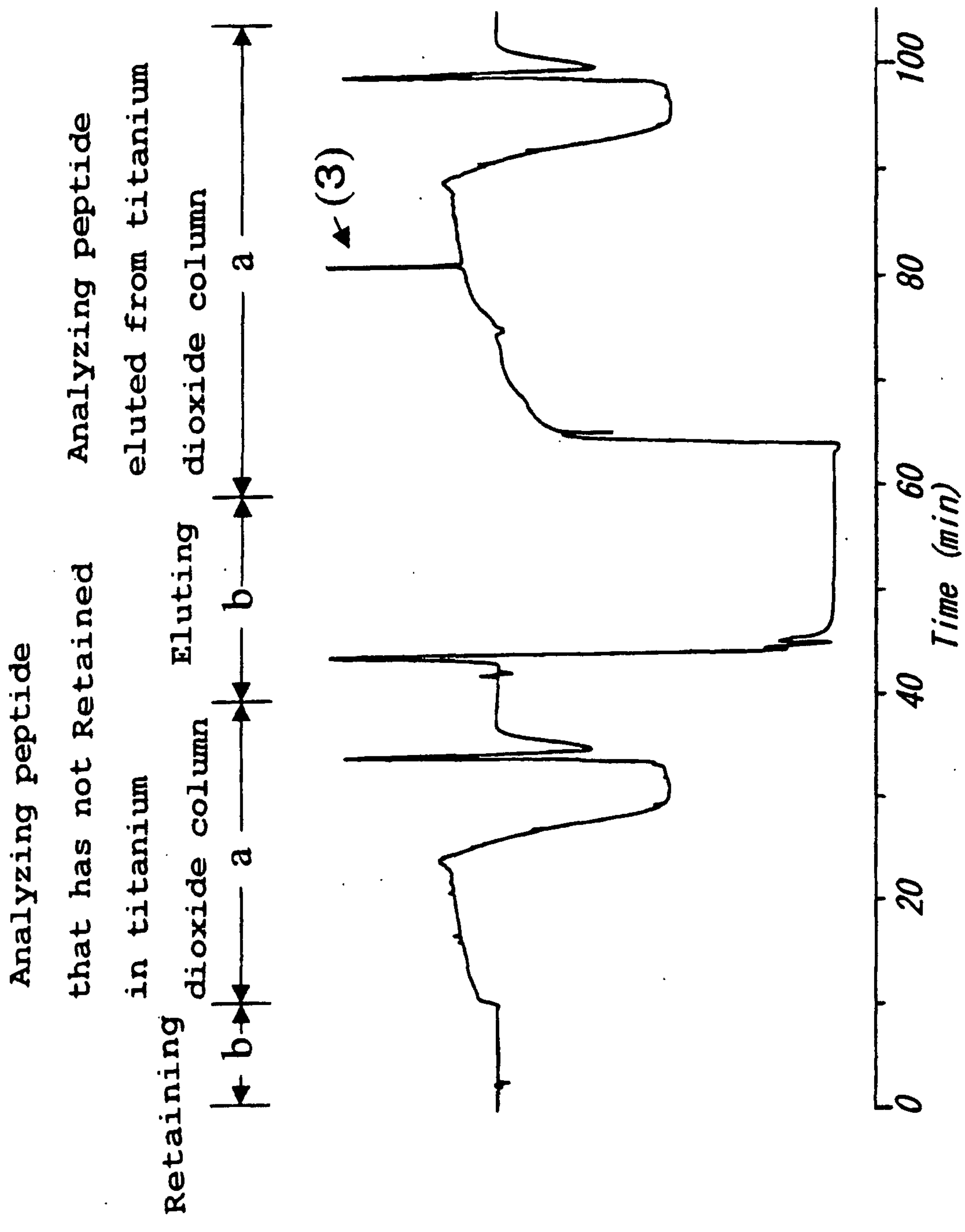
Analyzing peptide that has not Retained in titanium dioxide column
Analyzing peptide eluted from titanium dioxide column
Retaining
Eluting
b
a
b
a
(3)
0
20
40
60
80
100
Time (min)

Fig. 30

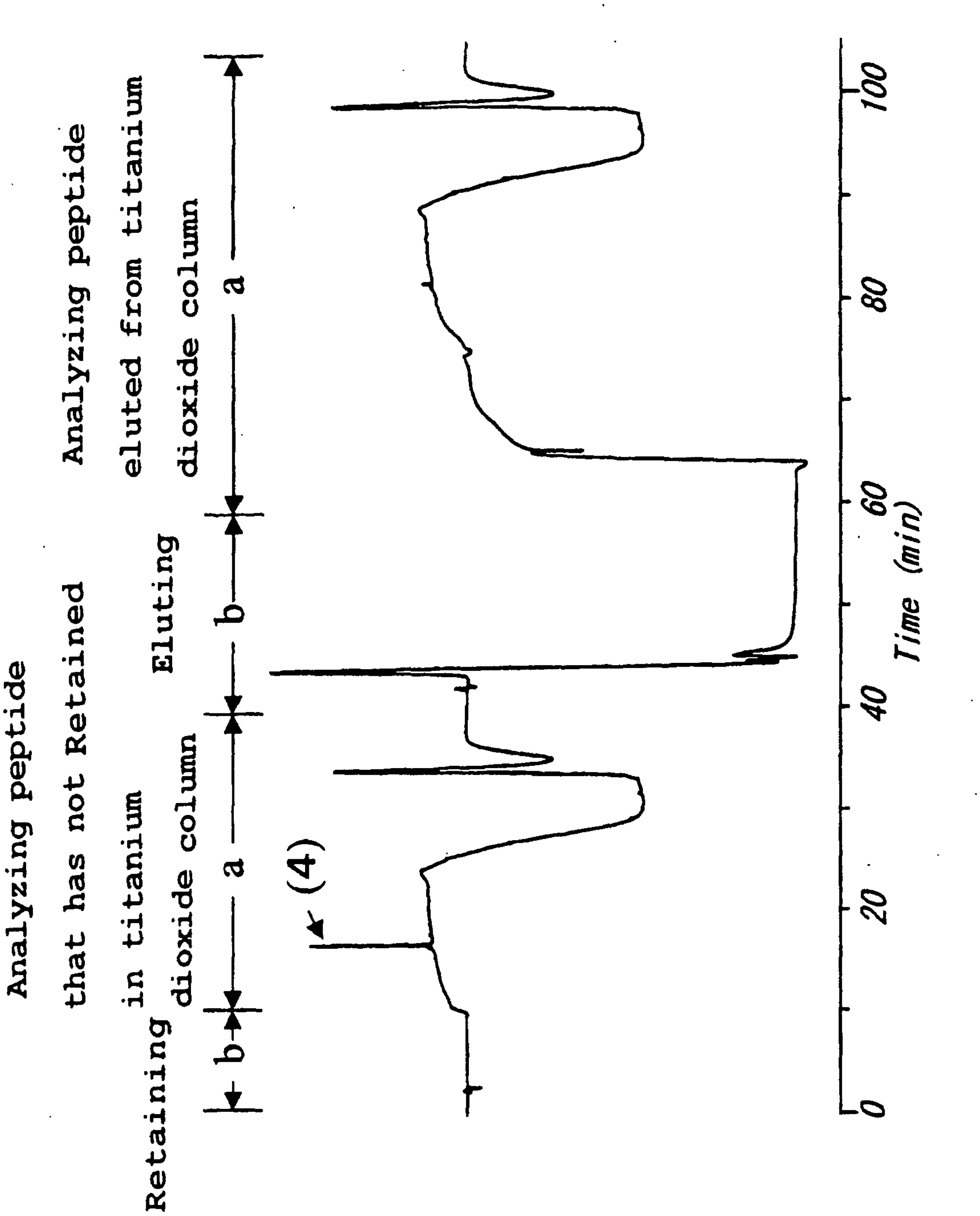
Retaining
Analyzing peptide that has not Retained in titanium dioxide column
Eluting
Analyzing peptide eluted from titanium dioxide column
b
a
b
a
(4)
0
20
40
60
80
100
Time (min)

Fig. 31

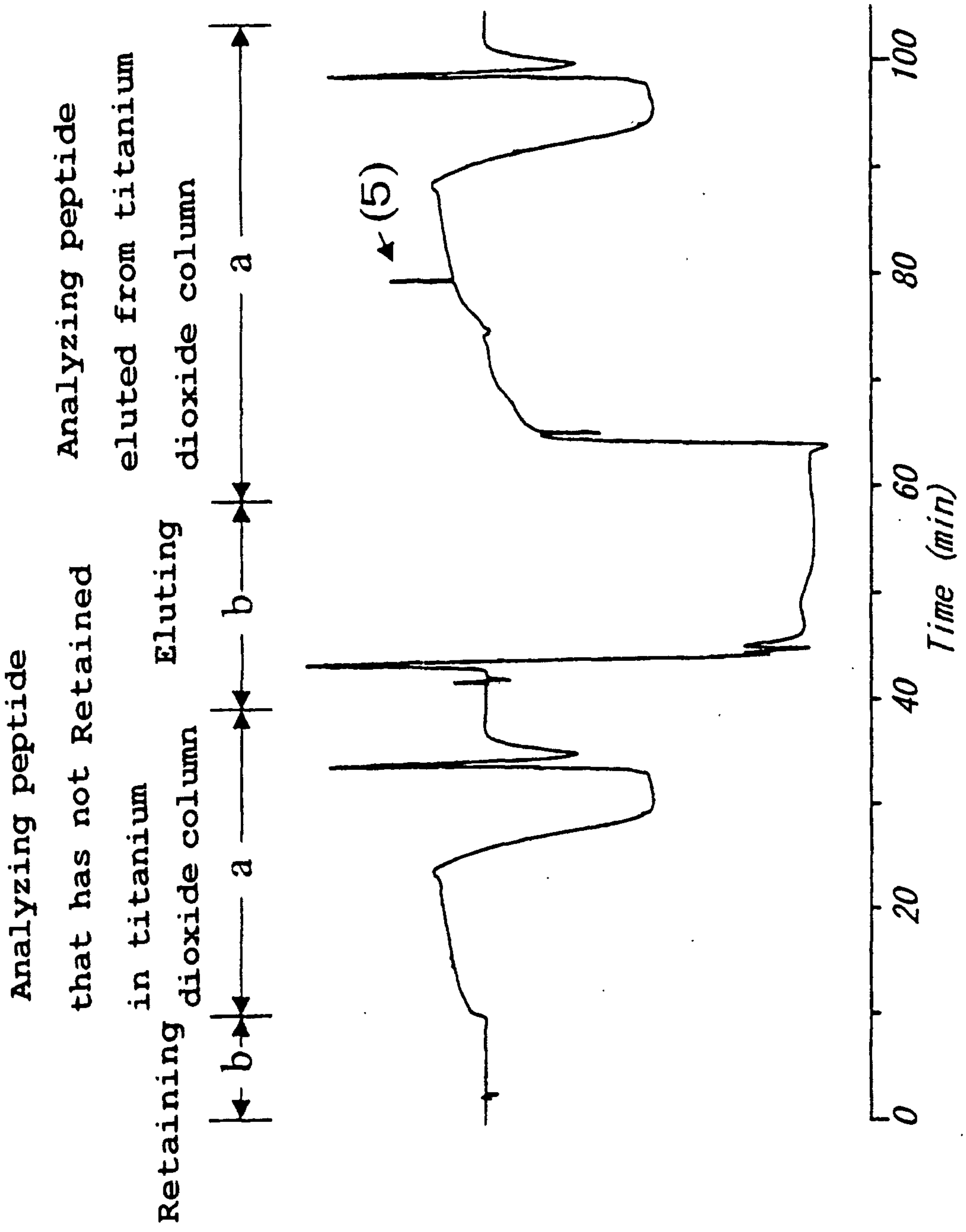
Retaining
Analyzing peptide that has not Retained in titanium dioxide column
Eluting
Analyzing peptide eluted from titanium dioxide column
b
a
b
a
(5)
0
20
40
60
80
100
Time (min)

Fig. 32

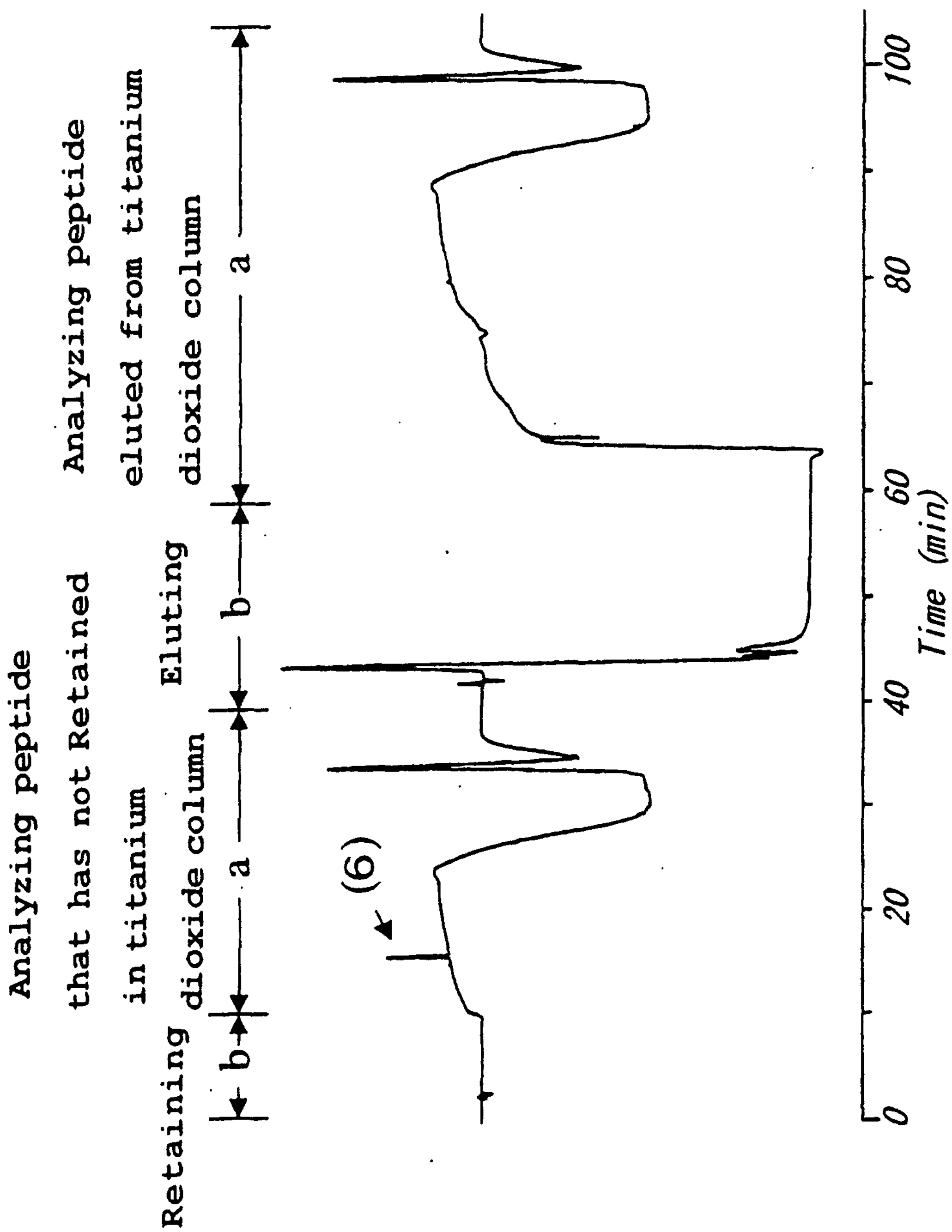
Retaining
Analyzing peptide that has not Retained in titanium dioxide column
Eluting
Analyzing peptide eluted from titanium dioxide column
b
a
b
a
(6)
0
20
40
60
80
100
Time (min)

Fig. 33

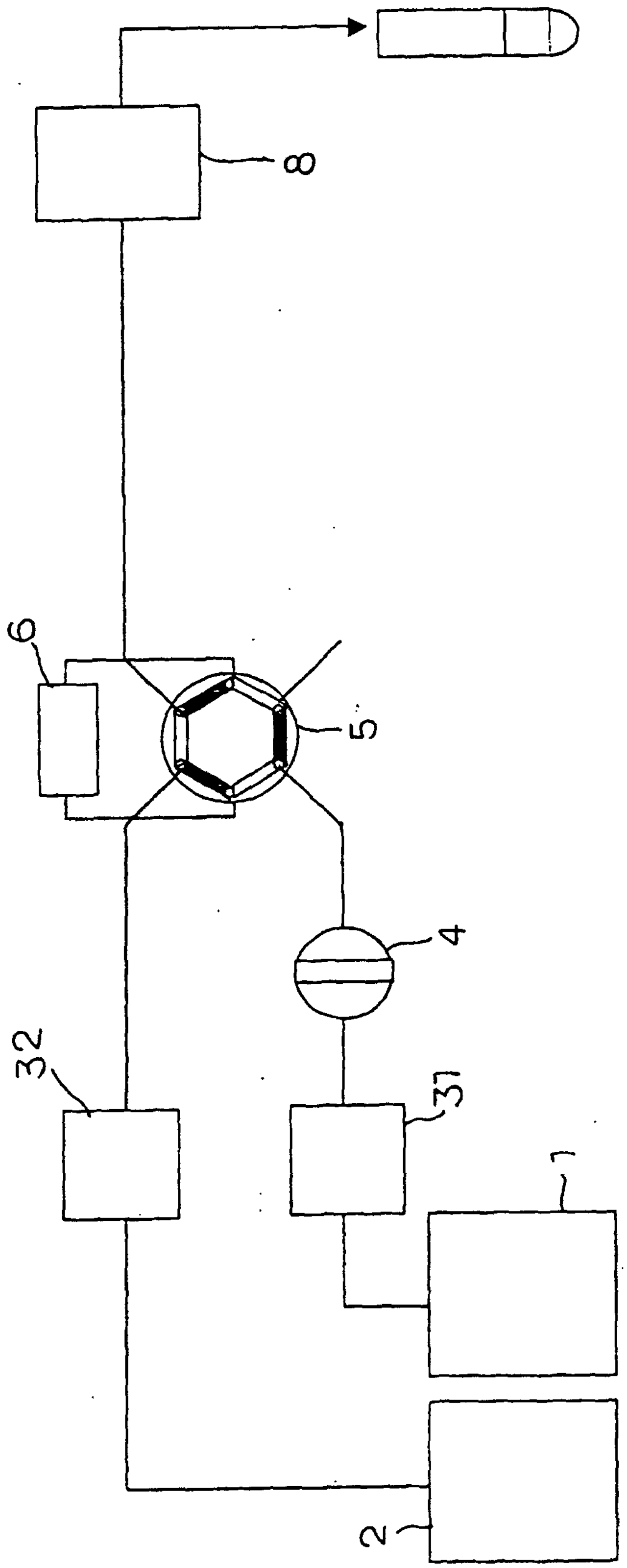

Fig. 34

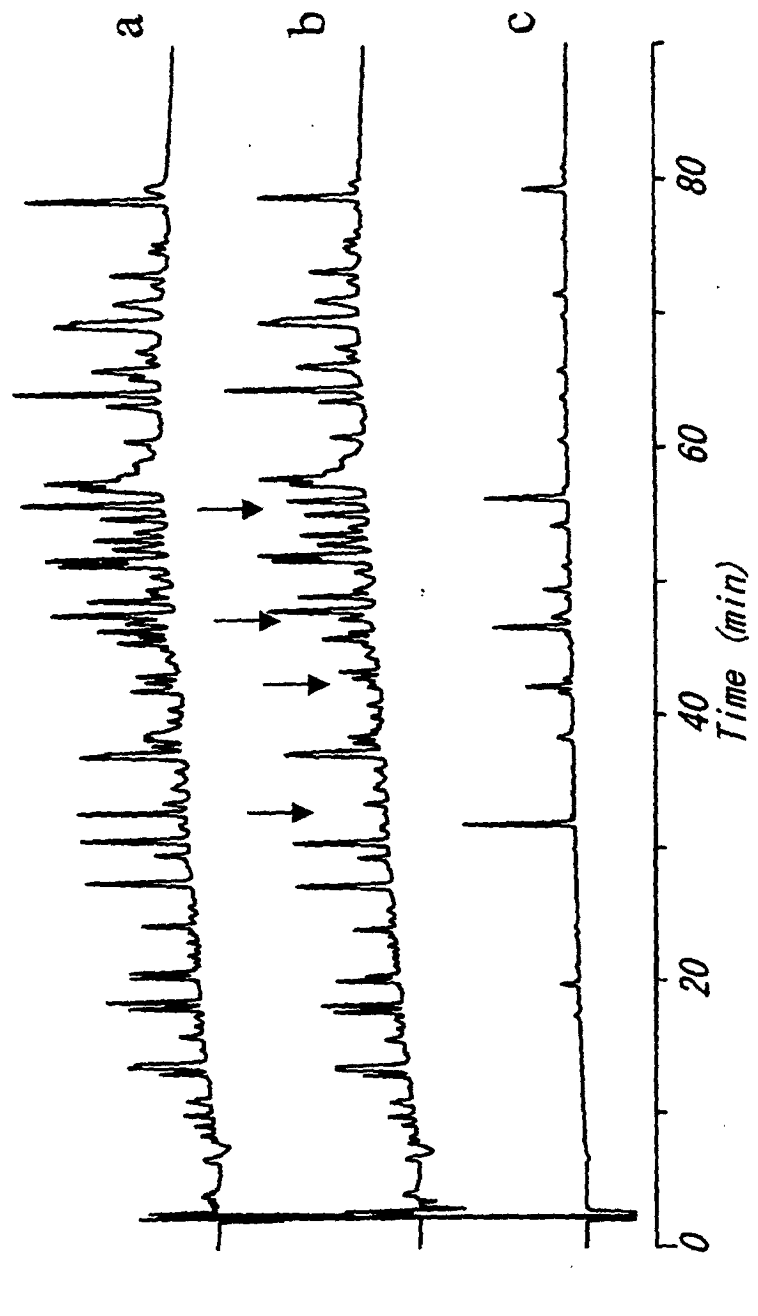

Fig. 35

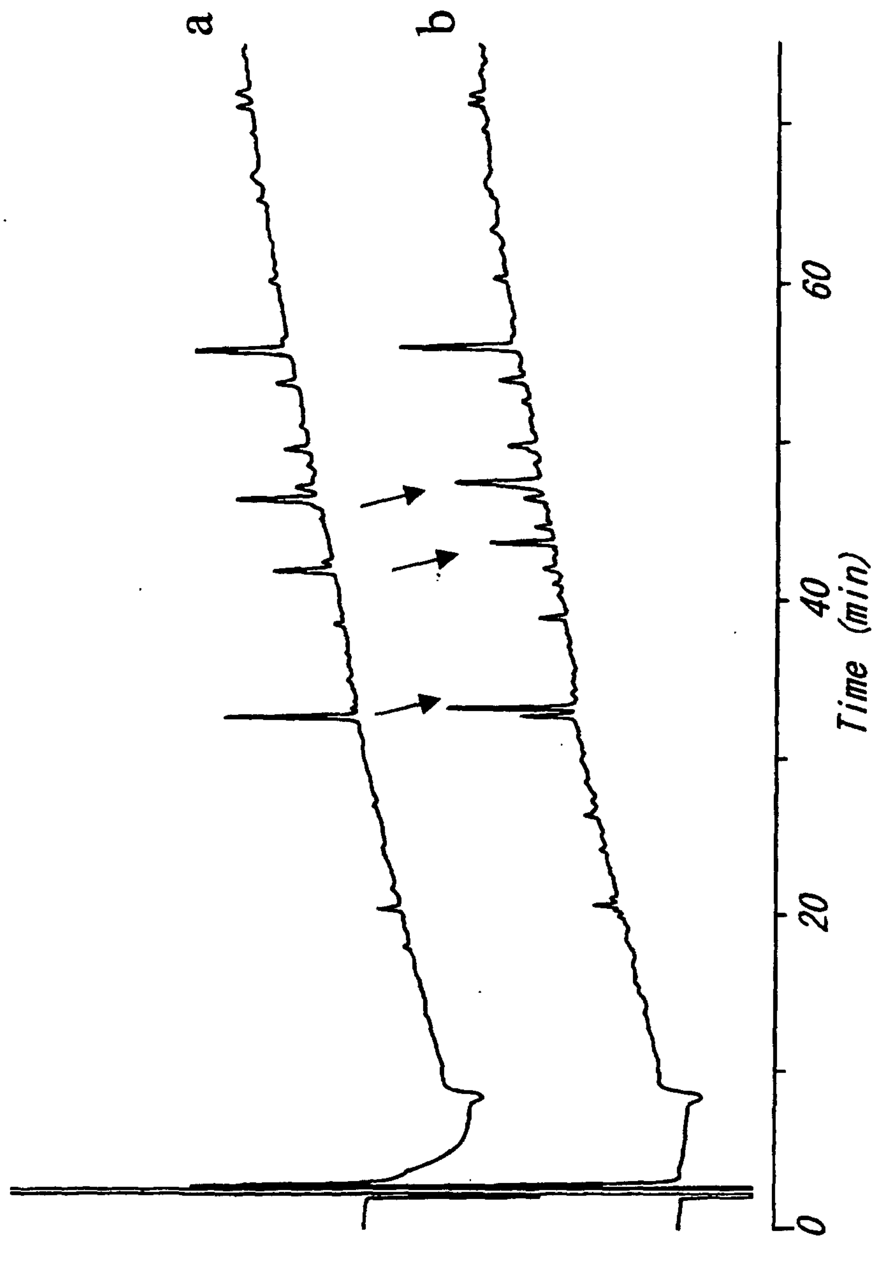

Fig. 36

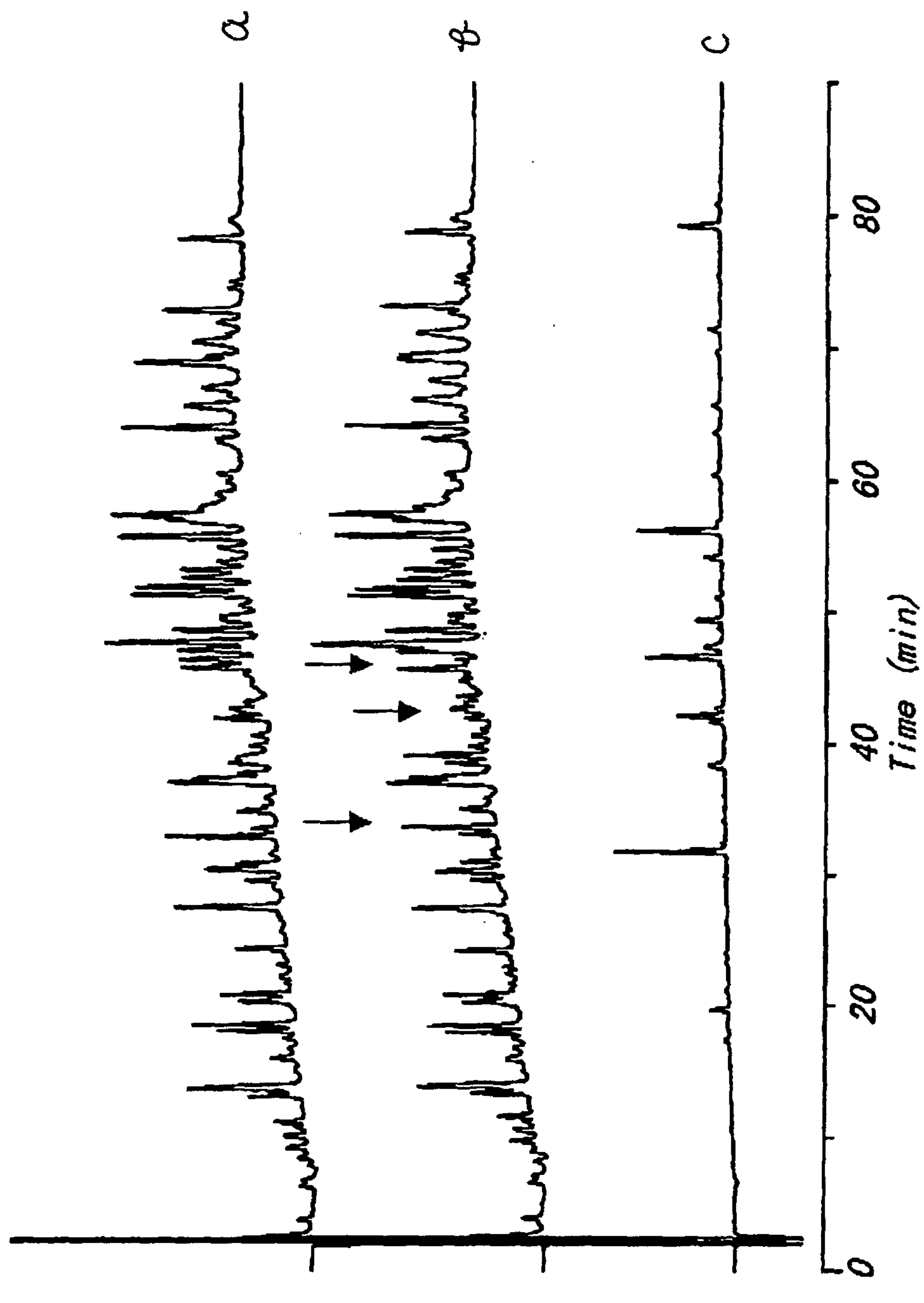

Fig. 37

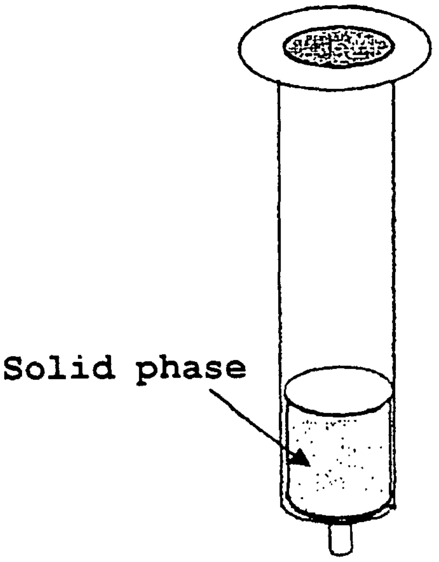


Fig. 38

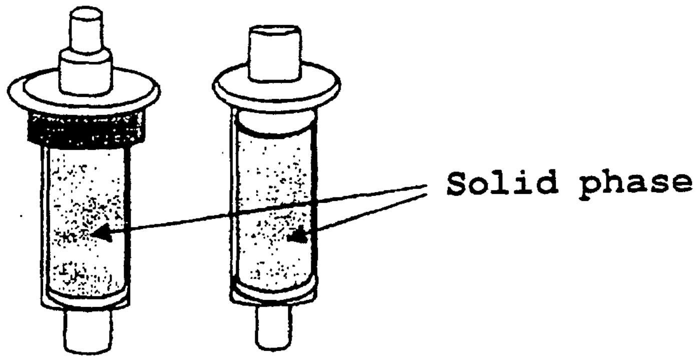

Fig. 39

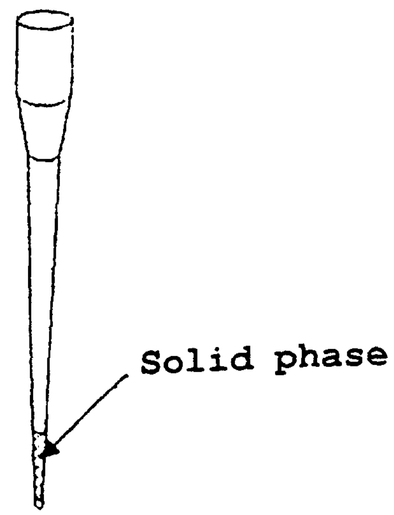


Fig. 40

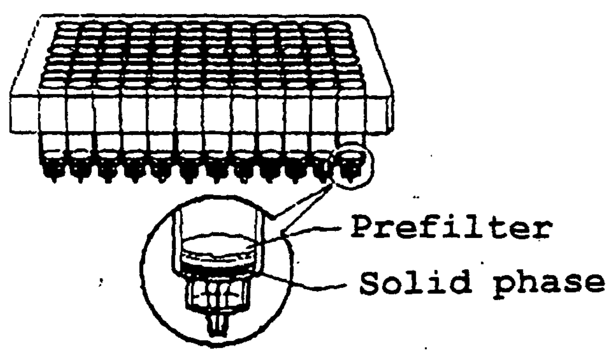

Fig. 41

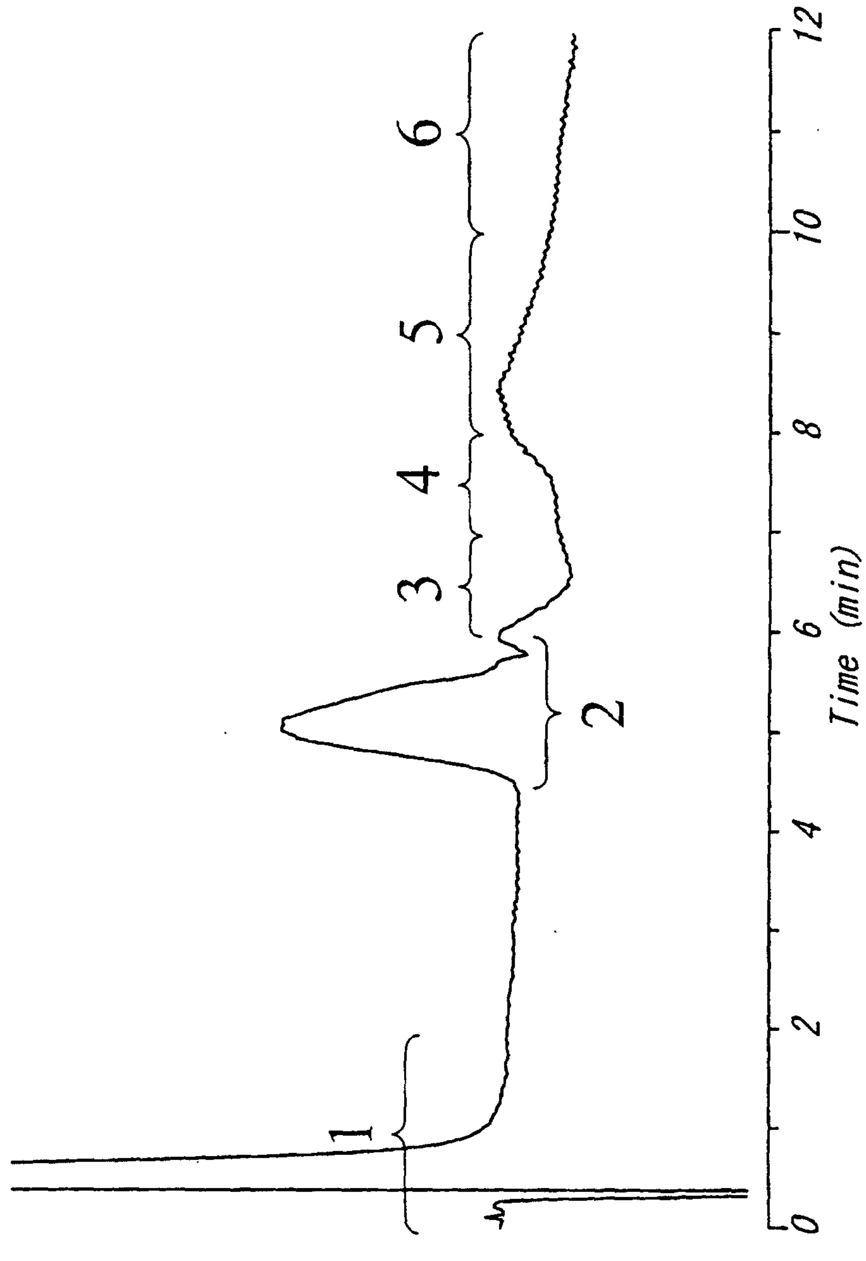

Fig. 42

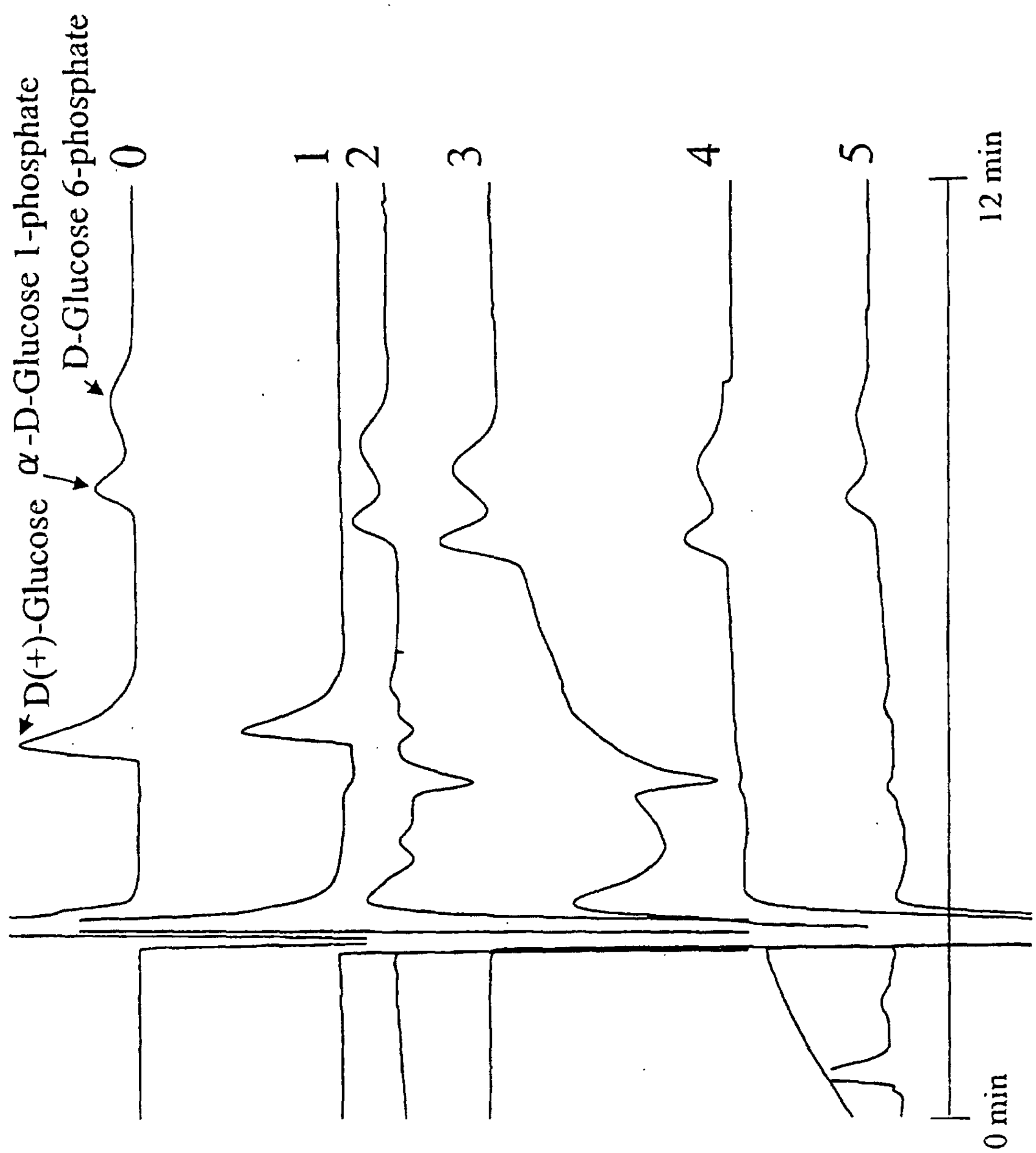
D(+)-Glucose
α-D-Glucose 1-phosphate
D-Glucose 6-phosphate
0
1
2
3
4
5
12 min
0 min

Fig. 43

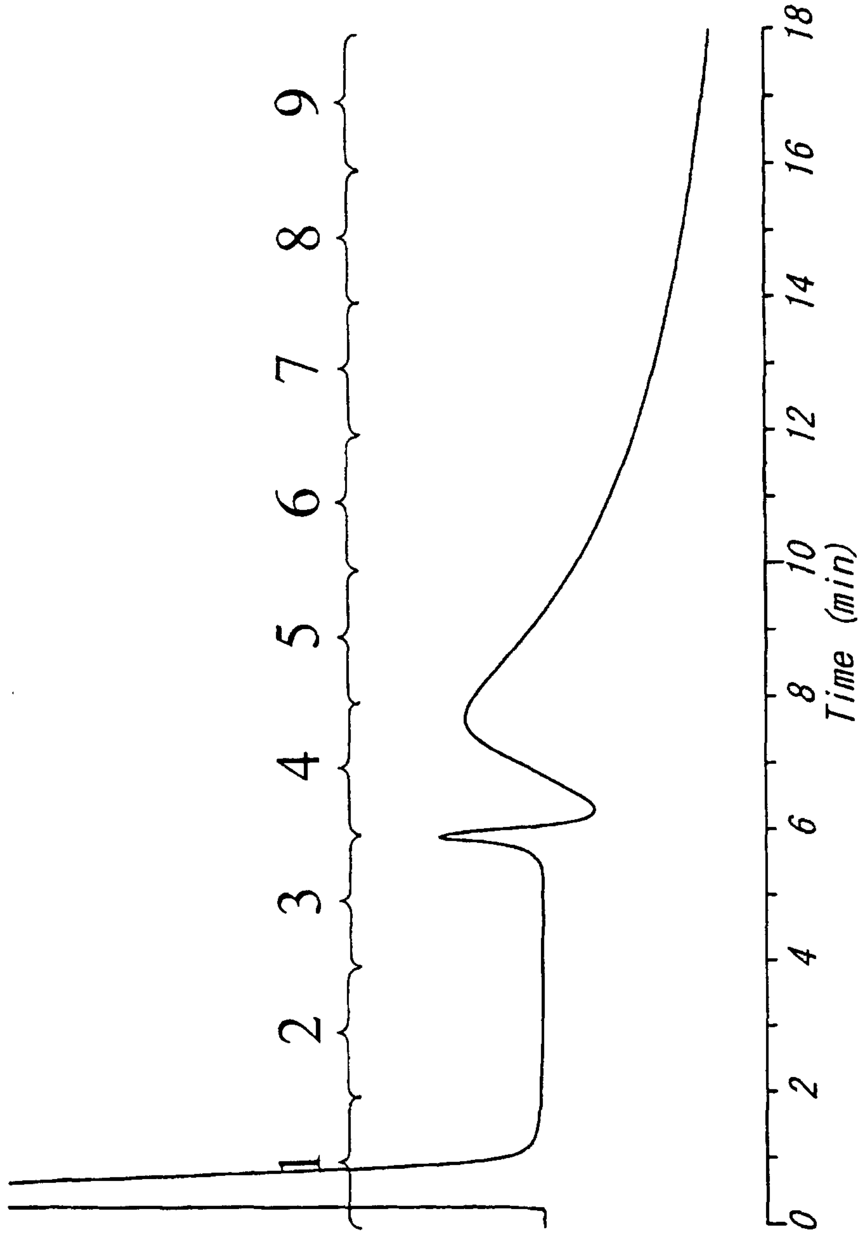

Fig. 44

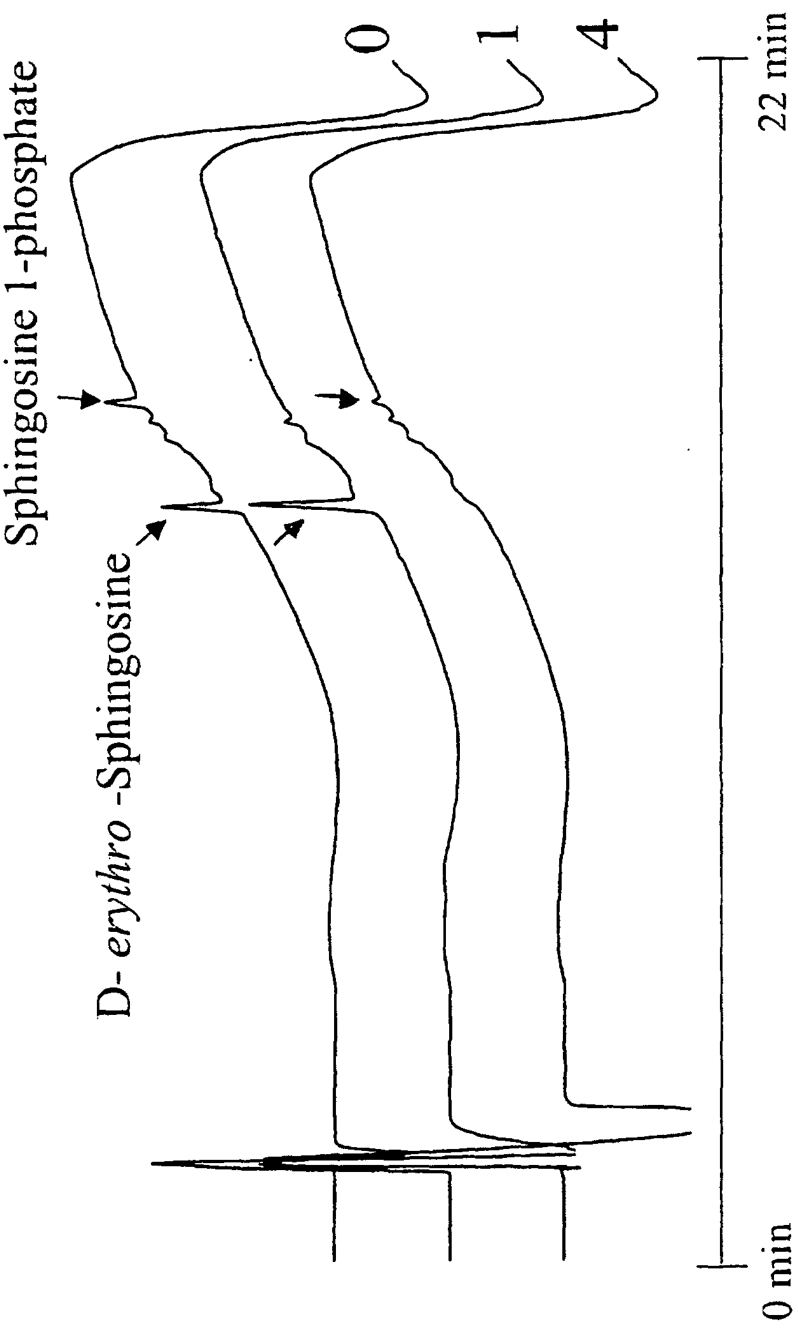
Sphingosine 1-phosphate
D- erythro -Sphingosine
0
1
4
22 min
0 min

Fig. 45

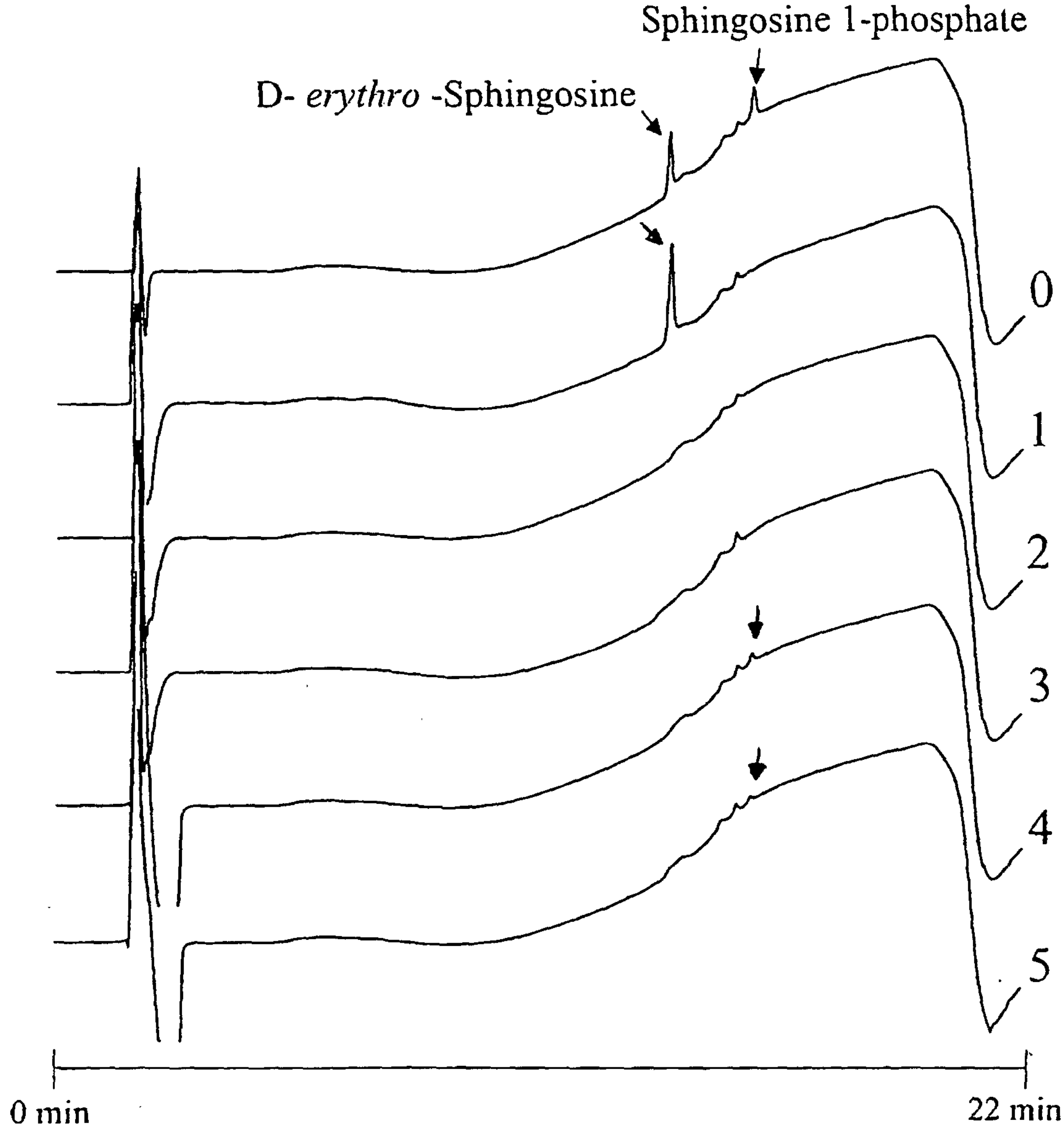
Sphingosine 1-phosphate
D- *erythro* -Sphingosine
0
1
2
3
4
5
0 min
22 min

Fig. 46

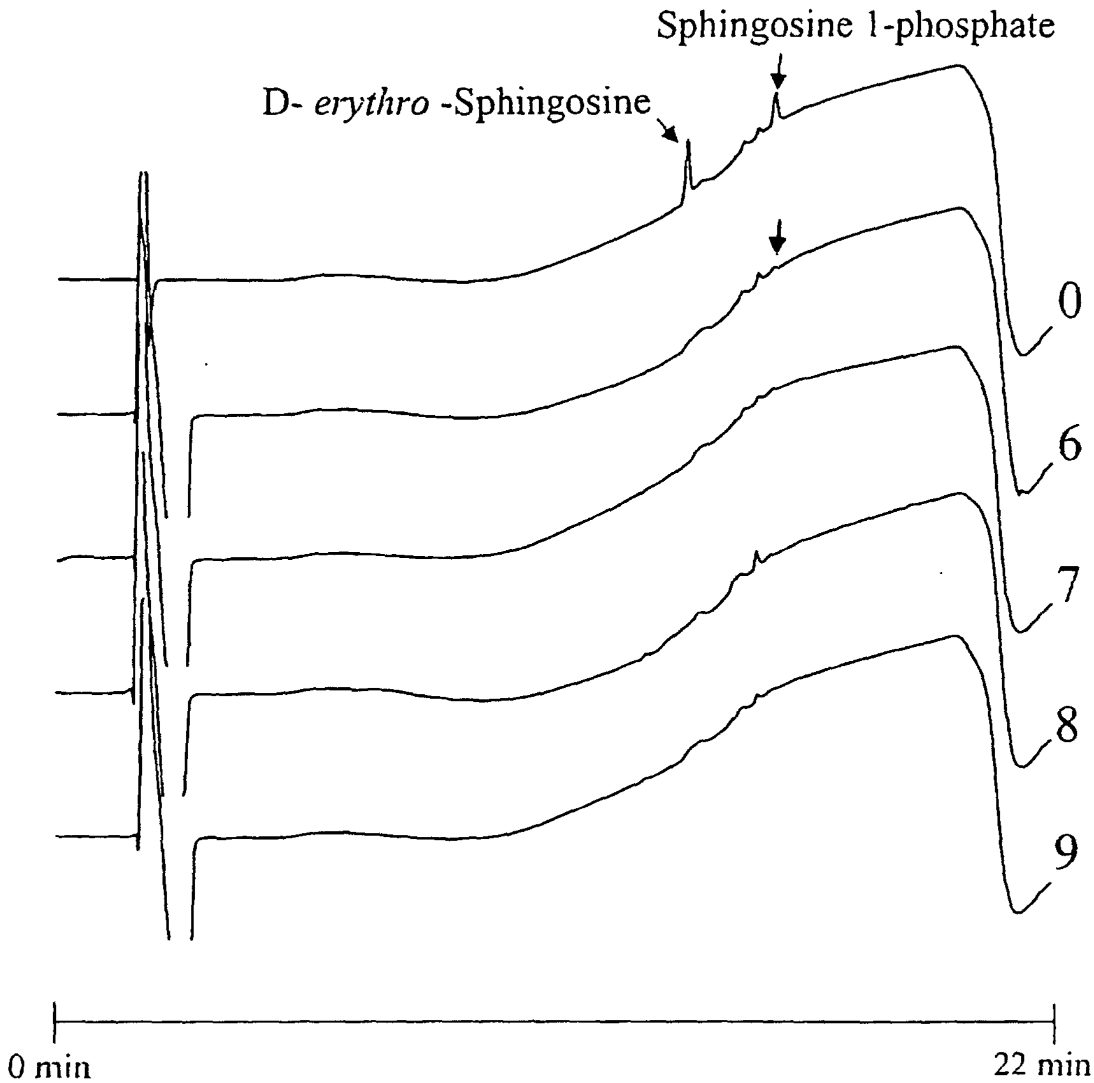
Sphingosine 1-phosphate
D- *erythro* -Sphingosine
0
6
7
8
9
0 min
22 min

Fig. 47

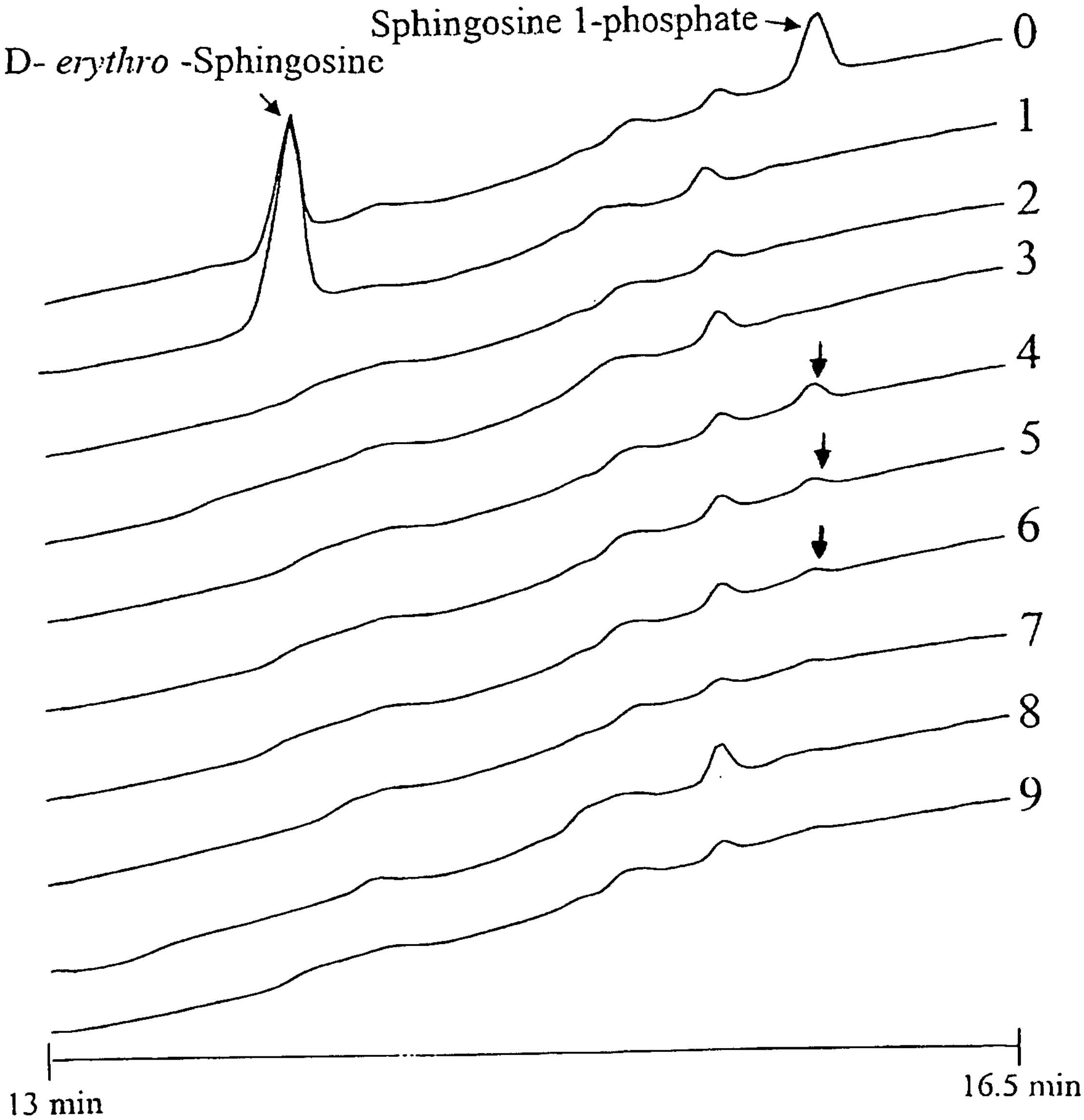
Sphingosine 1-phosphate
D- *erythro* -Sphingosine
0
1
2
3
4
5
6
7
8
9
13 min
16.5 min

Fig. 48

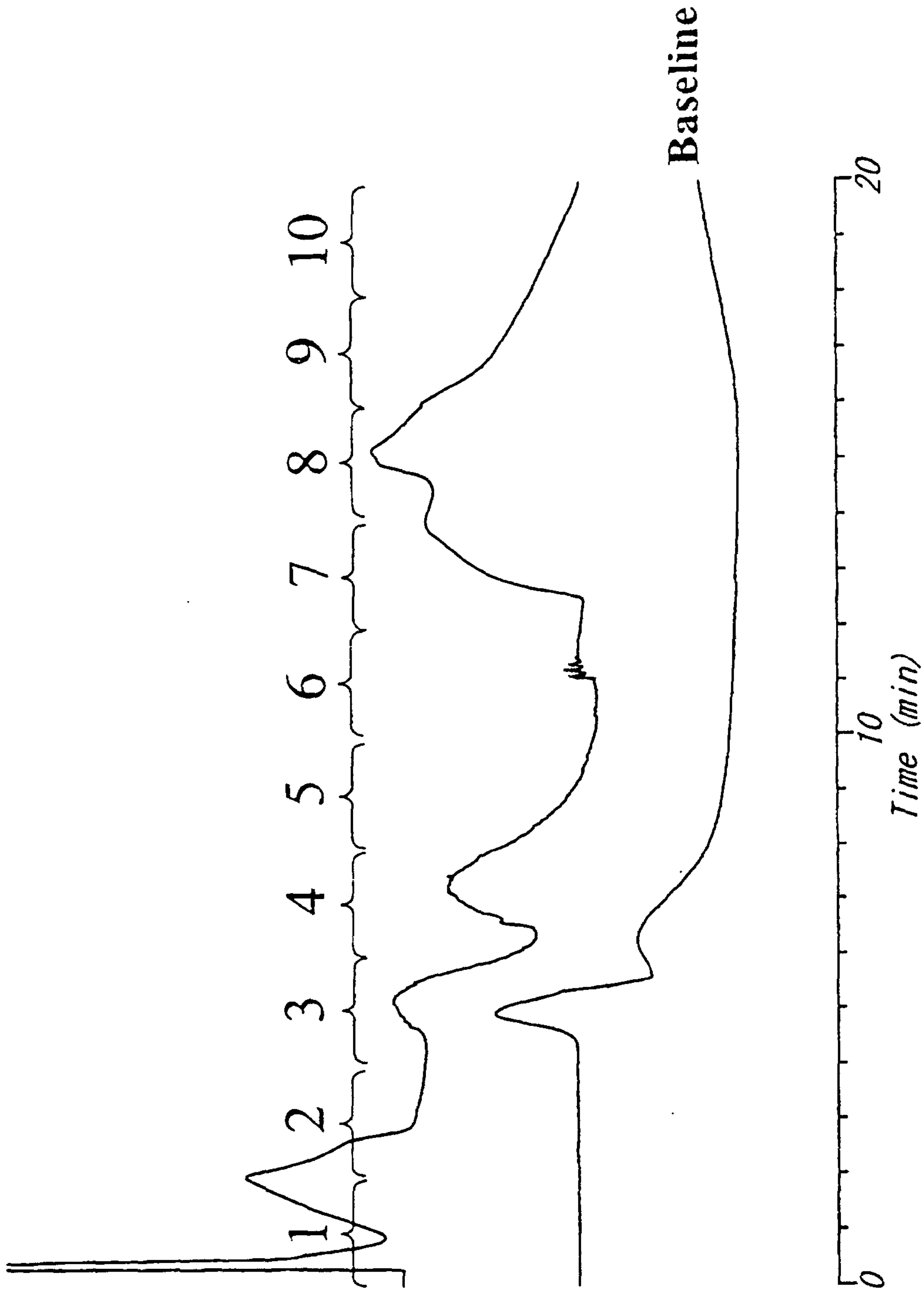

Fig. 49

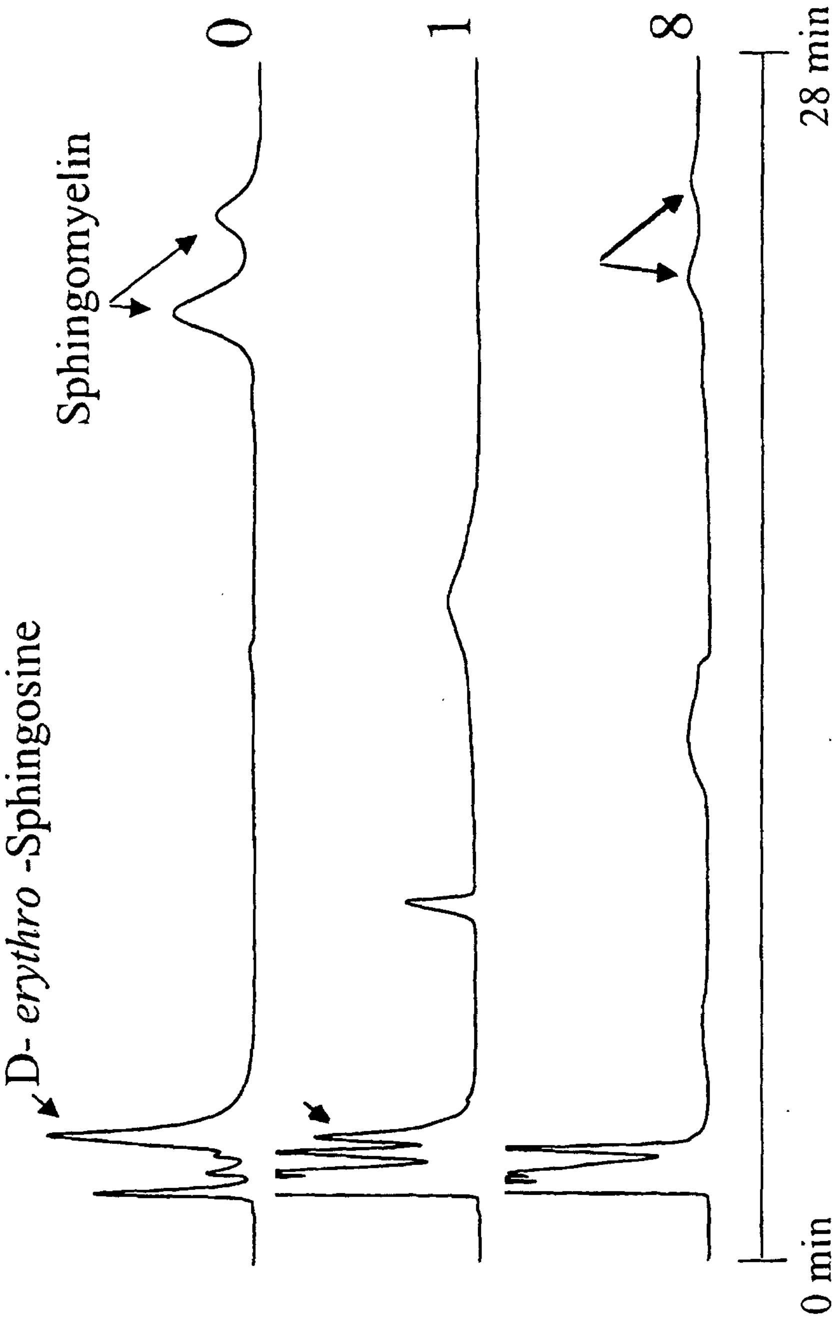

Fig. 50

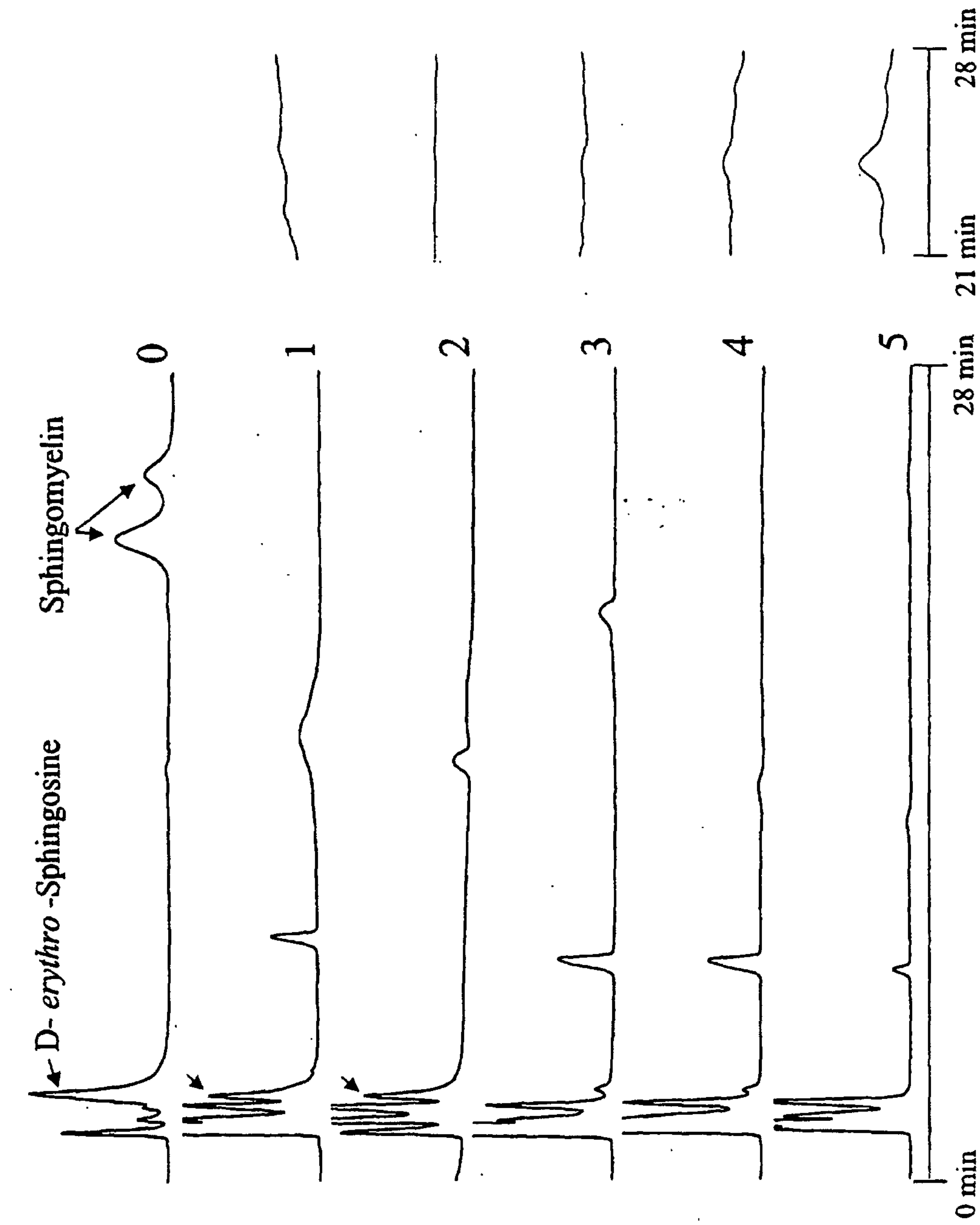
D- erythro -Sphingosine
Sphingomyelin
0
1
2
3
4
5
0 min
28 min
21 min
28 min

Fig. 51

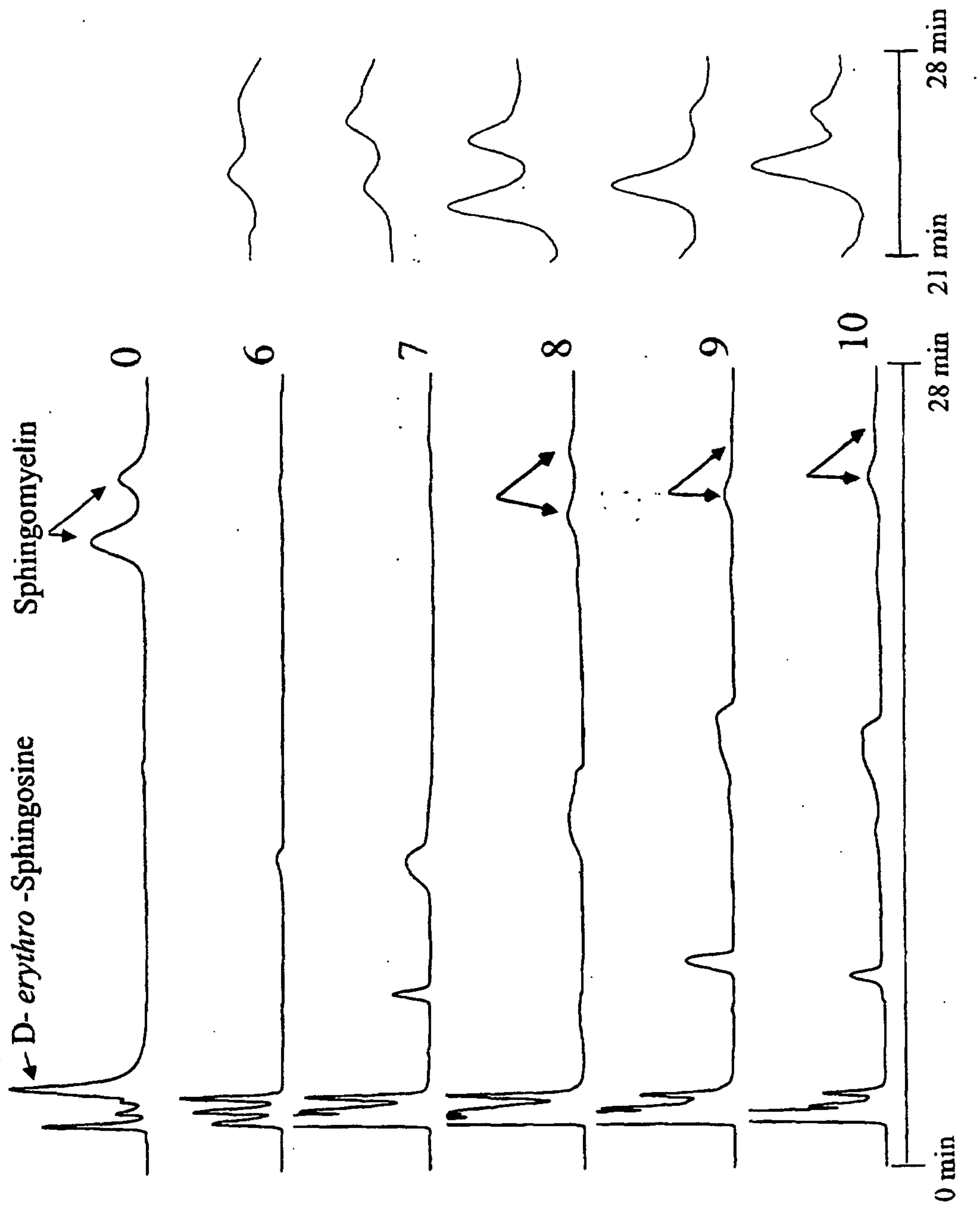
D- erythro -Sphingosine
Sphingomyelin
0
6
7
8
9
10
0 min
28 min
21 min
28 min

## INTERNATIONAL SEARCH REPORT

International application No.
PCT/JP03/00974

A. CLASSIFICATION OF SUBJECT MATTER
Int.Cl$^7$ G01N30/88, G01N30/48, G01N30/26, G01N30/74, G01N30/06, G01N27/62

According to International Patent Classification (IPC) or to both national classification and IPC

B. FIELDS SEARCHED

Minimum documentation searched (classification system followed by classification symbols)
Int.Cl$^7$ G01N30/88, G01N30/48, G01N30/26, G01N30/74, G01N30/06, G01N27/62

Documentation searched other than minimum documentation to the extent that such documents are included in the fields searched

| | | | |
|---|---|---|---|
| Jitsuyo Shinan Koho | 1922-1996 | Toroku Jitsuyo Shinan Koho | 1994-2003 |
| Kokai Jitsuyo Shinan Koho | 1971-2003 | Jitsuyo Shinan Toroku Koho | 1996-2003 |

Electronic data base consulted during the international search (name of data base and, where practicable, search terms used)
JOIS: [CHITANIA+SANKACHITAN] * [KUROMATOGURAFI+KYUCYAKU] (in Japanese)

C. DOCUMENTS CONSIDERED TO BE RELEVANT

| Category* | Citation of document, with indication, where appropriate, of the relevant passages | Relevant to claim No. |
|---|---|---|
| X | Akira SANO, Hiroshi NAKAMURA, Chromatography, Vol.17, No.4, pages 354 to 355, 1996 | 1-10 |
| X | Yoshihiko IKEGUTHI & Hiroshi NAKAMURA, Analytical Sciences, Vol.13, No.3, pages 479 to 483 (1997) | 1-10 |
| X | Hiroshi NAKAMURA, "Biryo Genso no Kagakuteki Sonkeitai Shikibetsu no tameno Sentakuteki na Bunri·Bunsekiho Kaihatsu ni Kansuru Kenkyu", Heisei 7-8 Nendo Kagaku Kenkyuhi Hojokin [Kiban Kenkyu (A)(1)] Kenkyu Seika Hokoku, pages 59 to 64, 1997 | 1-10 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 24 February, 2003 (24.02.03) | 11 March, 2003 (11.03.03) |
| Name and mailing address of the ISA/ Japanese Patent Office | Authorized officer |
| Facsimile No. | Telephone No. |

Form PCT/ISA/210 (second sheet) (July 1998)